# EUROPEAN PATENT APPLICATION

(11) **EP 3 712 180 A1**
(43) Date of publication of application: **23.09.2020**
(21) Application number: 18884473.2
(22) Date of filing: 16.11.2018
(51) Int. Cl.: C07K 16/40, C12N 15/10, A61K 39/00

(54) **ANTIBODY INHIBITING ACTIVATED RAS IN CELL BY INTERNALIZING INTO CYTOSOL OF CELL, AND USE THEREOF**

(30) Priority: 16.11.2017 KR 20170152998
(71) Applicant: Orum Therapeutics Inc., Daejeon 34050 (KR)
(72) Inventor: KIM, Yong Sung, Suwon-si Gyeonggi-do 16534 (KR); CHOI, Dong Ki, Daejeon 34052 (KR); SHIN, Seung Min, Seoul 02750 (KR); PARK, Seong-Wook, Suwon-si Gyeonggi-do 16496 (KR)
(74) Representative: Mewburn Ellis LLP
(86) International application number: PCT/KR2018/014110
(87) International publication number: WO 2019/107812

(57) **Abstract**

A tumor-specific cytosol-internalized RAS-inhibiting antibody, in which modified heavy-chain variable region and a light-chain variable region are combined, according to the present disclosure facilitates development into a therapeutic drug due to a high production yield, and can effectively suppress mutant RAS by means of tumor-specific internalization into the cytosol, and thus effective anti-cancer activity can be expected as a stand-alone drug or in combination treatment with existing medicine.

## Description

### [Technical Field]

The present disclosure relates to an antibody that binds, in the form of an intact immunoglobulin, to a membrane protein receptor on the surface of cells overexpressed in tumor tissues and thus undergoes endocytosis, is then located in the cytoplasm of the cells due to the endosomal escape capacity, and specifically binds to activated Ras(Ras·GTP) coupled to GTP in the cytoplasm to inhibit the activity of tumor mutant Ras, a method of preparing the same and the use thereof.

Specifically, the present disclosure relates to a heavy-chain variable region (VH) having high affinity for Ras·GTP that is produced by modifying a heavy-chain variable region (VH) of an antibody that penetrates, in an intact immunoglobulin form, into the cytoplasm of cells to directly inhibit intracellular Ras·GTP, an antibody including the same, a method of producing the same and the use thereof.

The antibody of the present disclosure includes an improved technology to impart tumor-tissue-specific cytoplasmic penetration ability for a light-chain variable region (VL).

The present disclosure relates to an intact immunoglobulin-type antibody that includes a combination of an improved light-chain variable region (VL), modified to impart tumor-tissue-specific cytoplasmic penetration ability thereto, with a heavy-chain variable region (VH) having improved affinity, and thus penetrates into the cytoplasm and directly inhibits intracellular Ras·GTP.

The present disclosure includes an improved technology for a heavy chain to advance the intracellular stability, *in-vivo* persistence and tumor-tissue specificity of the antibody.

In addition, the present disclosure relates to a method of inhibiting the growth of cancer or tumor cells using the antibody and a method of treating cancer or tumors using the antibody.

In addition, the present disclosure relates to a method of constructing a library for improving the affinity of a heavy-chain variable region specifically binding to Ras·GTP, and a library constructed by the method.

In addition, the present disclosure relates to a method for screening, using the library, a heavy-chain variable region that specifically binds to Ras·GTP and has improved affinity therefor.

### [Background Art]

Mutations and abnormal overexpression occur in enzymes or transcription- or signaling-associated proteins, which play an important role in intracellular protein-protein interactions (PPIs) in a variety of diseases including cancer. In order for small molecule drugs to specifically bind to these tumors and disease-related proteins, a hydrophobic pocket is required on the protein surface, but only about 10% of all intracellular-disease-related substances have such a hydrophobic pocket. For this reason, small molecule drugs cannot specifically target most intracellular tumor- and disease-related proteins. Ras, which is one of representative examples of tumor- and disease-related proteins having no hydrophobic pocket, acts as a molecular switch that delivers an extracellular signal to an intracellular signaling system through a cell membrane receptor on the cell surface. Cancer-related proteins in the Ras protein family are three isoform proteins, namely, KRas, NRas and HRas. KRas may be expressed as either of two splicing variants, namely KRas4A and KRas4B. After the Ras protein is expressed in the cytoplasm, it passes through the endoplasmic reticulum (ER) and the Golgi apparatus and then is located in the cell membrane due to the lipidation reaction of the C-terminal region, but the portion thereof having GTPase activity is exposed toward the cytoplasm. In the absence of external stimulation, the Ras protein exists as inactivated Ras(Ras·GDP) bound to GDP by GTPase-activating protein (GAP) in the cell. In the presence of external stimulation, the Ras protein exists as activated Ras(Ras·GTP) bound to GTP by guanine nucleotide exchange factor (GEF). Ras·GTP activates signals such as lower Raf-MEK-ERK and PI3K-Akt through protein-protein interactions with effector proteins such as Raf, PI3K and RalGDS in the cytoplasm to transfer a variety of signals pertaining to cell growth, apoptosis inhibition, migration, differentiation, etc. In normal cells, Ras·GTP is converted to Ras·GTP immediately after signaling due to the phosphate dissociation by GAP, so signaling is regulated in a way that only signals are temporarily transferred. However, the carcinogenic mutant Ras protein does not undergo a phosphate dissociation process by GAP but maintains the form of Ras·GTP and thus continuously interacts with the effector protein, thus continuously leading to downstream signaling and carcinogenesis of normal cells. The most frequently occurring Ras protein carcinogenic mutations include the 12th residue mutations (such as G12D, G12V, G13D and G12C), the 13th residue mutations (such as G13D) and the 61st residue mutations (such as Q61R and Q61H). It has been found that these cancer-related Ras mutations occur in about 30% of all tumors, with lung cancer (≈25%), colorectal cancer (≈30-40%) and pancreatic cancer (≈90%), depending on the carcinoma. These carcinogenic Ras mutations are known to cause strong resistance to conventional anti-cancer treatments.

The development of small molecule drugs has been mainly attempted to treat carcinogenic Ras mutant tumors, and the main strategies include inhibition of the activity of enzymes associated with C-terminal lipidation of Ras to thereby prevent the placement of Ras into the intracellular membrane, inhibition of protein-protein interaction between Ras and effect proteins to thereby directly target Ras, and the like. The C-terminal lipidation of Ras is a process for locating Ras expressed in the cytoplasm into the intracellular membrane for activation, and related enzymes thereof include farnesyltransferase (FTase), Ras-converting CAAX endopeptidase 1 (RCE1), isoprenylcysteine carboxylmethyltransferase (ICMT) and the like. Although small molecule drugs targeting the enzymes described above have been developed, they have side effects of cell growth inhibition and apoptosis in the normal Ras wild-type cell line and a limitation in which the drug is not effective due to the indirect (bypass) route by geranylgeranyltransferase 1 (GGTase1) in the KRas mutant tumor having the highest frequency of Ras mutants. As another strategy, small molecule drugs directly targeting Ras include Kobe0065 and Kobe2602, which have a mechanism of binding to activated Ras to inhibit the interaction with Raf, the subeffector protein, but these drugs have limitations in that the stability of the drug is low and a high dose of the drug is required for treatment. In addition, ARS853, which has a mechanism of covalently binding to the KRas G12C mutant to inhibit the interaction with the subeffector protein, Raf, has been developed, but it has a limitation in that it is a drug limited only to the KRas G12C mutant, which accounts for only a small proportion of KRas mutants. In addition, rigosertib having a mechanism of binding to the Ras-binding domain (RBD) of Raf, PI3K and RalGDS as subeffector proteins to inhibit binding to Ras, has been developed, but it has a limitation in that a high dose of the drug is required for treatment.

In addition to small molecule drugs, stapled peptides, which are being developed as structural analogs to alpha helixes, are developed as therapeutic agents targeting proteins inside cells owing to the advantages of improved structural stability of peptides as well as enhanced metabolic stability and cell permeability through linking of two non-adjacent units with hydrocarbon chains. Staple peptides are capable of cytoplasmic penetration using a strategy of mimicking, with a peptide, a site binding to Ras of a Ras guanine nucleotide exchange factor (RasGEF), which converts inactivated Ras to activated Ras, based on these properties. However, such staple peptides have limitations in that a high dose of the peptide is required to obtain the treatment effect and they have non-specific effects on Ras wild-type cell lines as well as Ras mutant cell lines.

In order to overcome these technical limitations of small molecule drugs and staple peptides, various studies have been conducted to impart the ability to penetrate into living cells to high-molecule materials, such as antibody fragments or recombinant proteins, and to effectively inhibit protein-protein interactions. In particular, protein transduction domains (PTDs) having basic amino acid sequences, hydrophobicity and amphipathicity have been found to have the ability to penetrate into living cells, and many attempts have been made to genetically fuse protein permeation domains with various types of antibody fragments to recognize specific proteins inside cells using the same. However, most thereof are not secreted from animal cells, or only a small amount is released into the supernatant, resulting in limitations of low production yield and poor penetration efficiency into the cells. In order to overcome this expression problem, studies have been conducted to fuse cell permeation domains through chemical covalent bonding or biotin-streptavidin-mediated binding, etc., but there is a limitation in that the structure of the corresponding protein is changed.

In general, intact immunoglobulin-type antibodies cannot penetrate directly into living cells due to the large size and hydrophilicity thereof. However, a cytotransmab, which is an intact immunoglobulin-type antibody having cytoplasmic penetration ability, has the ability to penetrate into the cytoplasm through a mechanism that involves binding to the cell membrane receptors, HSPGs (heparan sulfate proteoglycans), entering the cell by clathrin-mediated endocytosis, and then escaping from the endosome into the cytoplasm. HSPG, which is a cytotransmab receptor, is generally expressed in most animal tissues, and acts as a co-receptor for binding various receptors with growth factors and cytokine, functions to form tissues, heal wounds or the like, and is partially cut off from the cell membrane and thus acts as an effector protein in the blood as well. Due to these characteristics, the hepatocyte growth factor/scatter factor having a site binding to HSPG has a low half-life in the blood. In an attempt to overcome this problem, research to remove the HSPG-binding site to thereby increase the half-life in the blood has been reported.

The therapeutic immunoglobulin antibody has a longer development period than a small molecule drug, and must be produced at a high concentration for administration to a patient. For this reason, determining the developability into a therapeutic drug enabling stability and solubility of the antibody has arisen as a big issue in the early stages of development. Cytotransmab is introduced into the cells through binding via electrostatic attraction between the negative charges of the HS chain of the extracellular receptor HSPG and the positively charged amino acid residues of the complementarity-determining regions (CDRs) of the cytotransmab antibody. It has been reported that the positively charged amino acid residues in the CDRs of antibodies adversely affect antibody stability, and when these positively charged amino acid residues are replaced with hydrophobic or negatively charged amino acids, the physical properties thereof are significantly improved.

After the immunoglobulin antibody binds to several pathogens in the body, it may penetrate into the cells along with the pathogens. The immunoglobulin antibody can bind to the TRIM21 protein in the cytoplasm to thus create an intracellular immune system. TRIM21 is a cytoplasmic protein belonging to the E3 ligase family, which binds to the CH2-CH3 region of immunoglobulins to form a complex with pathogens and immunoglobulins, wherein the complex is decomposed through the ubiquitin-proteasome mechanism. It has been reported that, when the binding between the immunoglobulin antibody and TRIM21 is inhibited, the immune system against the pathogen does not work, and that TRIM21 plays an important role in the degradation of the immunoglobulin antibody. In addition, the immunoglobulin antibody has an immune system that induces tumor death by inducing antibody-dependent cell-mediated cytotoxicity (ADCC) through binding to Fcγ receptors of immune cells in the blood. Each subclass of immunoglobulin has a different binding affinity to the Fcγ receptor, and specifically, IgG2 and IgG4 antibodies lack ADCC function due to very low affinity with the Fcγ receptor. In the same subclass of immunoglobulin, IgG2 and IgG4 have a longer *in-vivo* half-life than IgG1 due to the affinity with the Fcγ receptor.

Therefore, the present inventors established a heavy-chain variable region (VH) library based on the conventional anti-Ras·GTP iMab antibody (RT11), which directly inhibits intracellular Ras activity through cytoplasmic penetration, selected the heavy-chain variable region (VH) having improved affinity for Ras·GTP in the cytoplasm, and constructed an intact immunoglobulin-type antibody by simultaneously expressing the heavy-chain variable region (VH) with a light chain having a humanized light-chain variable region (VL) that penetrates into the living cells and is located in the cytoplasm, thereby producing an anti-Ras·GTP iMab antibody with an improved Ras-mutant-specific cell growth inhibitory effect.

In addition, in order to discover an intact immunoglobulin-type anti-Ras·GTP iMab antibody having tumor-tissue-specific cytoplasmic penetration ability, the present inventors developed a single domain of a light-chain variable region (VL) that reduces non-specific binding ability to HSPG and has improved stability and productivity even after fusing peptides for imparting tumor-tissue specificity thereto, and developed a tumor-tissue-specific anti-Ras·GTP iMab antibody with high productivity by simultaneously expressing a light chain including the light-chain variable region (VL) and a heavy-chain including a single domain of a heavy-chain variable region (VH) having binding ability to Ras·GTP with high specificity.

In addition, the present inventors produced an anti-Ras·GTP iMab antibody having an improved Ras-mutant-specific cell growth inhibitory effect by modifying the heavy chain to improve the intracellular stability, *in-vivo* persistence and tissue specificity of the intact immunoglobulin-type anti-Ras·GTP iMab antibody having tumor-tissue-specific cytoplasmic penetration ability.

In addition, the present inventors found that the tumor-tissue-specific anti-Ras·GTP iMab antibody penetrates into various Ras-mutant-dependent cancer cell lines, exhibits the Ras·GTP-specific binding ability in the cytoplasm, and has improved cell growth inhibition effect compared to the conventional anti-Ras·GTP iMab antibody. In addition, the present inventors found that tumor-tissue-specific anti-Ras·GTP iMab antibody reduces the HSPG-binding ability to impart tumor-tissue specificity, and exhibits a high level of production yield even when fused with integrin- or EpCAM-targeting protopeptides overexpressed in tumor tissues, and exhibits activity of specifically inhibiting Ras·GTP in Ras-mutant-dependent tumors without adverse effects on cytoplasmic penetration or inhibition of Ras·GTP activity. Based on this finding, the present disclosure has been completed.

### Prior Document

### Patent Document

Korean Patent No. 10-1602870

Korean Patent No. 10-1602876

### [Disclosure]

### [Technical Problem]

It is one object of the present disclosure to provide a method for improving the functions of antibodies in the form of an intact immunoglobulin, which bind to the membrane protein receptor on the cell surface overexpressed in tumor tissue, undergo endocytosis, and then are located in the cytoplasm by escaping from the endosome, and directly inhibit tumor mutant Ras activity by binding to intracellular Ras·GTP, and a method of producing the antibodies.

It is another object of the present disclosure to provide an amino acid sequence of a heavy-chain variable region that specifically binds to Ras activated by GTP bound thereto.

It is another object of the present disclosure to provide an intact immunoglobulin antibody including the heavy-chain variable region.

The antibody may have cytoplasmic penetration ability, and may include a specific sequence of amino acids for this purpose. In addition, the antibody may include specific amino acid sequences required to fuse the peptides targeting EpCAM (epithelial cell adhesion molecule), integrin αvβ3 or integrin αvβ5, as membrane proteins expressed on the surface of cancer cells, with the light-chain variable region or the heavy-chain variable region in order to improve the cancer-cell-targeting ability.

In addition, the antibody may include a heavy-chain constant region or a light-chain constant region derived from human immunoglobulin selected from the group consisting of IgA, IgD, IgE, IgG1, IgG2, IgG3, IgG4, and IgM.

In addition, in order to improve cancer-cell-targeting ability, the heavy-chain constant region of the antibody may include at least one mutation of N434D of the region CH3, and L234A, L235A or P329G of the CH2 region (the amino acid position is determined according to EU numbering) .

In addition, the antibody may be selected from the group consisting of single-chain Fvs (scFV), single- chain antibodies, Fab fragments, F(ab') fragments, disulfide-binding Fvs (sdFV) and epitope-binding fragments of the antibodies.

In addition, the antibody may be a bispecific antibody (bispecific Ab), and may be fused with any one or more selected from the group consisting of proteins, peptides, small molecule drugs, toxins, enzymes and nucleic acids.

It is another object of the present disclosure to provide a method for preparing an intact immunoglobulin-type antibody that has improved affinity for intracellular Ras·GTP and a tumor tissue-specific cytoplasmic penetration ability.

It is another object of the present disclosure to provide a composition for preventing or treating cancer including the antibody. The cancer may have a mutation with an activated intracellular Ras. In particular, the mutation of Ras may be a mutation in 12nd, 13rd or 61st amino acid of the Ras.

The prevention or treatment of cancer provided in the present disclosure is characterized in that the antibody provided in the present disclosure has a mechanism for inhibiting the binding of activated Ras (Ras·GTP) with B-Raf, C-Raf or PI3K in the cytoplasm.

It is another object of the present disclosure to provide a composition for diagnosing tumors including the antibody.

It is another object of the present disclosure to provide a polynucleotide encoding the antibody.

It is another object of the present disclosure to provide a heavy-chain variable-region library that specifically binds to Ras·GTP and has improved affinity therefor, and a method for constructing the same.

It is another object of the present disclosure to provide a method for screening a heavy-chain variable region that specifically binds to Ras·GTP and has improved affinity therefor.

### [Technical Solution]

In accordance with one aspect of the present disclosure, the above and other objects can be accomplished by the provision of a heavy-chain variable region specifically binding to Ras(Ras·GTP) activated by GTP bound thereto, including CDR1 having an amino acid sequence represented by the following Formula 1, CDR2 having an amino acid sequence represented by the following Formula 2, and CDR3 having an amino acid sequence represented by the following Formula 3:

[Formula 1] D-X₁₁-SMS

wherein X₁₁ is F or Y,

[Formula 2] YISRTSHT-X₂₁-X₂₂-YADSVKG

wherein X₂₁ is T, I or L, and X₂₂ is Y, C, S, L or A,

[Formula 3] G-F-X₃₁-X₃₂-X₃₃-Y

wherein X₃₁ is K, F, R or N, X₃₂ is M or L, and X₃₃ is D or N.

In another aspect, the present disclosure provides a method for improving the function of an antibody to inhibit the activity of intracellular Ras·GTP by actively penetrating, in the form of intact immunoglobulin, into the cytoplasm in living cells through endocytosis by tumor-tissue-specific cell membrane receptors and endosomal escape, and a method for preparing the antibody.

Hereinafter, the present disclosure will be described in detail.

The method of the present disclosure is capable of increasing the efficiency of tumor suppression specific for Ras mutant tumors through an intact immunoglobulin-type antibody having a heavy-chain variable region (VH), which exhibits improved affinity for intracellular Ras·GTP and thus high binding ability thereto, and a light-chain variable region (VL), which has cytoplasmic penetration ability specific for tumor tissues.

That is, the present disclosure aims at developing an intact immunoglobulin-type antibody that enables tumor-specific cytoplasmic penetration and binds to intracellular Ras·GTP with high affinity after cytoplasmic penetration to inhibit Ras activity.

The antibody may be an intact immunoglobulin-type antibody or a fragment thereof.

The antibody may be a chimeric, human or humanized antibody.

The heavy-chain variable region of the antibody according to the present disclosure includes CDR1 having the amino acid sequence represented by the following Formula 1, CDR2 having the amino acid sequence represented by the following Formula 2, and CDR3 having the amino acid sequence represented by the following Formula 3:

[Formula 1] D-X₁₁-SMS

wherein X₁₁ is F or Y,

[Formula 2] YISRTSHT-X₂₁-X₂₂-YADSVKG

wherein X₂₁ is T, I or L, and X₂₂ is Y, C, S, L or A,

[Formula 3] G-F-X₃₁-X₃₂-X₃₃-Y

wherein X₃₁ is K, F, R or N, X₃₂ is M or L, and X₃₃ is D or N.

Specifically, in an embodiment of the present disclosure, in Formula 1, defining CDR included in the heavy-chain variable region, X₁₁ is F or Y, in Formula 2, X₂₁-X₂₂ is TY, IY, TC, TS, IS, LC, LL or IA, and in Formula 3, X₃₁-X₃₂-X₃₃ is KMD, RMD, FMN, RLD or NLD.

Specifically, in one embodiment of the present disclosure, the heavy-chain variable region having improved affinity for intracellular Ras·GTP is a sequence including amino acid sequences selected from the group consisting of SEQ ID NOS: 20 to 32.

The sequence information of the heavy-chain variable region (VH) is as follows.

| VH name | Sequence | SEQ ID NO |
|---|---|---|
| RT21 VH | 10 20 30 40 50 52a EVQLVESGGGLVQPGGSLRLSCAASGFTFSDYSMSWVRQAPGKGLEWVSYISRTSHTTY 60 70 80 82a 90 100a 110 YADSVKGRFTISRDNSKNTLYLQMNSLRAEDTAVYYCARGFKMDYWGQGTLVTVSS | 20 |
| RT22 VH | 10 20 30 40 50 52a EVQLVESGGGLVQPGGSLRLSCAASGFTFSDFSMSWVRQAPGKGLEWVSYISRTSHTTY 60 70 80 82a 90 100a 110 YADSVKGRFTISRDNSKNTLYLQMNSLRAEDTAVYYCARGFKMDYWGQGTLVTVSS | 21 |
| RT23 VH | 10                    20                    30                    40                    50 52a EVQLVESGGGLVQPGGSLRLSCAASGFTFSDYSMSWVRQAPGKGLEWVSYISRTSHTTY 60                    70                    80 82a                    90                    100a                    110 YADSVKGRFTISRDNSKNTLYLQMNSLRAEDTAVYYCARGFRMDYWGQGTLVTVSS | 22 |
| RT24 VH | 10                    20                    30                    40                    50 52a EVQLVESGGGLVQPGGSLRLSCAASGFTFSDFSMSWVRQAPGKGLEWVSYISRTSHTTY 60                    70                    80 82a                    90                    100a                    110 YADSVKGRFTISRDNSKNTLYLQMNSLRAEDTAVYYCAKGFFMNYWGQGTLVTVSS | 23 |
| RT25 VH | 10 20 30 40 50 52a EVQLVESGGGLVQPGGSLRLSCAASGFTFSDFSMSWVRQAPGKGLEWVSYISRTSHTTY 60 70 80 82a 90 100a 110 YADSVKGRFTISRDNSKNTLYLQMNSLRAEDTAVYYCARGFRMDYWGQGTLVTVSS | 24 |
| RT31 VH | 10 20 30 40 50 52a EVQLVESGGGLVQPGGSLRLSCAASGFTFSDFSMSWVRQAPGKGLEWVSYISRTSHTIY 60 70 80 82a 90 100a 110 YADSVKGRFTISRDNSKNTLYLQMNSLRAEDTAVYYCARGFKMDYWGQGTLVTVSS | 26 |
| RT32 VH | 10 20 30 40 50 52a EVQLVESGGGLVQPGGSLRLSCAASGFTFSDFSMSWVRQAPGKGLEWVSYISRTSHTTC 60 70 80 82a 90 100a 110 YADSVKGRFTISRDNSKNTLYLQMNSLRAEDTAVYYCARGFKMDYWGQGTLVTVSS | 27 |
| RT33 VH | 10 20 30 40 50 52a EVQLVESGGGLVQPGGSLRLSCAASGFTFSDFSMSWVRQAPGKGLEWVSYISRTSHTTS 60 70 80 82a 90 100a 110 YADSVKGRFTISRDNSKNTLYLQMNSLRAEDTAVYYCARGFKMDYWGQGTLVTVSS | 28 |
| RT34 VH | 10 20 30 40 50 52a EVQLVESGGGLVQPGGSLRLSCAASGFTFSDFSMSWVRQAPGKGLEWVS YISRTSHTIS 60 70 80 82a 90 100a 110 YADSVKGRFTISRDNSKNTLYLQMNSLRAEDTAV YYCARGFKMDYWGQGTLVTVSS | 29 |
| RT35 VH | 10 20 30 40 50 52a EVQLVESGGGLVQPGGSLRLSCAASGFTFSDFSMSWVRQAPGKGLEWVSYISRTSHTLC 60 70 80 82a 90 100a 110 YADSVKGRFTISRDNSKNTLYLQMNSLRAEDTAVYYCARGFRLDYWGQGTLVTVSS | 30 |
| RT36 VH | 10 20 30 40 50 52a EVQLVESGGGLVQPGGSLRLSCAASGFTFSDFSMSWVRQAPGKGLEWVSYISRTSHTLL 60 70 80 82a 90 100a 110 YADSVKGRFTISRDNSKNTLYLQMNSLRAEDTAVYYCARGFNLDYWGQGTLVTVSS | 31 |
| RT37 VH | 10          20          30          40          50 52a EVQLVESGGGLVQPGGSLRLSCAASGFTFSDFSMSWVRQAPGKGLEWVSYISRTSHTIA 60          70          80 82a          90          100a          110 YADSVKGRFTISRDNSKNTLYLQMNSLRAEDTAVYYCARGFKMDYWGQGTLVTVSS | 32 |

Regarding the heavy-chain variable region having improved affinity for intracellular Ras·GTP, the CRD1 sequence is selected from the group consisting of amino acid sequences represented by SEQ ID NOS: 2 to 4, and the CDR2 sequence is selected from the group consisting of amino acid sequences represented by SEQ ID NO: 5 and SEQ ID NOS: 10 to 16, and the CDR3 sequence is selected from the group consisting of amino acid sequences represented by SEQ ID NOS: 6 to 9 and SEQ ID NOS: 17 to 18.

The sequence information of the CDR is as follows:

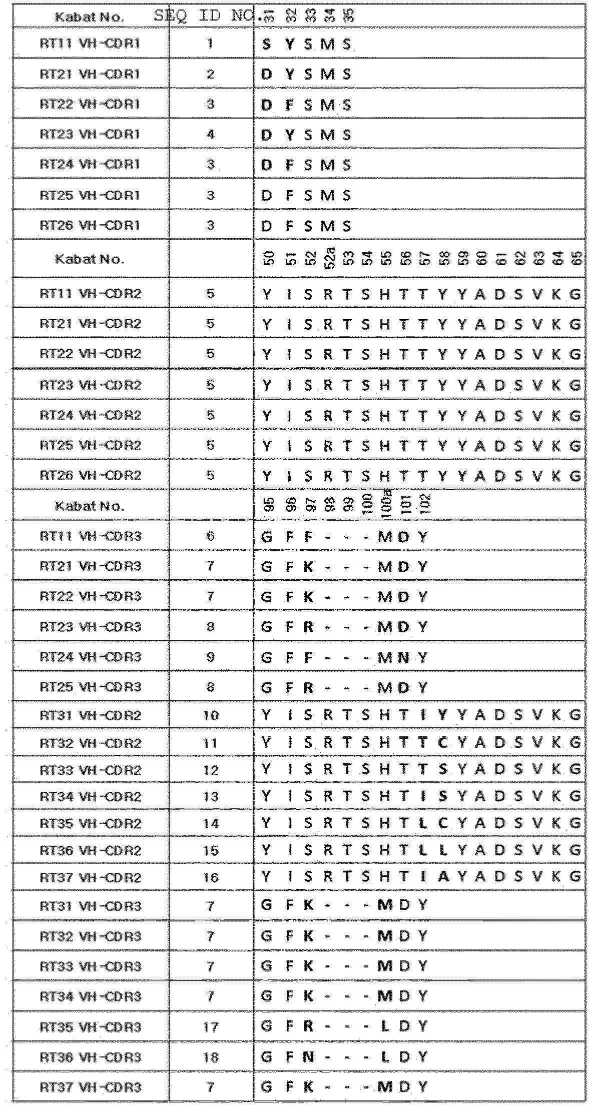

However, all amino acid numbers specified in Sequence numbers excluding the heavy-chain constant region and X₁₁, X₂₁-X₂₂ and X₃₁-X₃₂-X₃₃ included in the Formulas 1, 2 and 3 defining the CDRs provided herein were Kabat numbers (Kabat EA et al., 1991).

In addition, in another aspect, the present disclosure provides a light-chain variable region having cytoplasmic penetration ability specific for tumor cells and inhibited binding ability to HSPG, wherein the light-chain variable region may include an amino acid sequence selected from the group consisting of SEQ ID NOS: 34 to 43 and SEQ ID NOS: 44 to 60.

The sequence information of the light-chain variable region (VL) for this purpose is as follows.

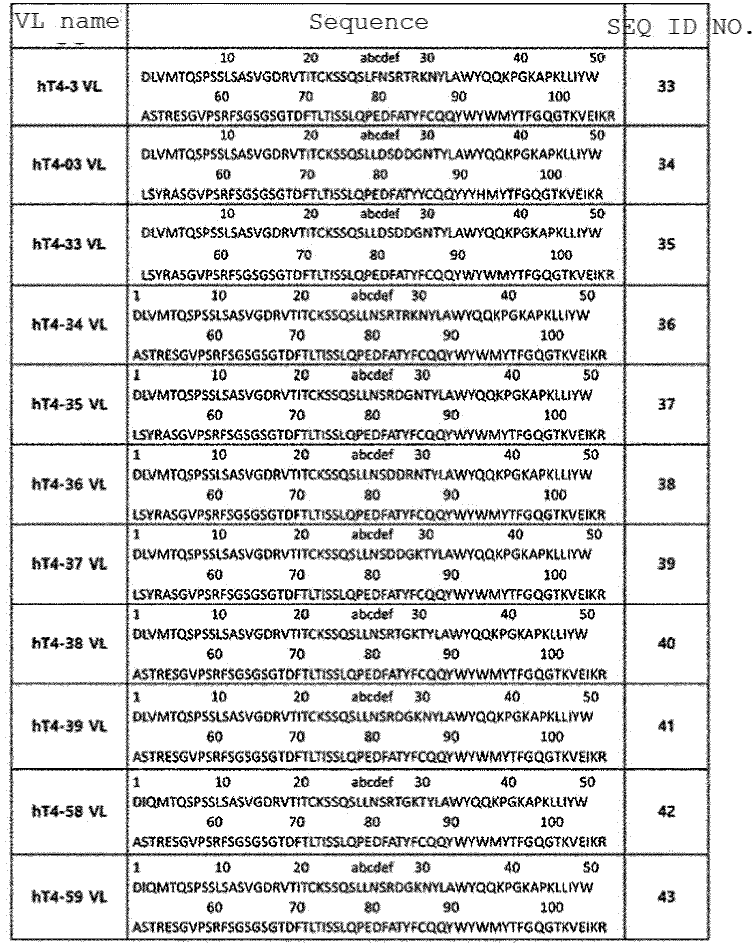

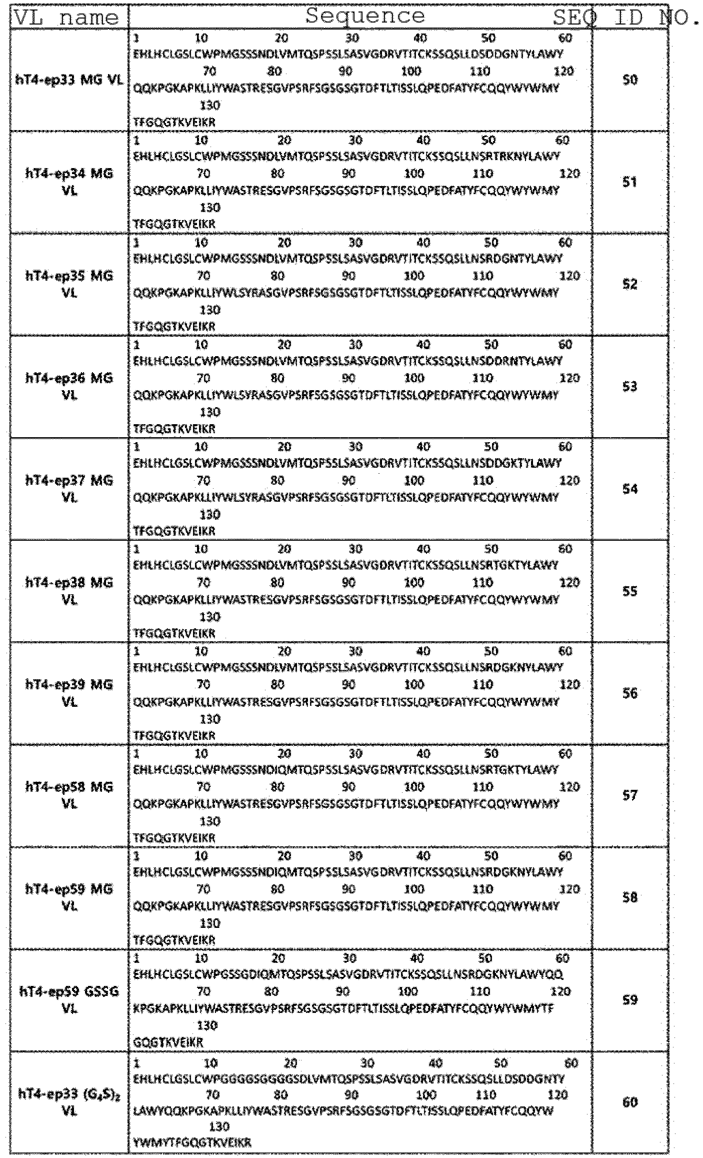

In addition, in one embodiment of the present disclosure, the heavy-chain variable region modified (improved) to improve tumor-tissue-targeting ability is a sequence including amino acid sequences selected from the group consisting of SEQ ID NOS: 61 to 63.

The sequence information of the heavy-chain variable region (VH) is as follows.

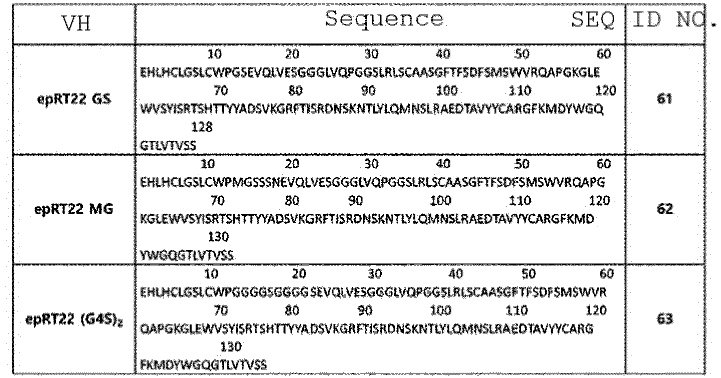

In addition, in one embodiment of the present disclosure, the heavy-chain constant region to improve the intracellular stability of the antibody having the tumor-tissue-specific cytoplasmic penetration antibody and to impart a long half-life thereto is a sequence including an amino acid sequence selected from the group consisting of SEQ ID NOS: 65 to 69.

The sequence information of the heavy-chain constant region (CH1-CH2-CH3) for this purpose is as follows.

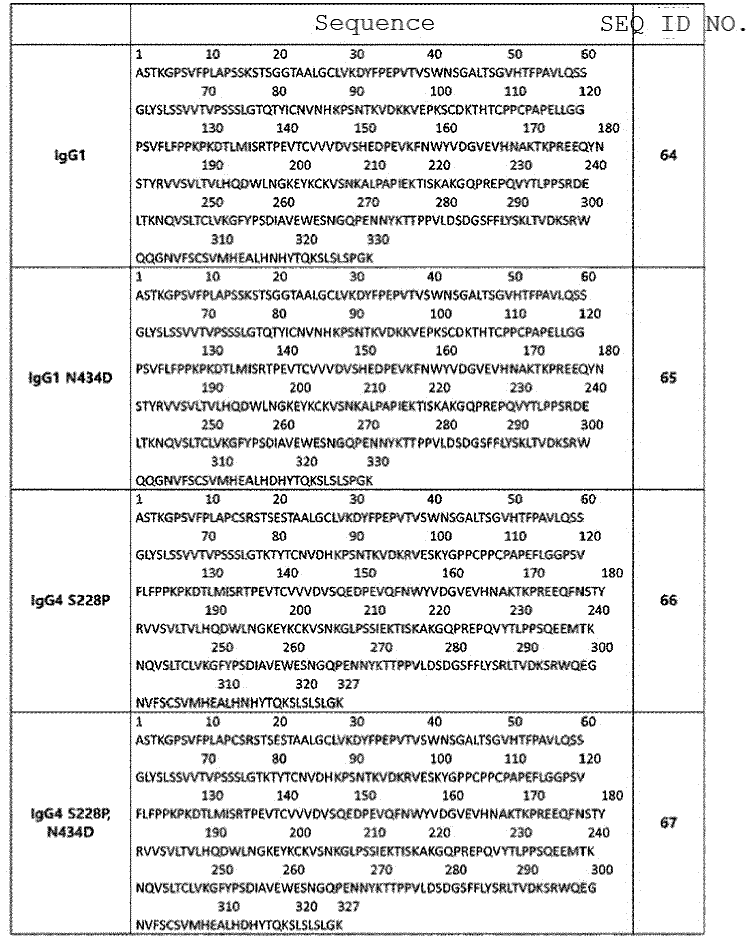

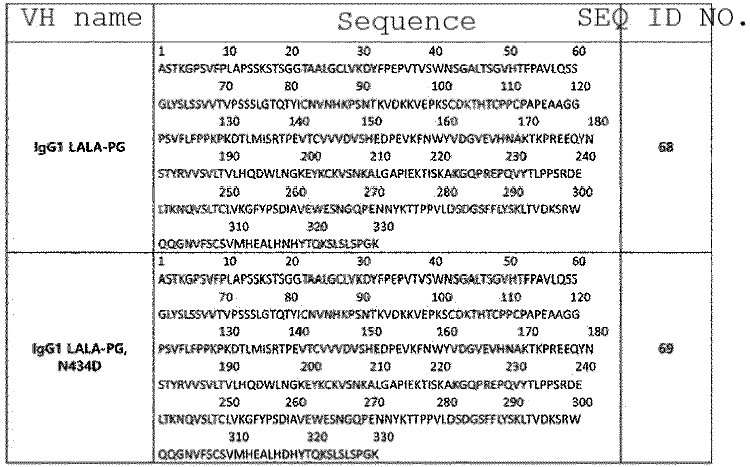

In addition, in another aspect, the present disclosure provides a method for constructing a heavy-chain variable region library that specifically binds to Ras·GTP and has improved affinity therefor.

The method includes:
(1) determining an amino acid site having high potential to bind to intracellular Ras·GTP among three complementarity determining regions (CDRs) involved in antigen binding of a RT11 heavy-chain variable region (VH) as a library template;
(2) designing a degenerated codon primer capable of encoding an amino acid in need of inclusion in a library at the determined amino acid site; and
(3) expressing the designed heavy-chain variable-region library in the form of scFab or Fab using a yeast surface expression system.

In addition, in another aspect, the present disclosure provides a method for screening a heavy-chain variable region that specifically binds to Ras·GTP and has improved affinity therefor,
the method including:
(1) expressing a heavy-chain variable-region library capable of binding to Ras·GTP using a yeast surface expression system;
(2) constructing Avi-KRas^{G12D} bound to GppNHp, a GTP analogue, in a stable form without deformation during biotinylation;
(3) binding the library with the GppNHp-bound Avi-KRas^{G12D}; and
(4) measuring the affinity of the binding between the library and the GppNHp-bound Avi-KRas^{G12D}.

In addition, in another aspect, the present disclosure provides an intact immunoglobulin antibody including the antibody fused with one or more selected from the group consisting of proteins, peptides, small molecule drugs, toxins, enzymes, nucleic acids and nanoparticles.

The proteins include antibodies, fragments of antibodies, immunoglobulins, peptides, enzymes, growth factors, cytokines, transcription factors, toxins, antigenic peptides, hormones, transport proteins, motor function proteins, receptors, signaling proteins, storage proteins, membrane proteins, transmembrane proteins, internal proteins, external proteins, secreted proteins, viral proteins, sugar proteins, truncated proteins, protein complexes, chemically modified proteins and the like.

The term "small molecule drug" refers to an organic compound, an inorganic compound or an organometallic compound that has a molecular weight of less than about 1,000 daltons and has activity as a therapeutic agent for diseases, which is widely used herein. The small molecule drug used herein includes oligopeptides and other biomolecules having a molecular weight of less than about 1,000 daltons.

As used herein, the term "nanoparticle" refers to a particle including a material having a diameter of 1 to 1,000 nm, and the nanoparticle may be a metal/metal core-shell complex including a metal nanoparticle, a metal nanoparticle core and a metal shell including the core, a metal/non-metal core-shell complex including a metal nanoparticle core and a non-metal shell surrounding the core, or a nonmetal/metal core-shell complex including a nonmetal nanoparticle core and a metal shell surrounding the core. According to one embodiment, the metal may be selected from gold, silver, copper, aluminum, nickel, palladium, platinum, magnetic iron and oxides thereof, but is not limited thereto, and the nonmetal may be selected from silica, polystyrene, latex and acrylic substances, but is not limited thereto.

As used herein, the term "fusion" refers to the integration of two molecules having different or identical functions or structures, and includes fusion through any physical, chemical or biological method capable of binding the tumor specific-cytoplasmic penetration antibody to the protein, small-molecule drug, nucleic acid or nanoparticle. The fusion may preferably be carried out using a linker peptide, and the linker peptide may mediate the fusion with the bioactive molecule at various positions of the antibody light-chain variable region according to the present disclosure, antibody, or fragment thereof.

In addition, the present disclosure provides a pharmaceutical composition for preventing or treating cancer containing a biologically active molecule selected from the group consisting of the antibody, or a peptide, protein, small molecule drug, nucleic acid and nanoparticle fused thereto.

The biologically active molecule selected from the group consisting of the antibody, or a peptide, protein, small molecule drug, nucleic acid and nanoparticle fused thereto, enables intercellular permeation and remains in the cytoplasm without affecting antibody specificity and high affinity of the human antibody heavy-chain variable region (VH) and thus is expected to be highly effective in the treatment and diagnosis of tumor- and disease-related factors, which are classified as target substances for the treatment of diseases using small molecule drugs and are present in the cytoplasm and form structurally complex interactions through a wide and flat surface between protein and protein.

In another aspect, the present disclosure provides a method of inhibiting the growth of cancer or tumor cells using the antibody and a method of treating cancer or tumors.

The cancer may be selected from the group consisting of squamous cell carcinoma, small cell lung cancer, non-small cell lung cancer, adenocarcinoma of the lungs, squamous cell carcinoma of the lungs, peritoneal cancer, skin cancer, skin or ocular melanoma, rectal cancer, anal cancer, esophageal cancer, small intestine cancer, endocrine cancer, parathyroid cancer, adrenal cancer, soft tissue sarcoma, urethral cancer, chronic or acute leukemia, lymphoma, hepatoma, gastrointestinal cancer, pancreatic cancer, glioblastoma, cervical cancer, ovarian cancer, liver cancer, bladder cancer, liver tumor, breast cancer, colon cancer, colorectal cancer, endometrial cancer or uterine cancer, salivary gland cancer, kidney cancer, liver cancer, prostate cancer, vulva cancer, thyroid cancer, liver cancer and head and neck cancer.

When the composition is prepared as a pharmaceutical composition for preventing or treating cancer, the composition may include a pharmaceutically acceptable carrier. The pharmaceutically acceptable carrier included in the composition is conventionally used in the preparation of a drug, and includes, but is not limited to, lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methylhydroxy benzoate, propylhydroxy benzoate, talc, magnesium stearate, mineral oil and the like. The pharmaceutical composition may further include a lubricant, a wetting agent, a sweetener, a flavoring agent, an emulsifier, a suspending agent, a preservative or the like, in addition to the above ingredients.

The pharmaceutical composition for preventing or treating cancer may be administered orally or parenterally. The parenteral administration may be intravenous injection, subcutaneous injection, intramuscular injection, intraperitoneal injection, endothelial administration, topical administration, intranasal administration, pulmonary administration or rectal administration. Upon oral administration, proteins or peptides are digested, so that an oral composition may be coated with an active drug or formulated so as to protect the same from degradation in the stomach. In addition, the composition may be administered using any device capable of delivering the active substance to target cells.

The suitable dose of the pharmaceutical composition for preventing or treating cancer may vary depending on factors such as the formulation method, administration method, and age, body weight, gender, pathological conditions, diet, administration time, administration route, excretion rate and responsiveness of the patient. For example, the suitable dose of the composition may be within the range of 0.001 to 100 mg/kg for an adult. The term "pharmaceutically effective amount" may mean an amount sufficient to prevent or treat cancer or to prevent or treat neovascular diseases.

The composition may be prepared into a unit dose form, or may be incorporated into a multi-dose container through formulation using a pharmaceutically acceptable carrier and/or excipient according to a method that can be easily implemented by those skilled in the art to which the present disclosure pertains. In this case, the formulation may be in the form of a solution, a suspension, a syrup or an emulsion in an oil or aqueous medium, or may be formulated in the form of an extract, a powder, a granule, a tablet or a capsule. The composition may further include a dispersant or a stabilizer. In addition, the composition may be administered alone or in combination with other therapeutics. In this case, the composition of the present disclosure may be administered sequentially or simultaneously with conventional therapeutics. Meanwhile, since the composition includes an antibody or an antigen-bonding fragment thereof, it can be formulated as an immunoliposome. Liposomes including the antibody can be prepared according to a method well-known in the art. The immunoliposome can be prepared through reverse phase evaporation in the form of a lipid composition including phosphatidylcholine, cholesterol and polyethyleneglycol-derived phosphatidyl ethanolamine. For example, Fab' fragments of antibodies can be fused to liposomes via disulfide interchange reaction. A chemotherapeutic agent such as doxorubicin may further be included in the liposomes.

In addition, in another aspect, the present disclosure provides a composition for the diagnosis of cancer containing a biologically active molecule selected from the group consisting of the antibody and a peptide, protein, small molecule drug, nucleic acid or nanoparticle fused thereto.

As used herein, the term "diagnosis" means determining the presence or features of pathophysiology. In the present disclosure, diagnosis serves to determine the onset or progress of cancer.

The intact immunoglobulin antibody or fragment thereof may bind to a phosphor for molecular imaging to diagnose cancer through imaging.

The phosphor for molecular imaging is any material that generates fluorescence, and preferably emits red or near-infrared fluorescence, and is more preferably a phosphor having a high quantum yield, but is not limited thereto.

The phosphor for molecular imaging is preferably, but without limitation, a phosphor, a fluorescent protein, or other imaging material capable of specifically binding to the intact immunoglobulin antibody or a fragment thereof.

The phosphor is preferably fluorescein, BODYPY, tetramethylrhodamine, Alexa, cyanine, allophycocyanin or derivatives thereof, but is not limited thereto.

The fluorescent protein is preferably a Dronpa protein, a fluorescent color gene (EGFP), a red fluorescent protein (DsRFP), a cy5.5 phosphor, which exhibits near-infrared cyanine fluorescence, or other fluorescent protein, but is not limited thereto.

Other imaging materials are preferably iron oxide, radioisotopes and the like, but are not limited thereto, and may be applied to imaging equipment such as MR and PET.

In addition, the present disclosure provides a polynucleotide encoding an antibody including a heavy-chain variable region (VH) having improved affinity for intracellular Ras·GTP and a light-chain variable region (VL) having cytoplasmic penetration ability specific for tumor tissues.

As used herein, the term "polynucleotide" refers to a polymer of deoxyribonucleotide or ribonucleotide present in single- or double-stranded form, encompasses RNA genome sequences and DNA (gDNA and cDNA) and RNA sequences transcribed therefrom, and includes analogues of naturally derived polynucleotides, unless mentioned otherwise.

The polynucleotide includes not only nucleotide sequences encoding the heavy-chain variable region (VH) having improved affinity for intracellular Ras·GTP, the light-chain variable region (VL) having cytoplasmic penetration ability specific for tumor tissues, and the heavy-chain constant region (CH1-CH2-CH3) to impart intracellular stability and long half-life, but also sequences complementary to the sequences. Such complementary sequences include completely complementary sequences as well as substantially complementary sequences. This refers to a sequence that can hybridize with the nucleotide sequence encoding any one amino acid sequence of SEQ ID NOS: 20 to 69 under stringent conditions known in the art.

The polynucleotide can be varied. Such variation includes addition, deletion, or non-conservative or conservative substitution of nucleotides. The polynucleotide encoding the amino acid sequence is interpreted to include a nucleotide sequence having substantial identity with the nucleotide sequence. The expression "nucleotide sequence having substantial identity" means a nucleotide sequence that has a homology of at least 80%, more preferably a homology of at least 90%, and most preferably a homology of at least 95%, when aligning the nucleotide sequence of the present disclosure with any other sequence so as to correspond thereto as much as possible and analyzing the aligned sequence using algorithms commonly used in the art.

In another aspect, the present disclosure provides a method for preparing an intact immunoglobulin-type antibody that penetrates into cells and is disposed in the cytoplasm using a heavy-chain variable region (VH) having improved affinity for intracellular Ras·GTP and a light-chain variable region (VL) having cytoplasmic penetration ability specific for tumor tissues, the method comprising:
(1) preparing an endosomal escape heavy-chain expression vector cloned with nucleic acids, substituted with a humanized heavy-chain variable region (VH) having improved affinity for intracellular Ras·GTP from a heavy-chain variable region (VH) included in a heavy chain comprising a human heavy-chain variable region (VH) and a human heavy-chain constant region (CH1-hinge-CH2-CH3);
(2) preparing a cytoplasmic penetration light-chain expression vector cloned with nucleic acids, substituted with a humanized light-chain variable region (VL) having cytoplasmic penetration ability and a humanized light-chain variable region (VL) having cytoplasmic penetration ability specific for tumor tissues from a light-chain variable region (VL) included in a light chain comprising a human light-chain variable region (VL) and a human light-chain constant region (CL) ;
(3) co-transforming the prepared heavy- and light-chain expression vectors into animal cells for protein expression to express an intact immunoglobulin-type antibody including a heavy-chain variable region (VH) having improved affinity for intracellular Ras·GTP and a light-chain variable region (VL) having cytoplasmic penetration ability specific for tumor tissues; and
(4) purifying and recovering the expressed intact immunoglobulin-type antibody.

The method can express a vector expressing a heavy chain and a vector expressing a light chain, thereby specifically expressing an antibody that penetrates into cells specifically for tumor tissues and is located in the cytoplasm and is capable of targeting intracellular Ras/GTP with high affinity. The vector may be a vector system that simultaneously expresses the light and heavy chains in one vector or a system that independently expresses the chains in separate vectors. In the latter case, both vectors can be introduced into the host cells through co-transformation and targeted transformation.

The light-chain variable region (VL), light-chain constant region (CL), heavy-chain variable region (VH) and heavy-chain constant region (CH1-hinge-CH2-CH3) provided by the present disclosure in the recombinant vector are operatively linked to a promoter. The term "operatively linked" means functional linkage between a nucleotide expression control sequence (e.g., a promoter sequence) and another nucleotide sequence, which enables the control sequence to regulate transcription and/or translation of the other nucleotide sequence.

The recombinant vector can typically be constructed as a vector for cloning or a vector for expression. The vector for expression may be a conventional vector used in the art to express foreign proteins in plants, animals or microorganisms. The recombinant vector may be constructed through any of various methods known in the art.

The recombinant vector may be constructed using prokaryotic or eukaryotic cells as hosts. For example, when a vector that is used is an expression vector and a prokaryotic cell is used as a host, the vector generally includes a potent promoter capable of conducting transcription (such as a pLλ promoter, trp promoter, lac promoter, tac promoter, T7 promoter, etc.), a ribosome-binding site to initiate translation, and a transcription/translation termination sequence. In addition, for example, when a eukaryotic cell is used as a host, the replication origin that operates in eukaryotic cells included in the vector includes, but is not limited to, an f1 replication origin, a SV40 replication origin, a pMB1 replication origin, an adeno replication origin, an AAV replication origin, a CMV replication origin, a BBV replication origin and the like. Also, a promoter derived from the genome of mammalian cells (e.g., a metallothionein promoter), or a promoter derived from a mammalian virus (e.g., adenovirus late promoter, vaccinia virus 7.5K promoter, SV40 promoter, cytomegalovirus (CMV) promoter, or HSV tk promoter) may be used, and the vector generally has a polyadenylation sequence as a transcription termination sequence.

In another aspect, the present disclosure provides a host cell transformed with the recombinant vector.

The host cell may be any host cell well-known in the art, and in the case of transforming prokaryotic cells, includes, for example, *E. coli* JM109, *E. coli* BL21, *E. coli* RR1, *E. coli* LE392, *E. coli* B, *E. coli* X 1776, *E. coli* W3110, strains of the genus *Bacillus,* such as *Bacillus subtilis* and *Bacillus thuringiensis,* and enterococci and strains such as *Salmonella typhimurium, Serratia marcescens* and various *Pseudomonas* species. In the case of transforming eukaryotic cells, as host cells, yeast (*Saccharomyces cerevisiae*), insect cells, plant cells and animal cells such as SP2/0, Chinese hamster ovary K1, CHO DG44, PER.C6, W138, BHK, COS-7, 293, HepG2, Huh7, 3T3, RIN and MDCK cell lines and the like can be used.

In another aspect, the present disclosure provides a method for producing an intact immunoglobulin-type antibody that penetrates specifically into tumor tissue cells and is located in the cytoplasm and is thus capable of targeting intracellular Ras/GTP with high affinity, the method including culturing the host cell.

Insertion of the recombinant vector into the host cell may be carried out using an insertion method well-known in the art. The delivery method may be a CaCl₂ method or an electroporation method, for example, when the host cell is a prokaryotic cell. When the host cell is a eukaryotic cell, the delivery method may be micro-injection, calcium phosphate precipitation, electroporation, liposome-mediated transfection, gene bombardment or the like, but is not limited thereto. The use of microorganisms such as *E. coli* realizes higher productivity than when using animal cells, but is not suitable for the production of intact Ig-type antibodies due to the problem of glycosylation, and is suitable for the production of antigen-binding fragments such as Fab and Fv.

The method for selecting the transformed host cell can be easily carried out according to methods well-known in the art using a phenotype expressed by a selection marker. For example, when the selection marker is a gene specific for antibiotic resistance, the transformant can be easily selected by culturing the transformant in a medium containing the antibiotic.

### [Description of Drawings]

FIG. 1 is a schematic diagram showing the strategy for selection and construction of a library based on the heavy-chain variable region of RT11 in order to improve the affinity of anti-Ras·GTP iMab to Ras·GTP.
FIG. 2A shows the result of FACS analysis to determine the binding ability of library expression yeast in each step with 10 nM Avi-KRas^{G12D}·GppNHp or 1000 nM Avi-KRas^{G12D}·GDP in order to confirm the specific enrichment in Avi-KRas^{G12D}·GppNHp through the selection process using MACS (magnetic-activated cell sorting) and FACS (fluorescence activated cell sorting).
FIG. 2B shows the result of flow cytometry to determine the binding ability of the yeast expressing 47 individual clones in the finally selected library with 5 nM Avi-KRas^{G12D}·GppNHp.
FIG. 3A is a schematic diagram illustrating the construction of complete IgG-type anti-Ras·GTP iMab (RT22-i3) fused with integrin-targeting protopeptide and having improved affinity for Ras·GTP based on RT11.
FIG. 3B shows the result of ELISA to analyze the binding ability of the RT11-based affinity-improved anti-Ras·GTP iMabs with 1, 10 or 100 nM Avi-KRas^{G12D}·GppNHp and 100 nM Avi-KRaS^{G12D}·GDP in order to determine the specific binding of the anti-Ras·GTP iMabs with GppNHp-bound Avi-KRas^{G12D}.
FIG. 3C shows the result of confocal microscopy analysis to determine the overlap with the activated intracellular Ras after treating the KRas mutant colorectal cancer cell line SW480 at 37°C for 12 hours with 1 µM of the anti-Ras·GTP iMabs having improved affinity therefor.
FIG. 4 is a schematic diagram showing a strategy for selecting and constructing a library based on the heavy-chain variable region of RT22 in order to improve the affinity of anti-Ras·GTP iMab for Ras·GTP.
FIG. 5A shows the result of 12% SDS-PAGE analysis under reducing or non-reducing conditions after purification of anti-RasGTP·iMab including a combination of the RT22-based affinity-improved heavy-chain variable regions (VH) with the light-chain variable region (hT4-i33) fused with the integrin-targeting protopeptide and having inhibited HSPG-binding ability.
FIG. 5B shows the result of ELISA analysis of the binding ability of each anti-Ras·GTP iMab to 1, 10 or 100 nM Avi-KRas^{G12D}·GppNHp or to 100 nM Avi-KRas^{G12D}·GDP to determine specific binding between GppNHp-bound Avi-KRas^{G12D} and anti-Ras·GTP iMabs including a combination of the RT22-based affinity-improved heavy-chain variable regions (RT31 VH, RT33 VH, RT34 VH) with the light-chain variable region (hT4-i33) fused with integrin-targeting protopeptide and having inhibited HSPG-binding ability.
FIG. 5C shows the result of ELISA analysis of binding ability of each anti-Ras·GTP iMab to 1, 10 or 100 nM Avi-KRas^{G12D}·GppNHp or 100 nM Avi-KRas^{G12D}·GDP to determine specific binding between GppNHp-bound Avi-KRas^{G12D} and anti-Ras·GTP iMabs including a combination of the RT22-based affinity-improved heavy-chain variable regions (RT31 VH, RT36 VH, RT37 VH) with the light-chain variable region (hT4-i33) fused with integrin-targeting protopeptide and having inhibited HSPG-binding ability.
FIG. 6 is a schematic diagram illustrating the construction of complete IgG-type anti-Ras·GTP iMabs (RT22-i33, RT22-ep33) having improved affinity including a light-chain variable region (VL) fused with integrin- or EpCAM-targeting protopeptides and having inhibited binding ability to HSPG.
FIG. 7 shows the result of confocal microscopy analysis to determine the overlap with the activated intracellular Ras after treating the KRas mutant colorectal cancer cell line SW480 with complete IgG-type anti-Ras·GTP iMabs (RT22-33 (1 µM), RT22-i33 MG (0.5 µM), RT22-ep33 MG (1 µM)) having improved affinity including a light-chain variable region (VL) fused with integrin- or EpCAM-targeting protopeptide and having inhibited binding ability to HSPG, and with cytotransmab as control groups (CT-33 (1 µM), CT-i33 MG (0.5 µM), CT-ep33 MG (1 µM)).
FIG. 8A shows the result of confocal microscopy performed after treatment of HeLa cells with 1 µM of the antibody at 37°C for 6 hours to determine a decrease in, or removal of HSPG-binding ability and cytoplasmic penetration of cytotransmab and the anti-Ras·GTP iMab including the light-chain variable region introduced with mutants of CDR1 and CDR2 for improving production yield and inhibiting HSPG-binding ability, and is a bar graph showing quantified Alexa488 fluorescence (green fluorescence).
FIG. 8B shows the result of non-specific cell-surface-binding ELISA in the HeLa cell line expressing HSPG to determine the HSPG-binding ability of cytotransmab and the anti-Ras ·GTP iMab including the light-chain variable region introduced with mutants of CDR1 and CDR2 for improving production yield and inhibiting HSPG-binding ability.
FIG. 8C shows the results of flow cytometry using FACS after treatment of the HeLa cell line with 500 nM antibody to determine the HSPG-binding ability of the anti-Ras·GTP iMab including the light-chain variable region introduced with mutants of CDR1 and CDR2 for improving production yield and inhibiting HSPG-binding ability.
FIG. 9A shows the result of ELISA to analyze the binding ability to 1, 10 or 100 nM Avi-KRas^{G12D}·GppNHp or 100 nM Avi-KRas^{G12D}·GDP of anti-Ras·GTP iMab including light-chain variable regions (hT4-i33 MG∼hT4-i37 MG VL) fused with integrin-targeting protopeptide and having inhibited binding ability to HSPG.
FIG. 9B shows the result of ELISA to analyze the binding ability to 1, 10 or 100 nM Avi-KRas^{G12D}·GppNHp or 100 nM Avi-KRas^{G12D}·GDP of cytotransmab and anti-Ras·GTP iMabs including light-chain variable regions (hT4-i38 MG to hT4-i39 MG VL) fused with integrin-targeting protopeptide and having inhibited binding ability to HSPG.
FIG. 9C shows the result of confocal microscopy analysis to determine the overlap with the activated intracellular Ras after treating the KRas mutant colorectal cancer cell line SW480 at 37°C for 12 hours with 1 µM of cytotransmab and anti-Ras·GTP iMabs including light-chain variable regions (VL) fused with integrin- or EpCAM-targeting protopeptide and having inhibited binding ability to HSPG.
FIG. 10A shows the result of ELISA to analyze the binding ability to 1, 10 or 100 nM Avi-KRas^{G12D}·GppNHp or 100 nM Avi-KRas^{G12D}·GDP of anti-Ras·GTP iMabs including light-chain variable regions (hT4-ep58 MG to hT4-ep59 MG VL) fused with EpCAM-targeting protopeptide and a modified antibody framework to improve the production yield based on hT4-38 and hT4-39.
FIG. 10B shows the result of confocal microscopy analysis to determine the overlap with the activated intracellular Ras after treating the KRas mutant colorectal cancer cell line SW480 at 37°C for 12 hours with 1 µM of anti-Ras·GTP iMabs (RT22-ep58, RT22-ep59) including light chains (hT4-58, hT4-59) fused with EpCAM-targeting protopeptide and having a modified antibody skeleton to improve the production yield based on hT4-38 and hT4-39.
FIG. 11A shows the result of confocal microscopy performed after treatment of HeLa cells with 1 µM of the antibody at 37°C for 6 hours to determine a decrease in, or the removal of, HSPG-binding ability and cytoplasmic penetration of cytotransmab and anti-Ras·GTP iMabs including light-chain variable regions introduced with mutants of CDR1 and CDR2 for improving production yield and inhibiting HSPG-binding ability, and is a bar graph showing quantified Alexa488 fluorescence (green fluorescence).
FIG. 11B shows the result of non-specific cell-surface-binding ELISA in the HeLa cell line expressing HSPG to determine the HSPG-binding ability of cytotransmab and the anti-Ras·GTP iMabs including the light-chain variable regions introduced with mutants of CDR1 and CDR2 for improving production yield and inhibiting HSPG-binding ability.
FIG. 11C shows the result of flow cytometry using FACS after treatment of the HeLa cell line with 500 nM of the antibody to determine the HSPG-binding ability of cytotransmab and the anti-Ras·GTP iMabs including the light-chain variable regions introduced with mutants of CDR1 and CDR2 for improving production yield and inhibiting HSPG-binding ability.
FIG. 12A shows the result of a test using FACS to identify the expression of integrin αvβ3 or integrin αvβ5 in human colorectal cancer cell lines SW480 and LoVo, and human blood cancer cell lines wild-type K562 and integrin αvβ3-expressing K562.
FIG. 12B shows the result of a test using FACS to determine the binding ability to cell-surface integrin αvβ3 or integrin αvβ5 of anti-Ras cell-penetrating antibodies specific for tumor tissue integrin (RT22-i38 MG and RT22-i39 MG), having no HSPG-binding ability and including a heavy chain (RT22) having improved affinity for Ras·GTP, and of conventional anti-Ras cell-penetrating antibodies specific for tumor tissue integrin (RT11-i).
FIG. 12C shows the result of a test using FACS to determine the expression of EpCAM in the human colorectal cancer cell lines SW480 and LoVo and the human cervical cancer cell line HeLa.
FIG. 12D shows the result of a test using FACS to determine the binding ability to the cell surface EpCAM of tumor-tissue EpCAM-specific anti-Ras cell-penetrating antibodies (RT22-ep58 MG and RT22-ep59 MG) having no HSPG-binding ability and including a heavy chain (RT22) having improved affinity for Ras·GTP.
FIG. 13A is a graph showing cell growth inhibition ability, determined through WST assay, after treatment at 37°C for 48 hours with 0.5 µM of anti-Ras·GTP iMab combined with a light-chain variable region fused with integrin-targeting protopeptide and having improved production yield and inhibited HSPG-binding ability in several Ras mutant and wild-type cell lines.
FIG. 13B is a graph showing cell growth inhibition ability, determined by WST assay, after treatment at 37°C for 48 hours with 1 µM of anti-Ras·GTP iMab combined with a light-chain variable region fused with EpCAM-targeting protopeptide and having improved production yield and inhibited HSPG-binding ability in several Ras mutant and wild-type cell lines.
FIG. 14A shows the result of a test to compare the tumor growth inhibition effect when intravenously injecting 20 mg/kg of anti-Ras cell-penetrating antibodies (RT22-i38 MG, RT22-i39 MG), fused with integrin-targeting protopeptide and having no HSPG-binding ability, and the conventional anti-Ras cell-penetrating antibody (RT11-i), fused with integrin-targeting protopeptide, into human colorectal cancer KRas mutant cell lines LoVo and SW480 xenograft mice a total of 9 times at 2-day intervals.
FIG. 14B is a graph showing the weight of an extracted tumor and an image showing the tumor after treatment with the anti-Ras cell-penetrating antibody fused with the integrin-targeting protopeptide and having no HSPG-binding ability.
FIG. 14C is a graph showing the weight of the mice measured to determine the non-specific side effects of the anti-Ras cell-penetrating antibody fused with the integrin-targeting protopeptide and having no HSPG-binding ability.
FIG. 15A shows the result of a test to compare the tumor growth inhibition effect when intravenously injecting 20 mg/kg of anti-Ras cell-penetrating antibodies (RT22-ep58 MG, RT22-ep59 MG), fused with EpCAM-targeting protopeptide and having no HSPG-binding ability, into human colorectal cancer cell lines LoVo and SW480 xenograft mice a total of 9 times at 2-day intervals.
FIG. 15B is a graph showing the weight of the extracted tumor and an image showing the tumor, after treatment with the anti-Ras cell-penetrating antibody, fused with the EpCAM-targeting protopeptide and having no HSPG-binding ability.
FIG. 15C is a graph showing the weight of the mice measured to determine the non-specific side effects of the anti-Ras cell-penetrating antibody fused with the EpCAM-targeting protopeptide and having no HSPG-binding ability.
FIG. 16A shows the result of a test to compare the tumor growth inhibition ability depending on dose, administration interval and administration route of the anti-Ras cell-penetrating antibody (RT22-i39 MG) fused with integrin-targeting protopeptide and having no HSPG-binding ability in human colorectal cancer KRas mutant cell line LoVo xenograft mice.
FIG. 16B is a graph showing the weight of the extracted tumor and an image showing the tumor after treatment with the anti-Ras cell-penetrating antibody fused with the integrin-targeting protopeptide and having no HSPG-binding ability.
FIG. 16C is a graph showing the weight of the mice, measured to determine the non-specific side effects of the anti-Ras cell-penetrating antibody fused with the integrin-targeting protopeptide and having no HSPG-binding ability.
FIG. 17A shows the result of a test to compare the tumor growth inhibition ability depending on dose, administration interval and administration route of the anti-Ras cell-penetrating antibody (RT22-ep59 MG) fused with integrin-targeting protopeptide and having no HSPG-binding ability in human colorectal cancer KRas mutant cell line LoVo xenograft mice.
FIG. 17B is a graph showing the weight of the extracted tumor and an image showing the tumor after treatment with the anti-Ras cell-penetrating antibody fused with the EpCAM-targeting protopeptide and having no HSPG-binding ability.
FIG. 17C is a graph showing the weight of the mice, measured to determine the non-specific side effects of the anti-Ras cell-penetrating antibody fused with the integrin-targeting protopeptide and having no HSPG-binding ability.
FIG. 18A shows the result of determination of the reduced intracytoplasmic degradation of tumor-tissue EpCAM-specific anti-Ras·GTP iMab using the improved split green fluorescent protein complementation system.
FIG. 18B shows the result of a soft agar colony formation method conducted to determine the inhibitory activity of non-adherent cell growth in a human colorectal cancer cell line by tumor-tissue EpCAM-specific anti-Ras GTP iMab having improved intracytoplasmic stability.
FIG. 18C shows the result of an anoikis method conducted to determine the inhibitory activity of non-adherent cell growth in a human colorectal cancer cell line by tumor-tissue EpCAM-specific anti-Ras·GTP iMab having improved intracytoplasmic stability.
FIG. 18D shows the result of an anoikis method conducted to determine the inhibitory activity of non-adherent cell growth in a human lung cancer cell line by tumor-tissue integrin-specific anti-Ras·GTP iMab having improved intracytoplasmic stability.
FIG. 19A shows the result of analysis through 12% SDS-PAGE in reducing or non-reducing conditions after purification of tumor-tissue EpCAM-specific anti-Ras GTP having improved *in-vivo* persistence.
FIG. 19B shows the result of pharmacokinetic evaluation of tumor-tissue EpCAM-specific anti-Ras GTP having improved *in-vivo* persistence in BALB/c node mice.
FIG. 20A shows the result of a test using FACS to determine the binding ability to cell-surface EpCAM of anti-Ras·GTP iMab, epRas23 MG, fused with the N-terminal of the heavy-chain variable region in order to improve tumor-tissue-targeting ability.
FIG. 20B shows the result of ELISA to analyze the binding ability to 25, 50, 100 nM Avi-KRas^{G12D}·GppNHp or 100 nM Avi-KRas^{G12D}·GDP of anti-Ras·GTP iMabs including EpCAM-targeting peptide fused with the N-terminal of the heavy-chain variable region in order to improve tumor-tissue-targeting ability.
FIG. 20C shows the result of an anoikis method conducted to determine the inhibitory activity of non-adherent cell growth in a human lung cancer cell line by epRas23 MG, anti-Ras·GTP iMab having an EpCAM-targeting peptide fused with the N-terminal of the heavy-chain variable region to improve tumor-tissue-targeting ability.
FIG. 20D is an image showing the bio-distribution in a mouse of anti-Ras·GTP iMab, epRas23 MG, including the EpCAM-targeting peptide fused with the N-terminal of the heavy-chain variable region to improve tumor-tissue-targeting ability, and a graph showing the fluorescence of the tumor and the entire body.
FIG. 20E is an image showing the bio-distribution in a mouse of anti-Ras·GTP iMab, epRas23 MG, including the EpCAM-targeting peptide fused with the N-terminal of the heavy-chain variable region to improve tumor-tissue-targeting ability, and a graph showing the fluorescence quantified using the extracted organ.
FIG. 21A shows the result of size-exclusion chromatography to determine whether or not the LALA-PG mutant to improve the *in-vivo* persistence of tumor-tissue-specific anti-Ras·GTP iMab is a multimer.
FIG. 21B shows the result of pharmacokinetic evaluation, in BALB/c nude mice, of the LALA-PG mutant to improve *in-vivo* persistence of tumor-tissue-EpCAM specific anti-Ras·GTP iMab.
FIG. 22A shows the result of size-exclusion chromatography to determine the presence of a multimer in the LALA-PG mutant to improve *in-vivo* persistence of tumor tissue integrin-specific anti-Ras·GTP iMab.
FIG. 22B shows the result of a test to compare the tumor growth inhibition effect of the LALA-PG mutant to improve the *in-vivo* persistence of tumor-tissue integrin-specific anti-Ras cell-penetrating antibody in human colorectal cancer KRas mutant cell line LS1034 xenograft mice.
FIG. 22C shows the result of a test to compare the tumor growth inhibition effect of the LALA-PG mutant to improve the *in-vivo* persistence of tumor-tissue integrin-specific anti-Ras cell-penetrating antibody in human colorectal cancer KRas mutant cell line SW403 xenograft mice.

### [Best Mode]

Hereinafter, the present disclosure will be described in more detail with reference to examples. However, it will be obvious to those skilled in the art that these examples are provided only for illustration of the present disclosure and should not be construed as limiting the scope of the present disclosure.

### Example 1. Preparation of Avi-KRas^{G12D} protein having GppNHp bound thereto

An Avi-KRas^{G12D} antigen including an Avi tag (GLNDIFEAQKIEWHE) fused to an N-term thereof for use in library selection was constructed. The Avi-KRas^{G12D} antigen was constructed in order to minimize structural denaturation which may cause problems with antigen biotinylation during library selection.

Specifically, DNA in which catalytic G domains (residues 1 to 169) of the KRas^{G12D} protein, excluding the C-terminal hypervariable region are fused to an 8X his tag and an Avi-tag at the N-terminus using a GSG linker was constructed by PCR and was cloned using the restriction enzymes NcoI and HindIII in the pET23 vector, which is a vector for *E. coli* expression. Then, the constructed pET23-8Xhis-Avi-KRas^{G12D} (1-169) vector was transformed through electroporation into the *E. coli* strain BL21(DE3)plysE along with a vector (pBirAcm) encoding BirA as a biotin ligase for *in-vivo* biotinylation, and then was selected in a selective medium containing 100 µg/ml ampicillin and 10 µg/ml chloramphenicol. After culturing the selected *E. coli* in the selective medium (LBCA) containing 5 mM MgCl₂ at 37°C until absorbance at 600 nm reached 0.6, 0.5 mM IPTG for protein expression and 50 µM d-biotin for *in-vivo* biotinylation were added thereto, followed by further culturing at 30°C for 4 hours. The stock solution for addition of 50 µM d-biotin was prepared by adding 12 mg of d-biotin to 10 mL of 10 mM bicin buffer (pH 8.3). After culturing *E. coli,* the *E. coli* collected using a centrifuge were resuspended in buffer containing 20 mM Tris, 500 mM NaCl, 5 mM imidazole and 2 mM β-ME, and *E. coli* (SONICS) was pulverized with ultrasound waves. Only the supernatant, from which the pulverized E. coli was removed, was collected using a centrifuge, and then purified using Ni-NTA resin for specifically purifying the protein fused with the His tag. The Ni-NTA resin was washed with 50 ml of washing buffer (20 mM Tris, pH 7.4, 300 mM NaCl, 20 mM imidazole, 2 mM MgCl₂ and 2 mM β-ME) and proteins were eluted with an elution buffer (20 mM Tris, pH 7.4, 300 mM NaCl, 250 mM imidazole, 2 mM MgCl₂ and 2 mM β-ME). The eluted proteins were buffered with a storage buffer (50 mM Tris-HCl, pH 8.0, 1 mM DTT, 2 mM MgCl₂) using a dialysis method. The purified proteins were quantified using absorbance and the absorption coefficient measured at a wavelength of 280 nm. Purity of about 98% or more was identified through SDS-PAGE analysis.

The fusion of the purified Avi-KRas^{G12D} protein with Avi-tag and biotin at high yield through the *in-vivo* biotinylation reaction was identified using a gel shift method. Specifically, the Avi-KRas^{G12D} protein was diluted in an SDS-PAGE loading buffer (25 mM Tris-HCl, pH 6.8, 1% SDS, 10% glycerol, 175 mM β-ME), allowed to react for 10 minutes and then allowed to react with 1 µg of streptavidin at room temperature for 30 minutes, the protein separated through SDS-PAGE was transferred to the PVDF membrane and identified using the anti-His antibody fused with HRP.

The Avi-KRas^{G12D} protein was reacted with GTP analog (GppNHp) or GDP and was then analyzed by SDS-PAGE. Specifically, in order to form a complex of the GTP analog (GppNHP) and Ras protein, the purified Ras protein was diluted in a substrate exchange buffer (40 mM Tris-HCl, pH 7.5, 200 mM (NH₄)₂SO₄, 10 µM ZnCl₂, 5 mM DTT), and an alkaline phosphatase fused with 2 units of beads per mg of Ras protein and GppNHp having a molar amount at least 10 times that of the Ras protein were added thereto, followed by allowing a reaction to proceed at room temperature for 1 hour. In order to form a complex of GDP and Ras protein, GDP in a molar amount at least 20 times that of the Ras protein and 20 mM EDTA were added and allowed to react at 30°C for 30 minutes, and then 60 mM MgCl₂ was added thereto, and the result was allowed to stand at 4°C for 30 minutes to stop the reaction. The GppNHp or GDP-bound Ras protein obtained through the method was analyzed through SDS-PAGE after the buffer was exchanged with a storage buffer (50 mM Tris-HCl, pH 8.0, 1 mM DTT, 2 mM MgCl₂) using a PD10 Sephadex G25 column. For long-term storage, the Ras protein bound to the substrate was stored at -80°C.

ELISA was performed to analyze the RT11 binding capacity of the Avi-KRas^{G12D} protein prepared through the above method and the His-KRas^{G12D} protein having no Avi-tag. Specifically, RT11, an anti-Ras·GTP iMab, was bound at a concentration of 5 µg/ml in a 96-well EIA/RIA plate (COSTAR Corning) at room temperature for 1 hour, and was then washed with 0.1% TBST (12 mM Tris, pH 7.4, 137 mM NaCl, 2.7 mM KCl, 0.1% Tween20, 5 mM MgCl₂) at room temperature for 10 minutes. After binding with 4% TBSB (12 mM Tris, pH 7.4, 137 mM NaCl, 2.7 mM KCl, 4% BSA, 10 mM MgCl₂) for 1 hour, the result was washed 3 times with 0.1% TBST for 10 minutes. Then, the KRas protein bound with GppNHp and the KRas protein bound with GDP were diluted in 4% TBSB and then bound at a concentration of 100 nM for 1 hour at room temperature, followed by washing three times with 0.1% TBST for 10 minutes. The result was bound to HRP-conjugated anti-his antibody (HRP-conjugated anti-his mAb) as a labeling antibody. The result was reacted with a TMB ELISA solution, and absorbance at 450 nm was quantified.

The above experiment showed that the KRas^{G12D} protein fused with Avi-tag can be used to select the RT11 affinity-improvement library.

### Example 2. Construction of anti-Ras·GTP iMab RT11-based high-diversity antibody library and selection of heavy-chain variable region (VH) with enhanced Ras·GTP-specific affinity

The anti-Ras ·GTP iMab RT11 in the conventional patent (Koran Patent No. 10-1602876) binds highly specifically to Ras·GTP and exhibits biological activity in various Ras mutant cell lines, but exhibits a level of affinity of about 12 nM for Ras ·GTP, which is a lower affinity than the affinity of various antibodies in the IgG format. In addition, anti-Ras·GTP iMab RT11, which exhibits biological activity through inhibition of binding between Ras·GTP and effector molecules, can enhance biological activity through the improvement of affinity with Ras·GTP. Accordingly, the present inventors tried to increase the affinity of anti-Ras·GTP iMab RT11 for Ras·GTP in addition to modifying (improving) the light-chain variable region to impart tissue specificity thereto in order to increase the therapeutic efficiency of anti-Ras·GTP iMab.

The light-chain variable region used during selection of the Ras·GTP specific heavy-chain variable region with improved affinity based on RT11 is the hT4-3 light-chain variable region with improved endosomal escape ability, which is obtained by introducing CDR3 in which WYW (Trp-Tyr-Trp) is located at residues 92-94 of the hT4 light-chain variable region (VL) for cytoplasmic penetration used in the conventional patent (Korean Patent No. 10-1602876) and substituting, with phenylalanine (Phe), the residue 87 of the light-chain variable region framework region corresponding to the interface between the light-chain variable region (VL) and heavy-chain variable region (VH) in order to overcome the increased exposure of hydrophobic residues to solvents and thus decreased production yield due to the introduction of WYW (Korean Patent Application No. 10-2016-0065365) .

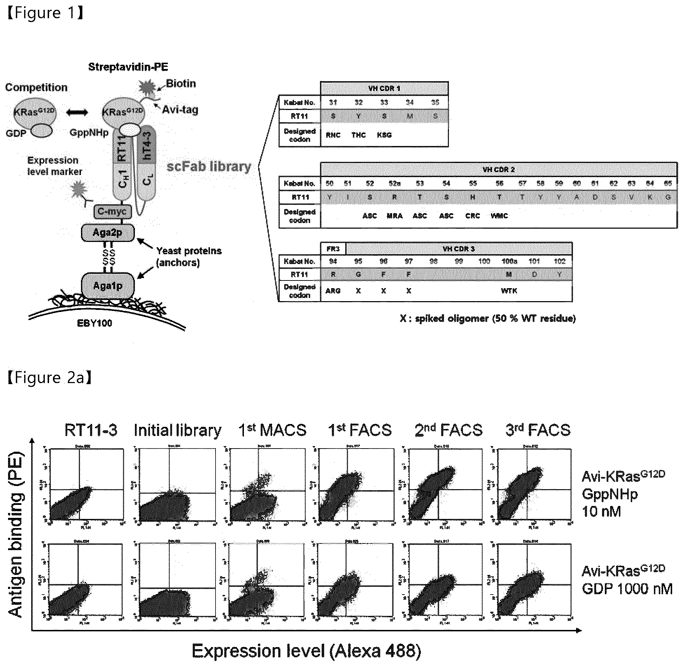

FIG. 1 is a schematic diagram showing the strategy for selection and construction of a library based on the heavy-chain variable region of RT11 in order to improve the affinity of anti-Ras·GTP iMab to Ras·GTP.

The binding structure between Ras·GTP and RT11 antibody was predicted using homology modeling and docking programs, and random mutations were introduced into CDRs and surrounding regions predicted to play an important role in antigen binding based on the prediction result.

Specifically, degenerated codons that may include a suitable amino acid sequence in the predicted binding structure were used for the residues of CDR1 (Nos. 31 to 33) and CDR2 (Nos. 52 to 56). The degenerate codons RNC, THC and KSG were sequentially used for CDR1 (Nos. 31 to 33) and the degenerate codons ASC, MRA, ASC, ASC, CRC and WMC were sequentially used for CDR2 (Nos. 52 to 56). ARG degenerate codons capable of encoding Arg and Lys present in the germline antibody sequence were used because the residue 94 of the CDR3-surrounding framework is a residue at a position that can affect the CDR structure. The residues (Nos. 95 to 97) of CDR3 were spiked oligomers capable of conserving the sequence of RT11 with a 50% probability. This is a technique that can maintain the wild-type amino acid at 50% during the PCR process by designing a primer such that, in each of 3 nucleotides encoding amino acids for each residue, wild-type nucleotides can be maintained at 79%, and the ratio of remaining nucleotides was adjusted to 7%. The CDR3 (No. 100a) residue is an important residue for VH/VL binding, and a WTK degenerate codon, which may include Phe, Ile and Leu present in the germline antibody sequence, including Met of the RT11 antibody was used therefor.

Specifically, hT4-3 VL with improved cytoplasmic penetration ability was used for the light-chain variable region in the affinity improvement library.

Specifically, the designed library-encoding DNA was amplified using a PCR technique and was then concentrated using an ethanol precipitation method. The yeast surface expression vector (C-aga2), which expresses aga2 protein at the c-terminus for homologous recombination, was treated with NheI and MluI restriction enzymes and then purified using agarose gel extraction and concentrated using ethanol precipitation. The 5 µg vector treated with restriction enzymes for 12 µg of each library-encoding DNA was transformed by electroporation into the yeast EBY100 for yeast surface expression (Baek D.S. and Kim Y.S., 2014), and the number of colonies grown in a selective medium, SD-CAA (20 g/L glucose, 6.7 g/L yeast nitrogen base without amino acids, 5.4 g/L Na 2HPO₄, 8.6 g/L NaH₂PO₄, 5 g/L casamino acids) through serial dilution was measured to determine the library size.

### Example 3. Selection of heavy-chain variable region (VH) with improved affinity for GppNHp-bound KRas^{G12D}

The RT11-3-based affinity improvement library constructed in Example 2 was selected using the GppNHp-bound KRas^{G12D} as an antigen.

Specifically, about 100 nM of the purified GppNHp-bound Avi-KRas^{G12D} was reacted with yeast inducing expression of the single-chain Fab (scFab)-type heavy-chain variable region library on the cell surface using an SG-CAA medium (20 g/L galactose, 6.7 g/L yeast nitrogen base without amino acids, 5.4 g/L Na₂HPO₄, 8.6 g/L NaH₂PO₄, 5 g/L casamino acids) at room temperature for 1 hour. Then, the yeast expressing the library linked with GppNHp-bound Avi-KRas^{G12D} was reacted with Streptavidin Microbead™ (Miltenyi Biotec) for 20 minutes at 4°C, and then a yeast expressing the heavy-chain variable region having high affinity for the GppNHp-bound Avi-KRas^{G12D} was enriched using MACS (magnetic activated cell sorting). The yeast expressing the selected library was cultured in selective media SD-CAA (20 g/L glucose, 6.7 g/L yeast nitrogen base without amino acids, 5.4 g/L Na₂HPO₄, 8.6 g/L NaH₂PO₄, 5 g/L casamino acids) and SG-CAA to induce library expression, the GppNHP-bound Avi-KRas^{G12D} and Avi-KRas^{G12D} antigen bound with GDP undergoing no *in-vivo* biotinylation for primary FACS screening were competitively reacted at a ratio of 1:10 with the library-expressing yeast for 1 hour at room temperature, reacted with PE-conjugated Streptavidin-R-phycoerythrin (SA-PE, Invitrogen), and suspended through FACS (fluorescence activated cell sorting, FACS Caliber, BD biosciences) . Then, secondary FACS screening was performed in the same manner as above at a ratio of 1:100 using 10 nM of the GppNHp-bound Avi-KRas^{G12D} antigen and the Avi-KRas^{G12D} antigen bound with GDP undergoing no *in-vivo* biotinylation, and tertiary FACS screening was performed in the same manner as above at a ratio of 1:100 using 1 nM of the GppNHp-bound Avi-KRas^{G12D} antigen and the GDP-bound Avi-KRas^{G12D} antigen undergoing no *in-vivo* biotinylation.

FIG. 2A shows the result of FACS analysis to determine the binding ability of library expression yeast in each step with 10 nM Avi-KRas^{G12D}·GppNHp or 1000 nM Avi-KRas^{G12D}·GDP in order to confirm the specific enrichment in Avi-KRas^{G12D}·GppNHp through the selection process using MACS (magnetic activated cell sorting) and FACS (fluorescence activated cell sorting). This identifies that clones having high affinity for GppNHp-bound Avi-KRas^{G12D} compared to RT11-3 were selected in a heavy-chain variable region (VH)-dependent manner through an ultra-fast selection process.

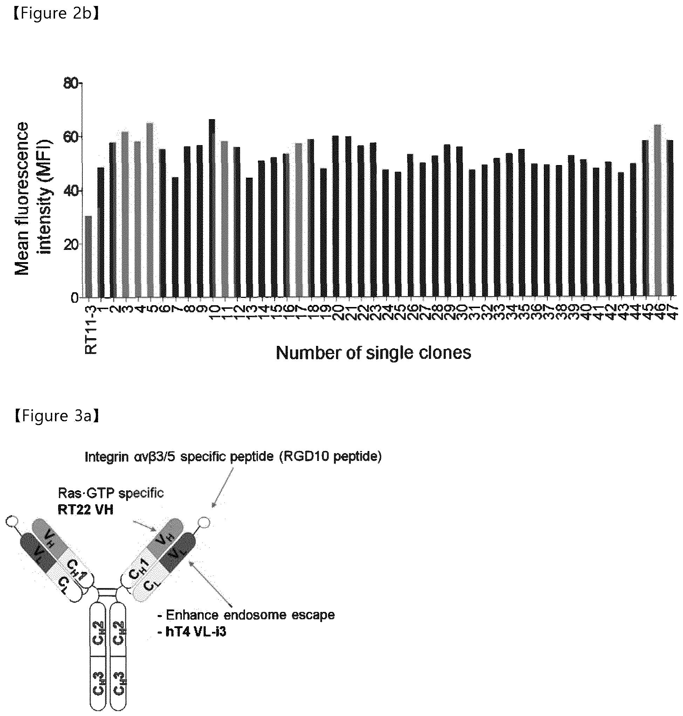

FIG. 2B shows the result of flow cytometry to determine the binding ability of the yeast expressing 47 individual clones in the finally selected library with 5 nM Avi-KRas^{G12D}·GppNHp.

Six unique clones (RT21, RT22, RT23, RT24, RT25, RT26) having high affinity and specificity to the GppNHp-bound Avi-KRas^{G12D} were selected through the analysis of individual clone binding ability as described above.

The following Table 1 shows the heavy-chain variable region sequences of six individual clones that have high binding ability to the selected GppNHp-bound Avi-KRas^{G12D}, and Table 2 shows the CDR sequences of the heavy-chain variable regions of Table 1.

**[Table 1]**

| VH name | Sequence | SEQ ID NO. |
|---|---|---|
| RT11 VH | 10 20 30 40 50 52a EVQLVESGGGLVQPGGSLRLSCAASGFTFSSYSMSWVRQAPGKGLEWVSYISRTSHTTY 60 70 80 82a 90 100a 110 YADSVKGRFTISRDNSKNTLYLQMNSLRAEDTAVYYCARGFFMDYWGQGTLVTVSS | 19 |
| RT21 VH | 10 20 30 40 50 52a EVQLVESGGGLVQPGGSLRLSCAASGFTFSDYSMSWVRQAPGKGLEWVSYISRTSHTTY 60 70 80 82a 90 100a 110 YADSVKGRFTISRDNSKNTLYLQMNSLRAEDTAVYYCARGFKMDYWGQGTLVTVSS | 20 |
| RT22 VH | 10 20 30 40 50 52a EVQLVESGGGLVQPGGSLRLSCAASGFTFSDFSMSWVRQAPGKGLEWVSYISRTSHTTY 60 70 80 82a 90 100a 110 YADSVKGRFTISRDNSKNTLYLQMNSLRAEDTAVYYCARGFKMDYWGQGTLVTVSS | 21 |
| RT23 VH |         10        20        30        40        50 52a EVQLVESGGGLVQPGGSLRLSCAASGFTFSDYSMSWVRQAPGKGLEWVSYISRTSHTTY 60        70        80 82a        90        100a        110 YADSVKGRFTISRDNSKNTLYLQMNSLRAEDTAVYYCARGFRMDYWGQGTLVTVSS | 22 |
| RT24 VH | 10 20 30 40 50 52a EVQLVESGGGLVQPGGSLRLSCAASGFTFSDFSMSWVRQAPGKGLEWVSYISRTSHTTY 60 70 80 82a 90 100a 110 YADSVKGRFTISRDNSKNTLYLQMNSLRAEDTAVYYCAKGFFMNYWGQGTLVTVSS | 23 |
| RT25 VH |           10        20        30        40        50 52a EVQLVESGGGLVQPGGSLRLSCAASGFTFSDFSMSWVRQAPGKGLEWVSYISRTSHTTY 60        70        80 82a        90      100a      110 YADSVKGRFTISRDNSKNTLYLQMNSLRAEDTAVYYCARGFRMDYWGQGTLVTVSS | 24 |
| RT26 VH | 10 20 30 40 5052a EVQLVESGGGLVQPGGSLRLSCAASGFTFSDFSMSWVRQAPGKGLEWVSYISRTSHTTY 60 70 80 82a 90 100a 110 YADSVKGRFTISRDNSKNTLYLQMNSLRAEDTAVYYCARGFFMNYWGQGTLVTVSS | 25 |

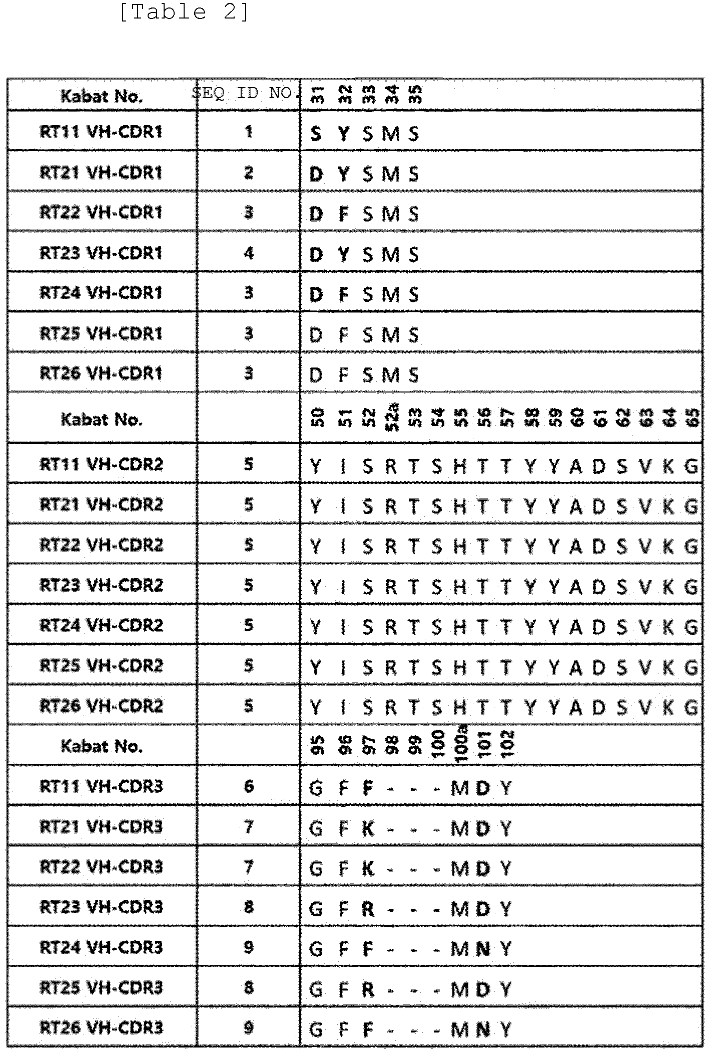

### Example 4. Analysis of antigen-binding capacity of anti-Ras·GTP iMab antigen with improved affinity

In order to construct the cytoplasmic penetration antibody (cytotransmab) introduced with the mutation in the heavy-chain variable region, a heavy chain including a heavy-chain variable region having improved affinity for Ras·GTP and a heavy-chain constant region (CH1-CH2-CH3) based on RT11 were cloned into an animal expression vector, and RGD10 protopeptides having specificity for integrins (Integrin αvβ3 and αvβ5), which are overexpressed in neovascular cells and various tumors, were fused with the N-terminus of the cytoplasmic penetration humanized hT4-3 light-chain using two GGGGS linkers using a genetic engineering technique, and were cloned into an animal expression vector. The RGD10 protopeptide has affinity similar to that of the RGD4C protopeptide, but has only one disulfide bond between two cysteines, and it can be fused using a genetic engineering technique. The heavy-chain expression vector having improved affinity for Ras·GTP and the cytoplasmic penetration humanized light-chain expression vector (hT4-i3 LC) fused with the RGD10 protopeptide were simultaneously subjected to transient transfection in HEK293F protein-expressing cells to express an anti-Ras ·GTP iMab mutation with improved affinity.

Specifically, in order to construct a heavy-chain expression vector for producing an intact immunoglobulin-type cytoplasmic penetration antibody, DNA encoding a heavy-chain including the heavy-chain constant region (CH1-CH2-CH3) and being introduced with the heavy-chain variable mutation having improved affinity based on RT11, which is fused with a DNA encoding a secretion signal peptide at the 5' end thereof, was cloned into the pcDNA3.4 vector with NotI/HindIII. Proteins were expressed and purified through transient transfection using the heavy-chain expression vector and the light chain of the previous cytoplasmic penetration antibody (Korean Patent Application No. 10-2016-0065365), and yields were compared. HEK293F cells suspended and grown in a serum-free FreeStyle 293 expression medium were transfected with a mixture of plasmids and polyethyleneimine (PEI) in a shake flask. During 200 mL transfection into the shake flask, the HEK293F cells were seeded in 100 ml of medium at a density of 2.0 x 10⁶ cells/ml and cultured at 120 rpm, 8% CO₂, and 37 degrees. In order to produce the antibody, suitable heavy-chain and light-chain plasmids were diluted in a 10 ml FreeStyle 293 expression medium to a total of 250 µg (2.5 µg/ml) including 125 µg of a heavy chain and 125 µg of a light chain and filtered, then mixed with 10 ml of a medium, in which 750 µg (7.5 µg/ml) of PEI was diluted, and reacted at room temperature for 10 minutes. Then, 100 ml of the reacted mixed medium was added to previously seeded cells, and then the cells were cultured at 120 rpm and 8% CO₂ for 4 hours, and the remaining 100 ml of FreeStyle 293 expression medium was added thereto, followed by culturing for 6 days. Proteins were purified from the cell culture supernatant collected in accordance with the standard protocol. Antibodies were applied to a Protein A Sepharose column and washed with PBS (pH 7.4). The antibodies were eluted at pH 3.0 using 0.1M glycine and 0.5M NaCl buffer, and then the sample was immediately neutralized with 1M Tris buffer. The eluted antibody fraction was concentrated in a fresh PBS (pH 6.5) buffer exchanged through a dialysis method. The purified protein was quantified using absorbance and absorption coefficient at a wavelength of 280 nm.

FIG. 3A is a schematic diagram illustrating the construction of complete IgG-type anti-Ras·GTP iMab (RT22-i3) fused with integrin-targeting protopeptide and having improved affinity for Ras·GTP based on RT11.

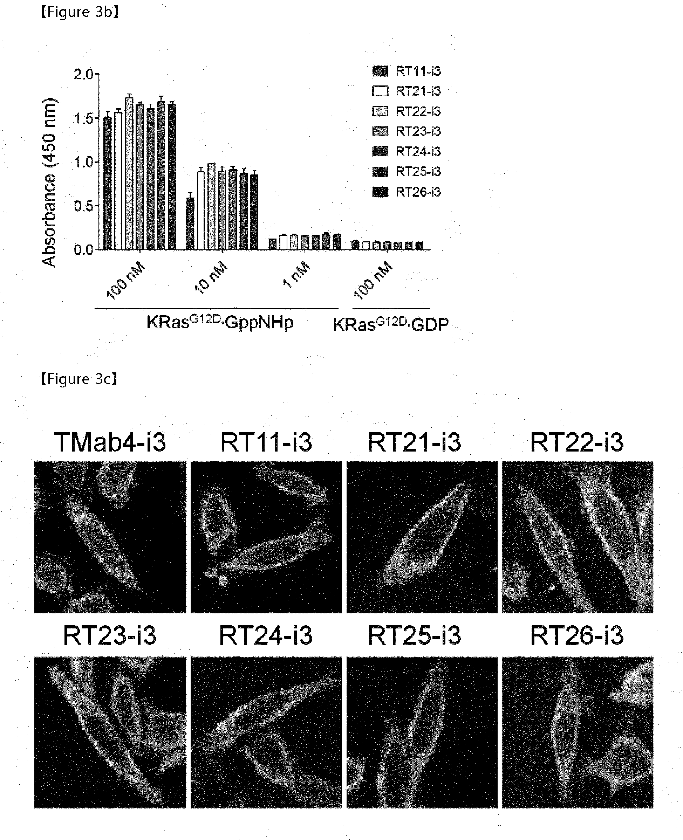

FIG. 3B shows the result of ELISA to analyze the binding ability of the RT11-based affinity-improved anti-Ras·GTP iMabs with 1, 10 or 100 nM Avi-KRas^{G12D}·GppNHp and 100 nM Avi-KRas^{G12D} ·GDP in order to determine the specific binding of the anti-Ras·GTP iMabs with GppNHp-bound Avi-KRas^{G12D}.

Specifically, RT11-i, an anti-Ras·GTP iMab, and six affinity-enhanced iMabs fused with RGD protopeptides were bound at a concentration of 5 µg/ml in a 96-well EIA/RIA plate at room temperature for 1 hour. Then, the result was washed with 0.1% TBST (12 mM Tris, pH 7.4, 137 mM NaCl, 2.7 mM KCl, 0.1% Tween20, 5 mM MgCl₂) three times for 10 minutes. Then, the result was bound to 4% TBSB (12 mM Tris, pH 7.4, 137 mM NaCl, 2.7 mM KCl, 4% BSA, 10 mM MgCl₂) for 1 hour and then washed 3 times with 0.1% TBST for 10 minutes. The GppNHp-bound KRas protein and the GDP-bound KRas protein were diluted in 4% TBSB, bound at various concentrations of 100 nM, 10 nM and 1 nM for 1 hour at room temperature, and washed 3 times with 0.1% TBST for 10 minutes. The result was bound to HRP-conjugated anti-his antibody (HRP-conjugated anti-his mAb) as a labeling antibody. The result was reacted with TMB ELISA solution, and the absorbance at 450 nm was quantified.

ELISA analysis showed that the selected 6 types of anti-Ras·GTP iMabs exhibited higher antigen-binding capacity than the conventional RT11-i3.

FIG. 3C shows the result of confocal microscopy analysis to determine the overlap with the activated intracellular Ras after treating the KRas mutant colorectal cancer cell line SW480 with the anti-Ras·GTP iMabs having improved affinity.

Specifically, an SW480 cell line was diluted in 0.5 ml of 2x10⁴ cells/well in a 24-well plate, cultured for 12 hours at 37°C under 5% CO₂, and then treated with TMab4-i, RT11-i, and 6 types of 1 µM anti-Ras·GTP iMab with improved affinity and then cultured at 37°C for 12 hours. Then, the medium was removed, the residue was washed with PBS, and proteins attached to the cell surface were removed with a weakly acidic solution (200 mM glycine, 150 mM NaCl pH 2.5). After washing with PBS, 4% paraformaldehyde was added and cells were immobilized at 25 degrees for 10 minutes. After washing with PBS, the cells were cultured in a buffer containing PBS supplemented with 0.1% saponin, 0.1% sodium azide and 1% BSA at 25 degrees for 10 minutes and a hole was formed in the cell membrane. Then, the cells were washed with PBS again and reacted with a buffer containing 2% BSA in addition to PBS for 1 hour at 25 degrees in order to inhibit non-specific binding. Each antibody was stained with an antibody that specifically recognizes human Fc linked with Alexa-488 (green fluorescence) . The nuclei were stained (blue fluorescence) using Hoechst33342, and KRas-labeled antibodies were stained (red fluorescence) and then observed with a confocal microscope. All of the anti-Ras·GTP iMabs except TMab4-i3 were found to have fluorescence overlapping that of the intracellular Ras.

Among them, the RT22-i3 iMab that exhibits the ability to bind to activated intracellular Ras and the highest binding capacity with Avi-KRas^{G12D} ·GppNHp during ELISA experiments was subjected to SPR (surface plasmon resonance) analysis using a Biacore2000 instrument to more quantitatively analyze the binding ability thereof to GppNHp-bound KRas^{G12D}.

Specifically, RT22-i3 was diluted to a concentration of 20 µl/ml in 10 mM NaAc buffer (pH 4.0) and immobilized at approximately 1800 response units (RU) on a CM5 sensor chip (GE Healthcare). Subsequently, Tris buffer (20 mM Tris-HCl, pH 7.4, 137 mM NaCl, 5 mM MgCl₂, 0.01% Tween 20) was analyzed at a flow rate of 30 µl/min, and GppNHp-bound GST-KRas^{G12D} complete antibody was analyzed at a concentration of 50 nM to 3.125 nM. After binding and dissociation analysis, regeneration of the CM5 chip was performed by flowing a buffer (20mM NaOH, 1M NaCl, pH10.0) at a flow rate of 30 µl/min for 1 minute. Each sensorgram, obtained by binding for 3 minutes and dissociation for 3 minutes, was normalized with reference to a blank cell and subtracted, and the affinity was thus calculated.

The following Table 3 shows the results of affinity analysis of anti-Ras·GTP iMab RT11-i and RT22-i3 for complete-form GppNHp-bound GST-KRas^{G12D} using SPR (BIACORE 2000).

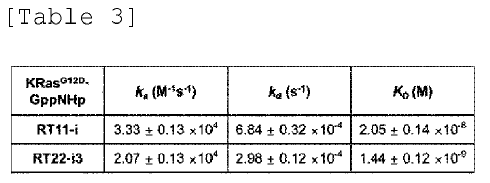

### Example 5. Library construction and selection for additional affinity improvement based on RT22 heavy-chain variable region (VH)

The anti-Ras ·GTP iMab (RT22-i3) including a heavy-chain variable region (RT22 VH) with improved binding ability to Ras·GTP has high affinity of about 1.4 nM. However, it was selected through combination with the light-chain variable region (hT4-3 VL), which maintains binding to the cell membrane receptor, HSPG. Since HSPG is expressed in most tissues, a light-chain variable region in which germline antibody sequences were introduced into CDR1 and CDR2 was constructed, and the anti-Ras·GTP iMab constructed using the light-chain variable region was found to have a serious decrease in production yield during fusion of RGD protopeptides. Accordingly, the CDR of the heavy-chain variable region (VH) combined with the light-chain variable region having tissue specificity was modified to overcome the problems of reduced affinity and production yield.

A description of the light-chain variable region having reduced HSPG binding and tissue specificity is given in detail below.

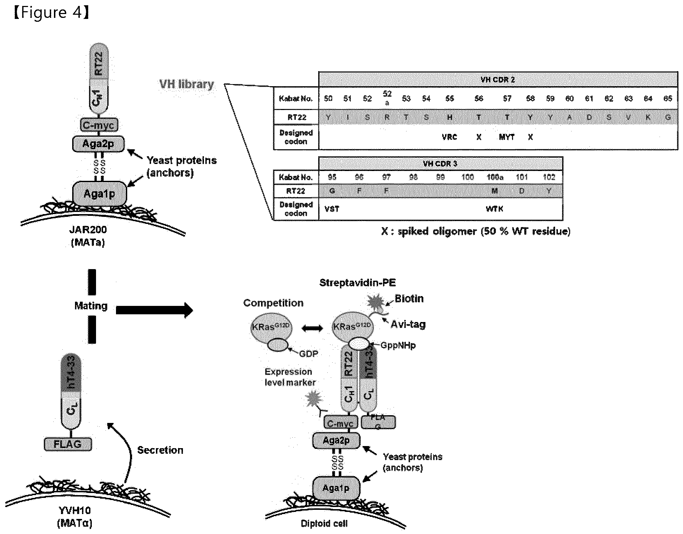

FIG. 4 is a schematic diagram showing a strategy for selecting and constructing a library based on the heavy-chain variable region of RT22 in order to improve the affinity of anti-Ras·GTP iMab for Ras·GTP.

To improve the affinity, 3D structural models were analyzed using Galaxy modeling, and mutations were imparted to CDR2 (Nos. 55 to 58) and CDR3 (Nos. 95, 97, and 100a), considered to have a significant effect on antigen binding, based on the analysis result.

Specifically, a degeneration codon (VRC) was used for the residue 55 of CDR2 so that the residue amino acid sequence could be maintained at a probability of 16.67% and a hydrophilic or negatively charged amino acid could be located thereat, and a degeneration codon (MYT) was used for residue 57 of CDR3 so that the affinity with the antigen could be increased in consideration of the size and direction of the side chain while maintaining the conventional amino acid sequence with a 25% probability. A degeneration codon (VST) was used for residue 95 of CDR3 so that the binding ability to Ras·GTP could be maintained or improved in consideration of the size and direction of the side chain while maintaining the conventional amino acid sequence with a 16.67% probability, and a degeneration codon (WTK) was used for residue 100a so that a hydrophobic amino acid could be located thereat. The same spiked oligomer as in Example 2 was used for residues 56 and 58 of CDR2 and residue 97 of CDR3. This is a mutation method in which all amino acids can be applied while preserving the conventional wild-type RT22 VH sequence at 50% probability, and is a technique that can maintain the wild-type amino acid at 50% during the PCR process by designing a primer such that, in each of 3 nucleotides encoding amino acids, the wild-type nucleotide can be maintained at 79%, and the ratio of remaining nucleotides is adjusted to 7%.

Specifically, a yeast expressing the initial RT22-based library on the surface thereof was mated with a yeast secreting the cytoplasmic penetration light chain (hT4-33 VL) from which HSPG-binding ability was removed and the resulting product was expressed in the form of Fab on the yeast surface.

Specifically, in order to construct the yeast secreting the cytoplasmic penetration light chain (hT4-33 VL) to be conjugated with the heavy-chain variable region (VH) library, pYDS-K-hT4-33 VL, obtained by cloning the DNA encoding hT4-33 VL having cytoplasmic penetration ability and reduced HSPG binding ability into the light-chain variable region yeast secretion vector, pYDS-K using restriction enzymes NheI and BsiWI, was transformed by electroporation into the YVH10 strain, which is a mating-α-type yeast-mating strain, and was mated with a yeast selectively cultured in a selective medium SD-CAA + Trp (20 g/L glucose, 6.7 g/L yeast nitrogen base without amino acids, 5.4 g/L Na 2HPO₄, 8.6 g/L NaH 2PO₄, 5 g/L casamino acids, 0.4 mg/L tryptophan) (SIGMA).

Specifically, in the case of yeast mating, when absorbance at 600 nm of 1 indicates 1 X 10⁷ yeast individuals. Among cultured yeast, yeast expressing the heavy-chain variable region library based on RT22 and yeast containing hT4-33 VL were mixed in amounts of 1.5 X 10⁷ individuals and washed three times with YPD (20 g/L dextrose, 20 g/L peptone, 10 g/L yeast extract, 14.7 g/L sodium citrate, 4.29 g/L citric acid, pH 4.5) (SIGMA), and then resuspended with 100 µl of YPD and dropped while preventing the same from spreading over the YPD plate, dried and cultured at 30 degrees for 6 hours. Then, the dried spreading yeast site was washed three times with YPD medium and cultured at 30°C for 24 hours in a selective medium SD-CAA to a final yeast concentration of 1 × 10⁶ or less, and only the mated yeast was selected.

### Example 6. Selection of heavy-chain variable region (VH) having high specific binding ability to GTP-bound KRas G12D ased on RT22

GppNHp, a GTP analogue, was bound to the *in-vivo* biotinylated Avi-KRas^{G12D} protein in the same manner as in Example 1, and was used for library selection. For the primary MACS selection, about 100 nM of the antigen was reacted with the Fab library expressed on the yeast surface at room temperature for 1 hour. Then, the yeast expressing the Fab library bound with the antigen was reacted with Streptavidin Microbead™ at 4 degrees for 20 minutes, and then the yeast expressing the heavy-chain variable region having high affinity to GppNHp-bound Avi-KRas^{G12D} was enriched using magnetic activated cell sorting (MACS). The selected library-expressing yeast was cultured in selective media SD-CAA+URA (20 g/L D-glucose, 6.7 g/L yeast nitrogen base without amino acids, 5.4 g/L Na₂HPO₄, 8.6 g/L NaH₂PO₄, 5 g/L casamino acids, 0.2 mg/L uracil) and SG-CAA+URA (20 g/L Galactose, 6.7 g/L yeast nitrogen base without amino acids, 5.4 g/L Na₂HPO₄, 8.6 g/L NaH₂PO₄, 5 g/L casamino acids, 0.2 mg/L Uracil) to induce library expression and primary to tertiary FACS selection was performed.

As a result of analyzing 47 individual clones of the final primary MACS and tertiary FACS selected libraries in the same manner as in Example 3, seven heavy-chain variable regions having unique amino acid sequences having high affinity for the GppNHp-bound Avi-KRas^{G12D} protein were selected.

The following Table 4 shows seven heavy-chain variable-region (VH) sequences having unique amino acid sequences of the individual clones selected from the affinity improvement library based on RT22 as a template.

The following Table 5 shows the sequences of CDRs 1, 2 and 3 of the selected heavy-chain variable regions (VH).

**[Table 4]**

| VH name | Sequence | SEQ ID NO. |
|---|---|---|
| RT31 VH | 10          20          30          40          50 52a EVQLVESGGGLVQPGGSLRLSCAASGFTFSDFSMSWVRQAPGKGLEWVSYISRTSHTIY 60          70          80 82a          90          100a          110 YADSVKGRFTISRDNSKNTLYLQMNSLRAEDTAVYYCARGFKMDYWGQGTLVTVSS | 26 |
| RT32 VH | 10 20 30 40 50 52a EVQLVESGGGLVQPGGSLRLSCAASGFTFSDFSMSWVRQAPGKGLEWVSYISRTSHTTC 60 70 80 82a 90 100a 110 YADSVKGRFTISRDNSKNTLYLQMNSLRAEDTAVYYCARGFKMDYWGQGTLVTVSS | 27 |
| RT33 VH | 10 20 30 40 50 52a EVQLVESGGGLVQPGGSLRLSCAASGFTFSDFSMSWVRQAPGKGLEWVSYISRTSHTTS 60 70 80 82a 90 100a 110 YADSVKGRFTISRDNSKNTLYLQMNSLRAEDTAVYYCARGFKMDYWGQGTLVTVSS | 28 |
| RT34 VH | 10 20 30 40 5052a EVQLVESGGGLVQPGGSLRLSCAASGFTFSDFSMSWVRQAPGKGLEWVSYISRTSHTIS 60 70 8082a 90 100a 110 YADSVKGRFTISRDNSKNTLYLQMNSLRAEDTAVYYCARGFKMDYWGQGTLVTVSS | 29 |
| RT35 VH | 10 20 30 40 5052a EVQLVESGGGLVQPGGSLRLSCAASGFTFSDFSMSWVRQAPGKGLEWVSYISRTSHTLC 60 70 80 82a 90 100a 110 YADSVKGRFTISRDNSKNTLYLQMNSLRAEDTAVYYCARGFRLDYWGQGTLVTVSS | 30 |
| RT36 VH | 10 20 30 40 50 52a EVQLVESGGGLVQPGGSLRLSCAASGFTFSDFSMSWVRQAPGKGLEWVSYISRTSHTLL 60 70 80 82a 90 100a 110 YADSVKGRFTISRDNSKNTLYLQMNSLRAEDTAVYYCARGFNLDYWGQGTLVTVSS | 31 |
| RT37 VH | 10          20          30          40          5052a EVQLVESGGGLVQPGGSLRLSCAASGFTFSDFSMSWVRQAPGKGLEWVSYISRTSHTIA 60          70          80 82a          90          100a          110 YADSVKGRFTISRDNSKNTLYLQMNSLRAEDTAVYYCARGFKMDYWGQGTLVTVSS | 32 |

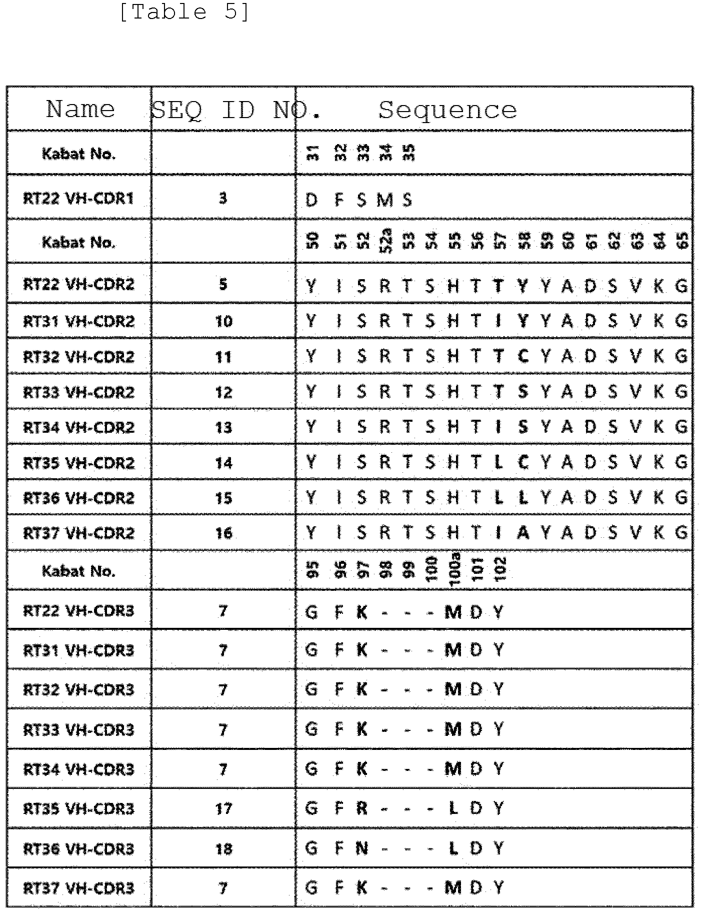

### Example 7. Expression and purification of RT22-based affinity-improved anti-Ras·GTP iMabs and analysis of binding capacity thereof to GppNHp-bound KRas^{G12D}

The heavy-chain variable region selected from the RT22-based library was cloned into a heavy-chain animal expression vector in the same manner as in Example 4, subjected to transient co-transfection into the cytoplasm-penetrating humanized light chain expression vector and the HEK293F protein-expressing cell to express separate clones, and purified in the same manner as in Example 4. Among the seven heavy-chain variable regions with improved affinity, RT32 and RT35 clones were excluded from expression purification because they included the cysteine in the CDR2 sequence and thus could not be present as a monomer (alone), and had the potential to form a dimer or oligomer through an unnatural disulfide bond when purified with IgG.

The RT22-based affinity-enhanced heavy-chain variable region was expressed in combination with a light-chain variable region (hT4-i33 VL: SEQ ID NO: 38) fused with RGD protopeptide that has an inhibitory activity of HSPG binding ability and endosome escape ability through introduction of a WYW mutation in CDR3. The description of the light-chain variable region (hT4-i33 VL) having reduced binding affinity to HSPG, SEQ ID NO: 38 and tissue specificity will be described in detail below.

As a result of expression, anti-Ras·GTP iMabs having improved affinity similar to RT22-i33, used as a template, also showed a low production yield of 1 mg/L, as in Example 3.

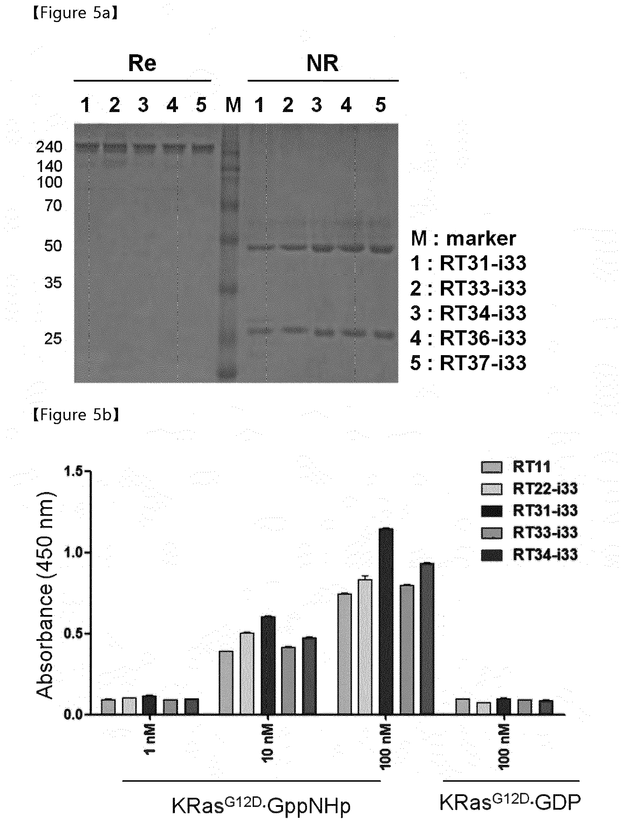

FIG. 5A shows the result of 12% SDS-PAGE analysis under reducing or non-reducing conditions after purification of anti-Ras·GTP iMab including a combination of the RT22-based affinity-enhanced heavy-chain variable regions (VH) with the light-chain variable region (hT4-i33) fused with the integrin-targeting protopeptide and having inhibited HSPG binding ability.

Specifically, a molecular weight of about 150 kDa was detected under non-reducing conditions, and the molecular weight of the heavy chain and the molecular weight of the light chain were found to be 50 kDa and 25 kDa, respectively, under reducing conditions. This indicated that the expressed and purified individual clones were present as monomers (alone) in the solution, and did not form dimmers or oligomers through unnatural disulfide bonds.

FIG. 5B shows the result of ELISA analysis of binding ability of each anti-Ras·GTP iMab to 1, 10 or 100 nM Avi-KRas^{G12D}·GppNHp or to 100 nM Avi-KRas^{G12D}·GDP to determine specific binding between GppNHp-bound Avi-KRas^{G12D} and anti-Ras·GTP iMabs including a combination of the RT22-based affinity-enhanced heavy-chain variable regions (RT31 VH, RT33 VH, RT34 VH) with the light-chain variable region (hT4-i33) fused with integrin-targeting protopeptide and having reduced HSPG binding ability.

Specifically, in the same manner as in Example 4, the anti-Ras·GTP iMab, RT11 and RT22-i33, used as a library template, were used as a control group, and five affinity-enhanced iMabs fused with RGD protopeptides were bound at a concentration of 5 µg/ml in a 96-well EIA/RIA plate at room temperature for 1 hour and washed three times with 0.1% TBST (12 mM Tris, pH 7.4, 137 mM NaCl, 2.7 mM KCl, 0.1% Tween20, 5 mM MgCl₂) for 10 minutes. Then, the result was bound in 4% TBSB (12 mM Tris, pH 7.4, 137 mM NaCl, 2.7 mM KCl, 4% BSA, 10 mM MgCl₂) for 1 hour and then washed 3 times with 0.1% TBST for 10 minutes. The GppNHp-bound KRas protein was diluted to various concentrations of 100 nM, 10 nM, and 1 nM, and the GDP-bound KRas protein was diluted in 4% TBSB to a concentration of 100 nM, bound for 1 hour at room temperature, and washed 3 times with 0.1% TBST for 10 minutes. The result was bound to an HRP-conjugated anti-his antibody (HRP-conjugated anti-his mAb) as a labeling antibody. The result was reacted with a TMB ELISA solution, and the absorbance at 450 nm was quantified.

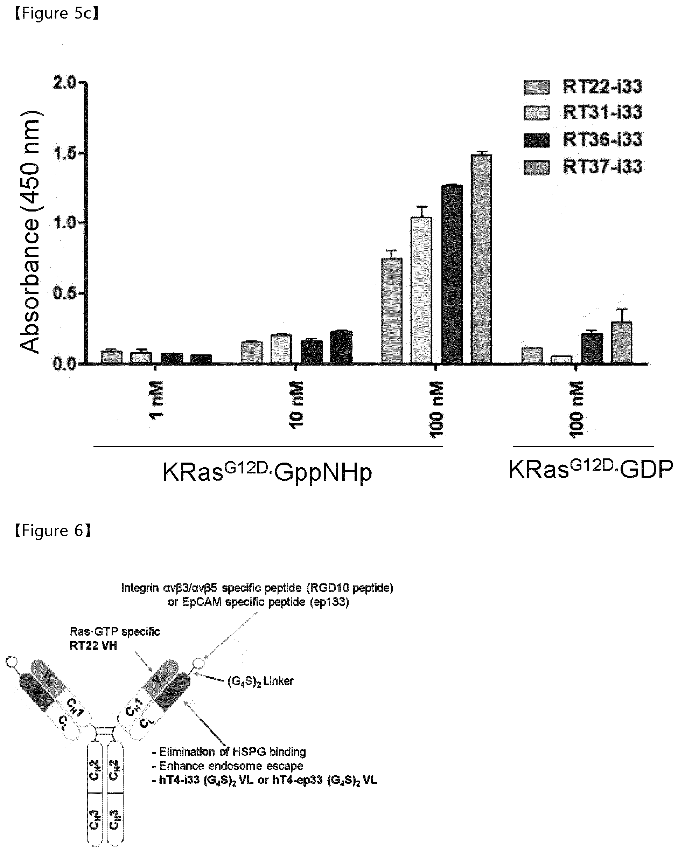

FIG. 5C shows the result of ELISA analysis of binding ability of each anti-Ras·GTP iMab to 1, 10 or 100 nM Avi-KRas^{G12D} ·GppNHp or 100 nM Avi-KRas^{G12D}•GDP to determine specific binding between GppNHp-bound Avi-KRas^{G12D} and anti-Ras·GTP iMabs including a combination of the RT22-based affinity-enhanced heavy-chain variable regions (RT31 VH, RT36 VH, RT37 VH) with the light-chain variable region (hT4-i33) fused with integrin-targeting protopeptide and having reduced HSPG binding ability.

Specifically, in the same manner as in Example 4, the anti-Ras·GTP iMab, RT11 and RT22-i33, used as a library template, were used as a control group, and five affinity-enhanced iMabs fused with RGD protopeptides were bound at a concentration of 5 µg/ml in a 96-well EIA/RIA plate at room temperature for 1 hour and washed three times with 0.1% TBST (12 mM Tris, pH 7.4, 137 mM NaCl, 2.7 mM KCl, 0.1% Tween20, 5 mM MgCl₂) for 10 minutes. Then, the result was bound in 4% TBSB (12 mM Tris, pH 7.4, 137 mM NaCl, 2.7 mM KCl, 4% BSA, 10 mM MgCl₂) for 1 hour and then washed 3 times with 0.1% TBST for 10 minutes. The GppNHp-bound KRas protein was diluted to various concentrations of 100 nM, 10 nM and 1 nM, and the GDP-bound KRas protein was diluted to a concentration of 100 nM in 4% TBSB, bound for 1 hour at room temperature, and washed 3 times with 0.1% TBST for 10 minutes. The result was bound to an HRP-conjugated anti-his antibody (HRP-conjugated anti-his mAb) as a labeling antibody. The result was reacted with TMB ELISA solution, and the absorbance at 450 nm was quantified.

In addition, ELISA analysis was performed using a BIACORE2000 instrument to determine the quantitative affinity for GppNHp-bound KRas^{G12D}, of the affinity-improved anti-Ras·GTP iMabs having higher antigen-binding ability than RT22-i33.

Specifically, in the same manner as in Example 4, RT22-i33 was used as a control group, and RT31-i33, RT34-i33 and RT36-i33 were diluted in 10 mM Na-acetate buffer (pH 4.0) and immobilized in an amount of approximately 1600 response units (RU) on a CM5 sensor chip (GE Healthcare) . Subsequently, Tris buffer (20 mM Tris-HCl, pH 8.0, 100 mM NaCl, 5 mM MgCl₂, 0.01% Tween 20) was analyzed at a flow rate of 30 µl/min, and GppNHp-bound KRas^{G12D} was analyzed at a concentration of 100 nM to 6.25 nM. After binding and dissociation analysis, regeneration of the CM5 chip was performed by flowing a buffer (20mM NaOH, 1M NaCl, pH 10.0) at a flow rate of 30 µl/min for 1.5 minutes. Each sensorgram, obtained by binding for 3 minutes and dissociation for 3 minutes, was normalized with reference to a blank cell and subtracted and the affinity was thus calculated.

The following Table 6 shows the result of affinity analysis of anti-Ras·GTP iMab having improved affinity for GppNHp-bound KRas^{G12D} using SPR (BIACORE 2000).

**[Table 6]**

| **KRas^{G12D.} GppNHp** | ***k*ₐ (M⁻¹s⁻¹)** | ***k*_{d} (s⁻¹)** | ***K*_{D}(M)** |
|---|---|---|---|
| **RT22-i33** | 9.92 ± 0.56 × 10⁴ | 8.44 ± 0.51 × 10⁻⁴ | 8.51 ± 0.82 × 10⁻⁹ |
| **RT31-i33** | 8.81 ± 0.42 × 10⁴ | 1.52 ± 0.13 × 10⁻⁴ | 1.72 ± 0.19 × 10⁻⁹ |
| **RT34-i33** | 6.2 ± 0.45 × 10⁴ | 2.28 ± 0.13 × 10⁻⁴ | 3.67 ± 0.39 × 10⁻⁹ |
| **RT36-i33** | 3.96 ± 0.24 × 10⁴ | 1.26 ± 0.05 × 10⁻⁴ | 3.19 ± 0.26 × 10⁻⁹ |

The results showed that all of RT31-i33, RT34-i33, and RT36-i33, which are anti-Ras·GTP iMabs with improved affinity based on RT22 VH, have higher affinity than the conventional RT22-i33.

### Example 8. Logic of development of light-chain variable region (VL) with improved cytoplasmic penetration ability specific for tumor tissue cells and endosomal escape ability

The results showed that the cytoplasm-penetrating anti-RasGTP iMab of the prior patents (Korean Patent Nos. 10-1602870 and 10-1602876) was constructed by substituting the heavy-chain variable region of the conventional cytoplasmic penetration antibody (cytotransmab), having cytoplasmic penetration ability, with the heavy-chain variable region (VH) having binding ability highly specific to Ras·GTP. The cytoplasmic penetration antibody (cytotransmab) is an antibody capable of penetrating into the cytoplasm based on the light-chain variable region (VL), and cell penetration is initiated through binding with heparan sulfate proteoglycan (HSPG) on the cell surface. However, HSPG is a cell-surface protein that is highly expressed in normal cells as well, and thus HSPG-binding ability thereof needs to be suppressed in order to modify the protein to be capable of penetrating specifically into the cytoplasm of tumor tissue cells.

Therefore, the present inventors found that CDR1 and CDR2, in hT4 VL of the light-chain variable region (VL) of the cytoplasmic penetration antibody (cytotransmab), which are expected to contribute to HSPG binding, were substituted with CDR sequences that have amino acid sequences having the same number of amino acids in the human germline sequence and not including a cationic patch sequence of CDR1, which is responsible for endocytosis. At this time, amino acids known to be important for the stability of the conventional light-chain variable region were preserved.

The light-chain variable region developed based on this logic is hT4-03 (Korean Patent Application No. 10-2016-0065365) .

In order to further improve the endosome escape ability of this light-chain variable region, CDR3 in where WYW (Trp-Tyr-Trp) is located was introduced into residues 92-94 of the light-chain variable region (VL) (Koran Patent Application No. 10-2016-0065365), and residue 87 of the light-chain variable region framework region corresponding to the interface between the light-chain variable region (VL) and the heavy-chain variable region (VH) was substituted with phenylalanine (Phe) in order to overcome the increased exposure of hydrophobic residues to solvents and thus decreased production yield due to the introduction of WYW (Korean Patent Application No. 10-2016-0065365).

This was designated as "hT4-33 light-chain variable region" (VL).

The following Table 7 shows the sequence and name of the light-chain variable region (hT4-33 VL) having improved cytoplasmic penetration specific for tumor tissue cells and endosome escape ability constructed using an overlapping PCR technique.

**[Table 7]**

| VL name | Sequence | SEQ ID NO. |
|---|---|---|
| **hT4-3 VL** | 10 20 abcdef 30 40 50 DLVMTQSPSSLSASVGDRVTITCKSSQSLFNSRTRKNYLAWYQQKPGKAPKLLIYW 60 70 80 90 100 ASTRESGVPSRFSGSGSGTDFTLTISSLQPEDFATYFCQQYWYWMYTFGQGTKVEIKR | **33** |
| **hT4-03 VL** | 10          20          abcdef     30          40          50 DLVMTQSPSSLSASVGDRVTITCKSSQSLLDSDDGNTYLAWYQQKPGKAPKLLIYW 60          70          80          90          100 LSYRASGVPSRFSGSGSGTDFTLTISSLQPEDFATYYCQQYYYHMYTFGQGTKVEIKR | **34** |
| **hT4-33 VL** | 10          20     abcdef  30          40          50 DLVMTQSPSSLSASVGDRVTITCKSSQSLLDSDDGNTYLAWYQQKPGKAPKLLIYW 60          70          80          90          100 LSYRASGVPSRFSGSGSGTDFTLTISSLQPEDFATYFCQQYWYWMYTFGQGTKVEIKR | **35** |

### Example 9. Selection of protopeptides for targeting tumor-tissue-specific cell surface proteins and inducing endocytosis.

RT11, which is an anti-Ras ·GTP iMab using hT4 VL, enters cells and binds to Ras·GTP in the cells to exhibit tumor growth inhibitory ability, but has the disadvantage of low tumor-tissue specificity due to the HSPG-binding ability thereof. In order to overcome this disadvantage, the light chain having reduced HSPG-binding capacity was obtained through Example 8 above and experiments were conducted using an iMab form introduced with a light-chain variable region fused at the N-terminal thereof with RGD10 protopeptides for targeting integrin receptors as in Examples above.

Additionally, various protopeptides to impart tumor-tissue specificity were selected in addition to the integrin-targeting protopeptides.

The EpCAM (epithelial cell adhesion molecule) receptor is a receptor that is mainly overexpressed in colorectal cancer cells, and is a target located on a suitable cell membrane for imparting tumor specificity to anti-Ras·GTP iMab. Accordingly, a light-chain variable region including an Ep133 protopeptide (EHLHCLGSLCWP) capable of targeting the EpCAM receptor (US Patent Application No. 14/428,017) fused with the N-terminus thereof was constructed.

The following Table 8 shows the sequences and names of protopeptides for targeting tumor-tissue-specific cell surface proteins and inducing endocytosis.

**[Table 8]**

| Peptide name | Target antigen | Sequence |
|---|---|---|
| **RGD10** | **Integrin αvβ3/αvβ5** | DGARYCRGDCFDG |
| **EP133** | **EpCAM** | EHLHCLGSLCWP |

### Example 10. Construction and purification of anti-Ras·GTP iMab introduced with light-chain variable region (VL) fused with target protopeptide for tumor-tissue-specific cytoplasmic penetration

FIG. 6 is a schematic diagram illustrating the construction of complete IgG-type anti-Ras·GTP iMabs (RT22-i33, RT22-ep33) having improved affinity including a light-chain variable region (VL) fused with integrin- or EpCAM-targeting protopeptides and having inhibited binding capacity to HSPG.

Specifically, for expression in animal cells of iMab fused with protopeptides, protopeptides were genetically fused to the N-terminus of hT4-33, the light-chain variable region (VL) using two G4S linkers. Subsequently, a DNA encoding a light chain including the protopeptide-fused hT4-33 light-chain variable region and a light-chain constant region (CL) was cloned with NotI/BsiWI into a pcDNA3.4 vector fused with a DNA encoding a secretory signal peptide at the 5' end thereof.

The heavy-chain variable region (RT22 VH) having specific binding ability to GTP-bound Ras and the cytoplasmic penetration humanized light chain expression vector were subjected to transient co-transfection into the HEK293F-protein-expressing cells to express individual clones in the same manner as in Example 4, and were purified in the same manner as in Example 4.

The following Table 9 shows the production yield of cytotransmab and anti-Ras·GTP iMab including a light-chain variable region (VL) fused with a tumor-tissue-specific protopeptide using a genetic-engineering technique.

**[Table 9]**

| IgG1,κ format | Heavy chain | Light chain | | | HSPG binding | Production yield (mg/1 L of transfected cells)^{a} |
|---|---|---|---|---|---|---|
| | VH | Targeting peptide | Linker | VL | | |
| RT22-i3 | RT22 | RGD10 | (G₄S)₂ | hT4-3 | +++ | 9.05 ± 0.78 |
| RT22-i33 | | | | hT4-33 | - | < 1 |
| RT22-ep33 | | Ep133 | | | - | <1 |
| CT-i3 | TMab4 | RGD10 | (G₄S)₂ | hT4-3 | +++ | 8.66 ± 0.34 |
| CT-i33 | | | | hT4-33 | - | < 1 |
| CT-ep33 | | Ep133 | | | - | < 1 |

| | | | | | | |
|---|---|---|---|---|---|---|
| ^{a}The 2 plasmids that encode the HC and LC of each IgG antibody were co-transfected with the equivalent molar ratio into HEK293F cells in 1 L of culture media. After 6 d of culture, antibodies were purified from the cell culture supernatant using a protein-A affinity column. | | | | | | |

It was found that the production yield of the anti-RasGTP iMab fused with integrin- or EpCAM-targeting protopeptide was significantly low, that is, 1 mg/1 L.

### Example 11. Determination of specific binding with intracellular Ras·GTP of RT22-i33 MG, RT22-ep33 MG

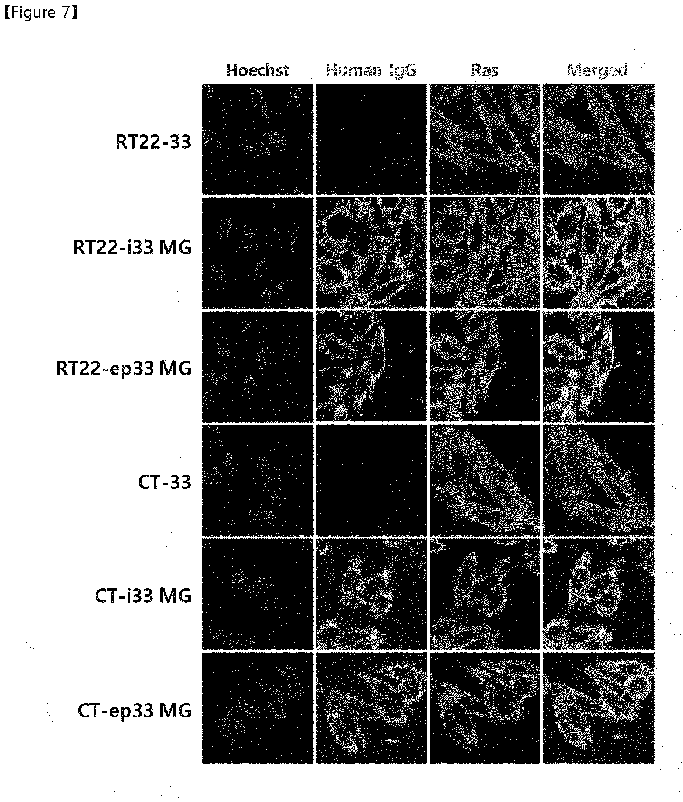

FIG. 7 shows the result of confocal microscopy analysis to determine the overlap with the activated intracellular Ras after treating the KRas mutant colorectal cancer cell line SW480 with complete IgG-type anti-Ras·GTP iMabs (RT22-33 (1 µM), RT22-i33 MG (0.5 µM), RT22-ep33 MG (1 µM)) having improved affinity including a light-chain variable region (VL) fused with integrin- or EpCAM targeting protopeptide and having cytoplasmic penetration ability specific for tumor tissue cells, and with cytotransmab as control groups (CT-33 (1 µM), CT-i33 MG (0.5 µM), CT-ep33 MG (1 µM)).

Specifically, SW480 cells were prepared in the same manner as in Example 4, treated with RT22-33 1 µM, RT22-i33 MG 0.5 µM, RT22-ep33 MG 1 µM, CT-33 1 µM, CT-i33 MG 0.5 µM, and CT-ep33 MG 1 µM, and then cultured at 37°C for 12 hours. Then, the cells were stained under the same conditions as in Example 4 and observed with a confocal microscope. RT22-i33 MG and RT22-ep33 MG, except for RT22-33, which does not undergo endocytosis, were found to have fluorescence overlapping that of intracellular Ras. In addition, CT-33, CT-i33 MG and CT-ep33 MG, not targeting intracellular Ras, were found to have no fluorescence overlapping that of intracellular Ras.

### Example 12. Design of CDR1 and CDR2 mutants of light-chain variable region (VL) to improve yield of tumor-tissue-specific anti-RasGTP iMab

Like the results of analysis of Example 10, it was found that the production yield did not decrease upon fusion of the protopeptide with the conventional light-chain variable region (hT4-3 VL) having HSPG-binding ability, but the production yield decreased upon fusion with the light-chain variable region (hT4-33 VL) having reduced HSPG-binding ability. In addition, CDR1 and CDR2 of the light-chain variable region (VL), which greatly affects HSPG-binding ability, were modified to improve production yield upon fusion with protopeptides recognizing specifically for tumor tissues (RGD10, Ep133).

Specifically, in order to maintain low binding capacity to HSPG, a mutation was formed in common by substituting the phenylalanine, residue 27c, which is considered to affect the formation of the loop structure of CDR1 of the light-chain variable region of hT4 VL, with leucine, preserved in the light-chain variable region having reduced HSPG-binding capacity, and mutations were formed in residues 27f to 30, which are considered to be located at the tip of the CDR1 loop structure due to the canonical structure and thus to be exposed to the side chain.

Specifically, in the case of hT4-34 VL, a point mutation was formed only in the residue 27c, which is considered to affect the formation of the loop structure of CDR1, among CDR1 and CDR2 of the light-chain variable region, using hT4 VL as a template.

Specifically, hT4-35 VL, hT4-36 VL and hT4-37 VL were mutated according to each strategy using hT4-33 VL having greatly reduced HSPG-binding ability as a template. The hT4-35 VL was designed by respectively mutating the aspartate at residues 27d and 27f of CDR1 to asparagine and arginine, preserved in hT4 VL. The hT4-36 VL was designed by respectively mutating aspartate and glycine at residues 27d and 29 of CDR1 to asparagine and arginine, and hT4-37 VL was designed by respectively mutating aspartate and asparagine at residues 27d and 30 of CDR1 to asparagine and lysine. The residue numbers followed the Kabat number.

Specifically, hT4-38 VL was designed by mutating arginine at residue 29 to glycine present in the sequence of hT4-33 VL and mutating asparagine at residue 31 to threonine present in the sequence of hT4-33 VL, while preserving arginine at residue 27f and lysine at residue 30, which had increased yield when maintaining the sequence of hT4 VL in order to minimize HSPG-binding capacity. Like hT4-38 VL, hT4-39 VL was designed by mutating threonine at residue 28 to aspartate and mutating arginine at residue 29 to glycine while preserving arginine at residue 27f and lysine at residue 30 in order to minimize HSPG-binding capacity using the sequence preserved in hT4-33 VL.

The following Table 10 shows the sequence of a light-chain variable region (VL) having a mutation for increasing production yield upon tumor-tissue-cell-specific cytoplasmic penetration and protopeptide fusion.

**[Table 10]**

| VL name | Sequence | SEQ ID NO. |
|---|---|---|
| **hT4-34 VL** | 1          10         20    abcdef  30         40         50 DLVMTQSPSSLSASVGDRVTITCKSSQSLLNSRTRKNYLAWYQQKPGKAPKLLIYW 60         70         80         90         100 ASTRESGVPSRFSGSGSGTDFTLTISSLQPEDFATYFCQQYWYWMYTFGQGTKVEIKR | **36** |
| **hT4-3S VL** | 1 10 20 abcdef 30 40 50 DLVMTQSPSSLSASVGDRVTITCKSSQSLLNSRDGNTYLAWYQQKPGKAPKLLIYW 60 70 80 90 100 LSYRASGVPSRFSGSGSGTDFTLTISSLQPEDFATYFCQQYWYWMYTFGQGTKVEIKR | **37** |
| **hT4-36 VL** | 1 10 20 abcdef 30 40 50 DLVMTQSPSSLSASVGDRVTITCKSSQSLLNSDDRNTYLAWYQQKPGKAPKLLIYW 60 70 80 90 100 LSYRASGVPSRFSGSGSGTDFTLTISSLQPEDFATYFCQQYWYWMYTFGQGTKVEIKR | **38** |
| **hT4-37 VL** | 1 10 20 abcdef 30 40 50 DLVMTQSPSSLSASVGDRVTITCKSSQSLLNSDDGKTYLAWYQQKPGKAPKLLIYW 60 70 80 90 100 LSYRASGVPSRFSGSGSGTDFTLTISSLQPEDFATYFCQQYWYWMYTFGQGTKVEIKR | **39** |
| **hT4-38 VL** | 1 10 20 abcdef 30 40 50 DLVMTQSPSSLSASVGDRVTITCKSSQSLLNSRTGKTYLAWYQQKPGKAPKLLIYW 60 70 80 90 100 ASTRESGVPSRFSGSGSGTDFTLTISSLQPEDFATYFCQQYWYWMYTFGQGTKVEIKR | **40** |
| **hT4-39 VL** | 1 10 20 abcdef 30 40 50 DLVMTQSPSSLSASVGDRVTITCKSSQSLLNSRDGKNYLAWYQQKPGKAPKLLIYW 60 70 80 90 100 ASTRESGVPSRFSGSGSGTDFTLTISSLQPEDFATYFCQQYWYWMYTFGQGTKVEIKR | **41** |

### Example 13. Determination of HSPG-binding ability of anti-Ras·GTP iMab introduced with mutants of CDR1 and CDR2 of light-chain variable region (VL) having improved production yield and tumor-tissue-cell-specific cytoplasmic penetration

The decrease degree in HSPG-binding ability of anti-Ras·GTP iMab introduced with mutants of CDR1 and CDR2 of a light-chain variable region (VL) for improving production yield and inhibiting HSPG-binding ability constructed in Example 12 above, compared to cytotransmab (TMab4) using a conventional hT4 light-chain, was observed using a confocal microscope and cell-based ELISA.

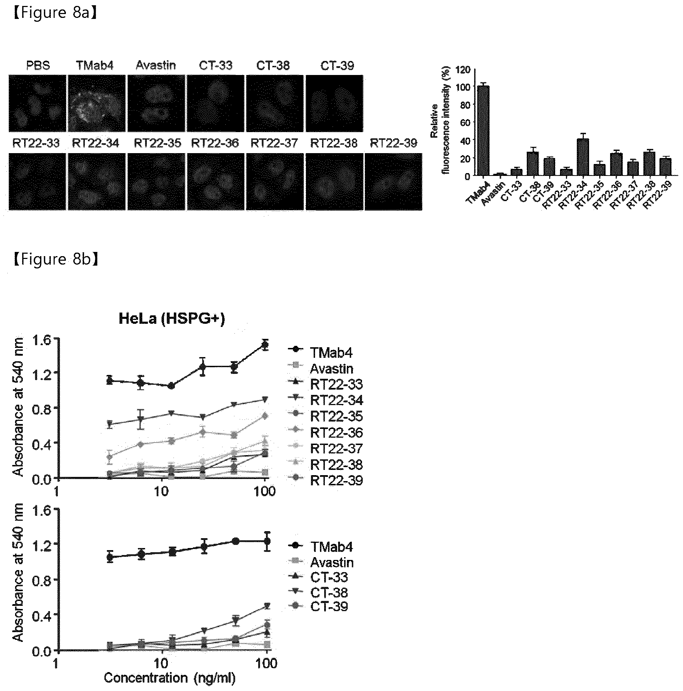

FIG. 8A shows the result of confocal microscopy measured after treatment of HeLa cells with 1 µM of the antibody at 37°C for 6 hours to determine a decrease in, or removal of HSPG-binding ability and cytoplasmic penetration of cytotransmab and the anti-Ras·GTP iMab including the light-chain variable region introduced with mutants of CDR1 and CDR2 for improving production yield and inhibiting HSPG-binding ability, and is a bar graph showing quantified Alexa488 fluorescence (green fluorescence).

Specifically, the HeLa cell line expressing HSPG was seeded at 5 × 10⁴ cells/well in a 24-well plate in 0.5 ml of a medium containing 10% FBS and cultured at 5% CO₂ and 37 degrees for 12 hours. When the cells were stabilized, PBS, TMab4, RT22-33, RT22-34, RT22-35, RT22-36, RT22-37, RT22-38, RT22-39, CT-33, CT-38 and CT-39 were cultured at 1 µM at 37 degrees for 6 hours. After washing with PBS and a weakly acidic solution in the same manner as in Example 4, the cells were immobilized, perforated and then blocked. Each antibody was stained with an antibody that specifically recognizes human Fc conjugated with Alexa488 (green fluorescence). Then, the nuclei were stained (blue fluorescence) using Hoechst33342 and observed under a confocal microscope.

The results showed that anti-RasGTP iMabs located in the cytoplasm through HSPG exhibited decreasing fluorescence intensity in the order of TMab4 (100%), RT22-34 (40%), RT22-36 (25%), RT22-38 (26%), RT22-39 (19%), RT22-37 (15.5%), RT22-35 (12.4%), and RT22-33 (6.8%).

FIG. 8B shows the result of non-specific cell-surface-binding ELISA in the HeLa cell line expressing HSPG to determine the HSPG-binding ability of cytotransmab and the anti-Ras·GTP iMab including the light-chain variable region introduced with mutants of CDR1 and CDR2 for improving production yield and inhibiting HSPG-binding ability.

Specifically, the HeLa (HSPG⁺) cell line was cultured in a 96-well plate such that the cells completely filled the bottom of the well, and was then washed three times with a washing buffer (HBSS buffer, 50 mM HEPES). Then, TMab4, RT22-33, RT22-34, RT22-35, RT22-36, RT22-37, RT22-38, RT22-39, CT-33, CT-38 and CT-39 were diluted to concentrations of 100, 50, 25, 12.5, 6.25, 3.125 ng/ml in a blocking buffer (HBSS buffer, 50 mM HEPES, 1% BSA) and cultured for 2 hours at 4°C. After washing three times with a washing buffer, the cells were linked to an HRP-conjugated anti-human mAb as a labeling antibody. The cells were reacted with TMB ELISA solution, and the absorbance at 450 nm was quantified.

With the same behavior as in FIG. 8A, cell-surface binding capacity was measured in the order of TMab4, RT22-34, RT22-38, RT22-36, RT22-39, RT22-37, RT22-35 and RT22-33.

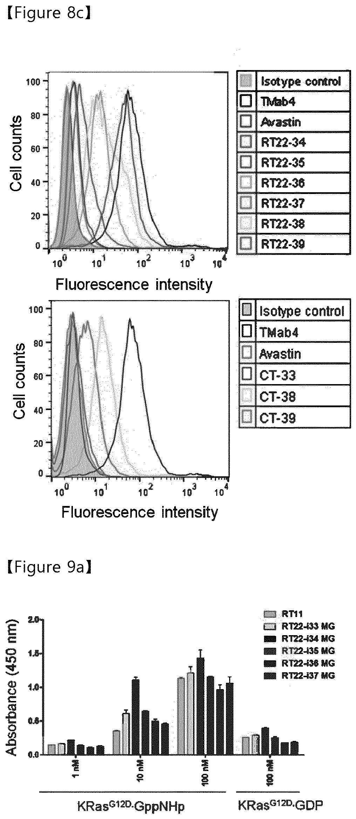

FIG. 8C shows the results of flow cytometry using FACS after treatment of the HeLa cell line with 500 nM antibody to determine the HSPG-binding ability of the anti-Ras·GTP iMab including the light-chain variable region introduced with mutants of CDR1 and CDR2 for improving production yield and inhibiting HSPG-binding ability.

Specifically, 1x10⁵ HeLa (HSPG⁺) cells were prepared for each sample. The cells were cultured in PBSF (PBS buffer, 2% BSA) supplemented with 500 nM of TMab4, RT22-33, RT22-34, RT22-35, RT22-36, RT22-37, RT22-38, and RT22-39 for 1 hour at 4°C. Then, each antibody was reacted with an antibody specifically recognizing human Fc conjugated to Alexa488 (green fluorescence) for 30 minutes at 4°C. The resulting product was washed with PBS and analyzed using flow cytometry. The result showed that fluorescence intensity binding to the cells was measured in the order of TMab4, RT22-34, RT22-38, RT22-36, RT22-39, RT22-37, RT22-35 and RT22-33, in the same behavior as in the confocal microscopy results.

Anti-Ras ·GTP iMab using the hT4-34 light chain having a remaining HSPG-binding capacity of 40%, which was found based on the result, was not used in subsequent biochemical identification experiments.

### Example 14. Expression and purification of tumor-tissue-specific anti-Ras·GTP iMab introduced with mutants of CDR1 and CDR2 of light-chain variable region (VL) having improved production yield and tumor-tissue-cell-specific cytoplasmic penetration

RGD or Ep133 protopeptides were fused to the light-chain variable regions (VL) introduced with mutants for providing tumor-tissue-cell-specific cytoplasmic penetration and improving production yield to conduct cloning upon fusion with protopeptides using a Mg linker using a genetic-engineering technique in the same manner as in Example 10.

The following Table 11 shows the sequence of the light-chain variable regions (VL) having improved production yield and tumor-tissue-cell-specific cytoplasmic penetration ability and being fused with an integrin-targeting protopeptide.

**[Table 11]**

| VL name | Sequence | SEQ ID NO. |
|---|---|---|
| **hT4-i34 MG VL** | 1 10 20 30 40 50 60 DGARYCRGDCFDGMGSSSNDLVMTQSPSSLSASVGDRVTITCKSSQSLLNSRTRKNYLAW 70 80 90 100 110 120 YQQKPGKAPKLLIYWASTRESGVPSRFSGSGSGTDFTLTISSLQPEDFATYFCQQYWYWM 130 YTFGQGTKVEIKR | **44** |
| **hT4-i35 MG VL** | 1 10 20 30 40 50 60 DGARYCRGDCFDGMGSSSNDLVMTQSPSSLSASVGDRVTITCKSSQSLLNSRDGNTYLAW 70 80 90 100 110 120 YQQKPGKAPKLLIYWLSYRASGVPSRFSGSGSGTDFTLTISSLQPEDFATYFCQQYWYWM 130 YTFGQGTKVEIKR | **45** |
| **hT4-i36 MG VL** | 1                    10                   20                   30                   40                   50                   60 DGARYCRGDCFDGMGSSSNDLVMTQSPSSLSASVGDRVTITCKSSQSLLNSDDRNTYLAW 70                   80                   90                   100                  110                  120 YQQKPGKAPKLLIYWLSYRASGVPSRFSGSGSGTDFTLTISSLQPEDFATYFCQQYWYWM 130 YTFGQGTKVEIKR | **46** |
| **hT4-i37 MG VL** | 1                    10                   20                   30                   40                   50                   60 DGARYCRGDCFDGMGSSSNDLVMTQSPSSLSASVGDRVTITCKSSQSLLNSDDGKTYLAW 70                   80                   90                   100                  110                  120 YQQKPGKAPKLLIYWLSYRASGVPSRFSGSGSGTDFTLTISSLQPEDFATYFCQQYWYWM 130 YTFGQGTKVEIKR | **47** |
| **hT4-i38 MG VL** | 1 10 20 30 40 50 60 DGARYCRGDCFDGMGSSSNDLVMTQSPSSLSASVGDRVTITCKSSQSLLNSRTGKTYLAW 70 80 90 100 110 120 YQQKPGKAPKLLIYWASTRESGVPSRFSGSGSGTDFTLTISSLQPEDFATYFCQQYWYWM 130 YTFGQGTKVEIKR | **48** |
| **hT4-i39 MG VL** | 1          10          20          30          40          50          60 DGARYCRGDCFDGMGSSSNDLVMTQSPSSLSASVGDRVTITCKSSQSLLNSRDGKNYLAW 70          80          90          100          110          120 YQQKPGKAPKLLIYWASTRESGVPSRFSGSGSGTDFTLTISSLQPEDFATYFCQQYWYWM 130 YTFGQGTKVEIKR | **49** |

The following Table 12 shows the sequences of the light-chain variable regions (VL) having a mutation for improving production yield and inhibiting HSPG-binding ability, and fused with EpCAM-targeting protopeptide.

**[Table 12]**

| VL name | Sequence | SEQ ID NO. |
|---|---|---|
| **hT4-ep33 MG VL** | 1 10 20 30 40 50 60 EHLHCLGSLCWPMGSSSNDLVMTQSPSSLSASVGDRVTITCKSSQSLLDSDDGNTYLAWY 70 80 90 100 110 120 QQKPGKAPKLLIYWASTRESGVPSRFSGSGSGTDFTLTISSLQPEDFATYFCQQYWYWMY 130 TFGQGTKVEIKR | **50** |
| **hT4-ep34 MG VL** | 1          10          20          30          40          50          60 EHLHCLGSLCWPMGSSSNDLVMTQSPSSLSASVGDRVTITCKSSQSLLNSRTRKNYLAWY 70          80          90          100         110         120 QQKPGKAPKLLIYWASTRESGVPSRFSGSGSGTDFTLTISSLQPEDFATYFCQQYWYWMY 130 TFGQGTKVEIKR | **51** |
| **hT4-ep35 MG VL** | 1 10 20 30 40 50 60 EHLHCLGSLCWPMGSSSNDLVMTQSPSSLSASVGDRVTITCKSSQSLLNSRDGNTYLAWY 70 80 90 100 110 120 QQKPGKAPKLLIYWLSYRASGVPSRFSGSGSGTDFTLTISSLQPEDFATYFCQQYWYWMY 130 TFGQGTKVEIKR | **52** |
| **hT4-ep36 MG VL** | 1 10 20 30 40 50 60 EHLHCLGSLCWPMGSSSNDLVMTQSPSSLSASVGDRVTITCKSSQSLLNSDDRNTYLAWY 70 80 90 100 110 120 QQKPGKAPKLLIYWLSYRASGVPSRFSGSGSGTDFTLTISSLQPEDFATYFCQQYWYWMY 130 TFGQGTKVEIKR | **53** |
| **hT4-ep37 MG VL** | 1 10 20 30 40 50 60 EHLHCLGSLCWPMGSSSNDLVMTQSPSSLSASVGDRVTITCKSSQSLLNSDDGKTYLAWY 70 80 90 100 110 120 QQKPGKAPKLLIYWLSYRASGVPSRFSGSGSGTDFTLTISSLQPEDFATYFCQQYWYWMY 130 TFGQGTKVEIKR | **54** |
| **hT4-ep36 MG VL** | 1 10 20 30 40 50 60 EHLHCLGSLCWPMGSSSNDLVMTQSPSSLSASVGDRVTITCKSSQSLLNSRTGKTYLAWY 70 80 90 100 110 120 QQKPGKAPKLLIYWASTRESGVPSRFSGSGSGTDFTLTISSLQPEDFATYFCQQYWYWMY 130 TFGQGTKVEIKR | **55** |
| **hT4-ep39 MG VL** | 1 10 20 30 40 50 60 EHLHCLGSLCWPMGSSSNDLVMTQSPSSLSASVGDRVTITCKSSQSLLNSRDGKNYLAWY 70 80 90 100 110 120 QQKPGKAPKLLIYWASTRESGVPSRFSGSGSGTDFTLTISSLQPEDFATYFCQQYWYWMY 130 TFGQGTKVEIKR | **56** |

A RT22 heavy-chain animal expression vector and a light-chain expression vector including a light-chain variable region having CDR1 and CDR2 mutations fused with integrin-targeting protopeptide for providing tumor-tissue specificity and improving production yield, were subjected to transient co-transfection into HEK293F-protein-expressing cells in the same manner as in Example 4, and then the process of purifying the antibody was conducted in the same manner as in Example 4.

In addition, the quantitative affinity for GppNHp-bound KRas^{G12D} of the anti-Ras·GTP iMabs introduced with CDR1 and CDR2 mutations in the light-chain variable region (VL) for improving production yield and inhibiting HSPG-binding ability was analyzed using a BIACORE2000 instrument.

Specifically, RT22-i35 MG, RT22-i37 MG, RT22-i38 MG, RT22-i39 MG, RT22-ep37 MG, RT22-ep38 MG and RT22-ep39 MG were diluted to a concentration of 20 µl/ml in 10 mM NaAc buffer (pH 4.0) and immobilized at approximately 1800 response units (RU) on a CM5 sensor chip (GE Healthcare) . Subsequently, Tris buffer (20 mM Tris-HCl, pH 7.4, 137 mM NaCl, 5 mM MgCl₂, 0.01% Tween 20) was analyzed at a flow rate of 30 µl/min, and complete GppNHp-bound GST-KRas^{G12D} was analyzed at a concentration of 50 nM to 3.125 nM. After binding and dissociation analysis, regeneration of the CM5 chip was performed by flowing a buffer (20 mM NaOH, 1M NaCl, pH 10.0) at a flow rate of 30 µl/min for 1 minute. Each sensorgram, obtained by binding for 3 minutes and dissociation for 3 minutes, was normalized with reference to a blank cell and subtracted, and the affinity was thus calculated.

The following Table 13 shows the production yield of the anti-RasGTP iMabs obtained by expressing an RT22 heavy chain in combination with hT4-i33 and hT4-ep33, and light chains (hT4-i34 MG to hT4-i39 MG and hT4-ep34 MG to hT4-ep39) introduced with mutations of CDR1 and CDR2 to improve production yield and inhibit HSPG-binding capacity, and of the cytotransmabs obtained by expressing a TMab4 heavy chain in combination with the light chains, and the result of analysis of the affinity of tumor-tissue-specific anti-Ras·GTP iMab based on SPR using a BIACORE2000 instrument.

**[Table 13]**

| IgG1,κ format | Heavy chain | Light chain | | | Production yield (mg/1 L of transfected cells)^{a} | KRas^{G12D.} GppNHp *K*_{D}(M)^{b} |
|---|---|---|---|---|---|---|
| | VH | Targeting peptide | Linker | VL | | |
| RT22-i33 MG | RT22 | RGD10 | MGSSSN | hT4-33 | 6.79 ± 1.85 | ND |
| RT22-134 MG | | | | hT4-34 | 13.3 ± 0.63 | ND |
| RT22-i35 MG | | | | hT4-35 | 11.2 ± 1.27 | 7 ± 0.41 × 10⁻⁹ |
| RT22-i36 MG | | | | hT4-36 | 6.2 ± 0.72 | ND |
| KT22-i37 MG | | | | hT4-37 | 12.1 ± 1.25 | 8.6 ± 0.35×10⁻⁹ |
| RT22-i38 MG | | | | hT4-38 | 45.1 ± 3.54 | 2.0 ± 0.02×10⁻⁹ |
| RT22-i39 MG | | | | hT4-39 | 75.1 ± 7.73 | 6.4 ± 0.5×10⁻⁹ |
| RT22-ep33 MG | | Ep133 | | hT4-33 | 2.8 ± 0.21 | ND |
| RT22-ep34 MG | | | | hT4-34 | 15.5 ± 1.48 | ND |
| RT22-ep35 MG | | | | hT4-35 | 2.0 ± 0.16 | ND |
| RT22-ep36 MG | | | | hT4-36 | 7.6 ± 0.41 | ND |
| RT22-ep37 MG | | | | hT4-37 | 16.5 ± 1.36 | 8.0 ± 0.28×10⁻⁹ |
| RT22-ep38 MG | | | | hT4-38 | 21 ± 0.88 | 3.6 ± 0.28×10⁻⁹ |
| RT22-ep39 MG | | | | hT4-39 | 37.1 ± 3.78 | 8.2 ± 0.16×10⁻⁹ |
| CT-i33 MC | TMab4 | RGD10 | | hT4-33 | 5.31 ± 0.25 | ND |
| CT-i38 MG | | | | hT4-38 | 31.8 ± 2.87 | ND |
| CT-i39 MG | | | | hT4-39 | 69.7 ± 5.34 | ND |
| CT.ep33 MG | | Ep133 | | hT4-33 | 2.6 ± 0.21 | ND |
| CT-op38 MG | | | | hT4-38 | 18.2 ± 0.92 | ND |
| CT-ep39 MG | | | | hT4-39 | 32.7 ± 2.04 | ND |

| | | | | | | |
|---|---|---|---|---|---|---|
| ^{a} The 2 plasmids that encode the HC and LC of each IgG antibody were co-transfected with the equivalent molar ratio into HEK293F cells in 1 L of culture media. After 6 d of culture, antibodies were purified from the cell culture supernatant using a protein-A affinity column. ^{b} ND, not determined | | | | | | |

It was found that the affinity for GppNHp-bound KRas^{G12D} of tumor-tissue-specific anti-Ras·GTP iMabs introduced with mutants of light-chain variable region (VL) CDR1 and CDR2 to improve production yield was not changed.

The anti-Ras·GTP iMab using the hT4-36 light chain, which did not increase the production yield, found through the above results, was not used in subsequent biochemical identification experiments.

### Example 15. Analysis of binding ability to GppNHp-bound KRas^{G12D} of tumor-tissue-specific anti-Ras·GTP iMab introduced with mutants of CDR1 and CDR2 of light-chain variable region (VL) having improved production yield and tumor-tissue-cell-specific cytoplasmic penetration

FIG. 9A shows the result of ELISA to analyze the binding ability to 1, 10 or 100 nM Avi-KRas^{G12D}·GppNHp or 100 nM Avi-KRas^{G12D}·GDP of anti-Ras·GTP iMab including light-chain variable regions (hT4-i33 MG∼hT4-i37 MG VL) fused with integrin-targeting protopeptide and having tumor-tissue-cell-specific cytoplasmic penetration.

Specifically, in order to compare the antigen-binding ability of the CDR1 and CDR2 mutant light chains, the heavy-chain variable region binding to GppNHp is fixed at RT22 VH, and analysis was conducted on RT11, RT22-i33 MG, RT22-i34 MG, RT22-i35 MG, RT22-i36 MG and RT22-i37 MG.

Specifically, ELISA was conducted in the same manner as Example 4, and tumor-tissue-specific anti-Ras·GTP iMabs introduced with mutants of CDR1 and CDR2 were fixed on a 96-well EIA/RIA plate, after which the GppNHp-bound KRas protein was bound at various concentrations of 100 nM, 10 nM and 1 nM and the GDP-bound KRas protein was bound at a concentration of 100 nM and was then bound to an HRP-conjugated anti-his antibody (HRP-conjugated anti-his mAb) as a labeling antibody. The result was reacted with TMB ELISA solution, and the absorbance at 450 nm was quantified.

The result of analysis of the antigen-binding ability by ELISA showed that, compared to the conventional RT22-i33, hT4-i34 VL, maintaining the cation patch of CDR1, had improved binding capacity to the GppNHp-bound KRas^{G12D} antigen due to the light chain, while the heavy chain was the same as in the conventional case.

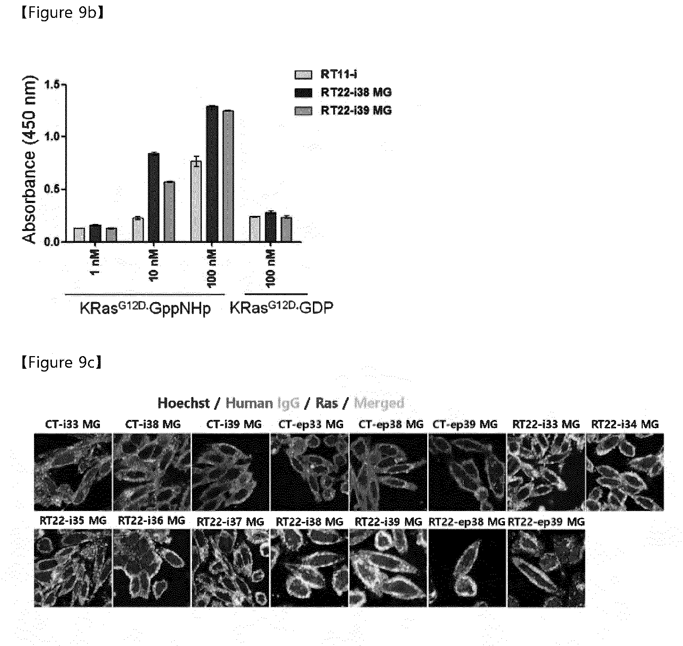

FIG. 9B shows the result of ELISA to analyze the binding ability to 1, 10 or 100 nM Avi-KRas^{G12D}·GppNHp or 100 nM Avi-KRas^{G12D}·GDP of cytotransmab and anti-Ras·GTP iMabs including light-chain variable regions (hT4-i38 MG to hT4-i39 MG VL) fused with integrin-targeting protopeptide and having inhibited binding ability to HSPG.

Specifically, ELISA was conducted in the same manner as Example 4, and cytotransmabs and tumor-tissue-specific anti-Ras·GTP iMabs introduced with mutants of CDR1 and CDR2 were fixed on a 96-well EIA/RIA plate, after which the GppNHp-bound KRas protein was bound at various concentrations of 100 nM, 10 nM and 1 nM and the GDP-bound KRas protein was bound at a concentration of 100 nM and then was bound to an HRP-conjugated anti-his antibody (HRP-conjugated anti-his mAb) as a labeling antibody. The result was reacted with TMB ELISA solution, and the absorbance at 450 nm was quantified.

The result of analysis of the antigen-binding ability by ELISA showed that, compared to the conventional RT11-i, hT4-38 and hT4-39 light-chain variable regions improved binding capacity to the GppNHp-bound KRas^{G12D} antigen.

FIG. 9C shows the result of confocal microscopy analysis to determine the overlap with the activated intracellular Ras after treating the KRas mutant colorectal cancer cell line SW480 at 37°C for 12 hours with 0.5 uM of cytotransmabs and anti-Ras·GTP iMabs including light-chain variable regions (VL) fused with integrin- or EpCAM-targeting protopeptide and having inhibited binding ability to HSPG.

Specifically, SW480 cells were prepared in the same manner as in Example 4, and were treated with 1 µM CT-i33 MG (=TMab4-i33 MG), CT-i38 MG, CT-i39 MG, RT22-i33 MG, RT22-i34 MG, RT22-i35 MG, RT22-i36 MG, RT22-i37 MG, RT22-i38 MG, RT22-i39 MG, CT-ep33 MG, CT-ep38 MG, CT-ep39 MG, RT22-ep38 MG and RT22-ep39 MG, and then cultured for 12 hours at 37°C under 5% CO₂. Then, the cells were stained under the same conditions as in Example 4 and observed with a confocal microscope. RT22-i33 MG, RT22-i34 MG, RT22-i35 MG, RT22-i36 MG, RT22-i37 MG, RT22-i38 MG, RT22-i39 MG, RT22-ep38 MG, and RT22-ep39 MG, excluding CT-i33 MG, CT-i38 MG, CT-i39 MG, CT-ep33 MG, CT-ep38 MG and CT-ep39 MG, were found to have fluorescence overlapping that of the intercellular Ras.

### Example 16. Construction of light-chain variable-region (VL) mutants with modified antibody framework to improve production yield

The light-chain variable region (VL) CDR1 mutants constructed in Example 12 increased yield when fused with the protopeptide for providing tumor-tissue specificity compared to the conventional hT4-33, but antibodies (RT22-ep38 MG, RT22-ep39 MG) fused with the Ep133 protopeptide, showing the best production yield, exhibited production yields that were approximately 2 times lower than antibodies fused with RGD10 protopeptides (RT22-i38 MG, RT22-i39 MG) . Therefore, in order to further increase the stability and yield of the antibody fused with the Ep133 protopeptide, an additional mutant of the light-chain variable region (VL) antibody framework was constructed.

Specifically, most of sequences 2 and 3 of the antibody skeleton in the light chain used in the human complete IgG-type antibody include isoleucine and glutamine, present in human germline sequences, but the sequences 2 and 3 are preserved as leucine and valine in the light chain used in Anti-Ras·GTP iMab. Thus, a light-chain variable region (VL) antibody framework mutant was constructed by mutating these sequences 2 and 3 to isoleucine and glutamine, which are mainly present in human complete-IgG type antibodies, in order to increase the stability and yield.

The following Table 14 shows sequence information of a light-chain variable region (VL) having a modified antibody skeleton to improve the production yield based on hT4-38 and hT4-39 and a light-chain variable region (VL) fused with EpCAM-targeting protopeptide and having a modified antibody skeleton to improve the production yield.

**[Table 14]**

| VL name | Sequence | SEQ ID NO. |
|---|---|---|
| hT4-S8 VL | 1 10 20 abcdef 30 40 50 DIQMTQSPSSLSASVGDRVTITCKSSQSLLNSRTGKTYLAWYQQKPGKAPKLLIYW 60 70 80 90 100 ASTRESGVPSRFSGSGSGTDFTLTISSLQPEDFATYFCQQYWYWMYTFGQGTKVEIKR | 42 |
| hT4-59 VL | 1 10 20 abcdef 30 40 50 DIQMTQSPSSLSASVGDRVTITCKSSQSLLNSRDGKNYLAWYQQKPGKAPKLLIYW 60 70 80 90 100 ASTRESGVPSRFSGSGSGTDFTLTISSLQPEDFATYFCQQYWYWMYTFGQGTKVEIKR | 43 |
| hT4-ep58 MG VL | 1          10          20          30          40          50          60 EHLHCLGSLCWPMGSSSNDIQMTQSPSSLSASVGDRVTITCKSSQSLLNSRTGKTYLAWY 70          80          90          100          110          120 QQKPGKAPKLLIYWASTRESGVPSRFSGSGSGTDFTLTISSLQPEDFATYFCQQYWYWMY 130 TFGQGTKVEIKR | 57 |
| hT4-ep59 MG VL | 1          10          20          30          40          50          60 EHLHCLGSLCWPMGSSSNDIQMTQSPSSLSASVGDRVTITCKSSQSLLNSRDGKNYLAWY 70          80          90          100          110          120 QQKPGKAPKLLIYWASTRESGVPSRFSGSGSGTDFTLTISSLQPEDFATYFCQQYWYWMY 130 TFGQGTKVEIKR | 58 |

### Example 17. Expression and purification of tissue-cell-specific anti-Ras·GTP iMab introduced with light-chain variable-region (VL) mutants having modified antibody framework to improve production yield

A RT22 heavy-chain animal expression vector and a light-chain expression vector including a light-chain variable region fused with EpCAM-targeting protopeptide for providing tumor-tissue specificity, and having a modified antibody framework to improve the production yield, were subjected to transient co-transfection into HEK293F-protein-expressing cells in the same manner as in Example 10, and then the process of purifying the antibody was conducted in the same manner as in Example 4.

Then, the quantitative affinity for GppNHp-bound KRas^{G12D} of the tissue-cell-specific anti-Ras·GTP iMab introduced with the light-chain variable region (VL) having a modified antibody framework to improve production yield was analyzed using a BIACORE2000 instrument.

Specifically, RT22-ep58 MG and RT22-ep59 MG were diluted to a concentration of 20 µl/ml in 10 mM NaAc buffer (pH 4.0) and immobilized at approximately 1800 response units (RU) on a CM5 sensor chip (GE Healthcare) . Subsequently, Tris buffer (20 mM Tris-HCl, pH 7.4, 137 mM NaCl, 5 mM MgCl₂, 0.01% Tween 20) was analyzed at a flow rate of 30 µl/min, and complete GppNHp-bound GST-KRas^{G12D} was analyzed at a concentration of 50 nM to 3.125 nM. After binding and dissociation analysis, regeneration of the CM5 chip was performed by flowing a buffer (20 mM NaOH, 1M NaCl, pH 10.0) at a flow rate of 30 µl/min for 1 minute. Each sensorgram, obtained by binding for 3 minutes and dissociation for 3 minutes, was normalized with reference to a blank cell and subtracted, and the affinity was thus calculated.

The following Table 15 shows the production yield of the anti-RasGTP iMab obtained by expressing an RT22 heavy chain in combination with light chains (hT4-ep58 MG and hT4-ep59 MG) fused with EpCAM-targeting protopeptide for providing tumor-tissue specificity and introduced with an antibody framework mutation to improve the production yield, and of the cytotransmabs obtained by expressing a TMab4 heavy chain in combination with the light chains, and the result of analysis of the affinity of tumor-tissue-specific anti-Ras·GTP iMab based on SPR using a BIACORE2000 instrument.

**[Table 15]**

| IgG1,κformat | Heavy chain | Light chain | | | Production yield (mg/1 L of transfected cells)^{a} | KRas^{G12D.} GppNHp *K*_{D}(M) |
|---|---|---|---|---|---|---|
| | VH | Targeting peptide | Linker | VL | | |
| RT22-ep58 MG | RT22 | Ep133 | MGSSSN | HT4-58 | 29.4 ± 6.03 | 3.73 ± 0.16×10⁻⁹ |
| RT22-ep59 MG | | | | hT4-59 | 69,8 ± 6.58 | 8.59 ± 0.32×10⁻⁹ |
| CT-ep58 MG | TMab4 | | | hT4-58 | 28 ± 432 | ND |
| CT-ep59 MG | | | | hT4-59 | 57.2± 7.09 | ND |

| | | | | | | |
|---|---|---|---|---|---|---|
| ^{a}The 2 plasmids that encode the HC and LC of each IgG antibody were co-transfected with the equivalent molar ratio into HEK293F cells in 1 L of culture media. After 6 d of culture, antibodies were purified from the cell culture supernatant using a protein-A affinity column. | | | | | | |

It was found that the affinity for GppNHp-bound KRas^{G12D} of the anti-Ras·GTP iMab was not changed compared to RT22-ep38 MG and RT22-ep59 MG, tumor-tissue-specific anti-Ras ·GTP iMabs introduced with mutants of light-chain variable region (VL) CDR1 and CDR2 to improve production yield.

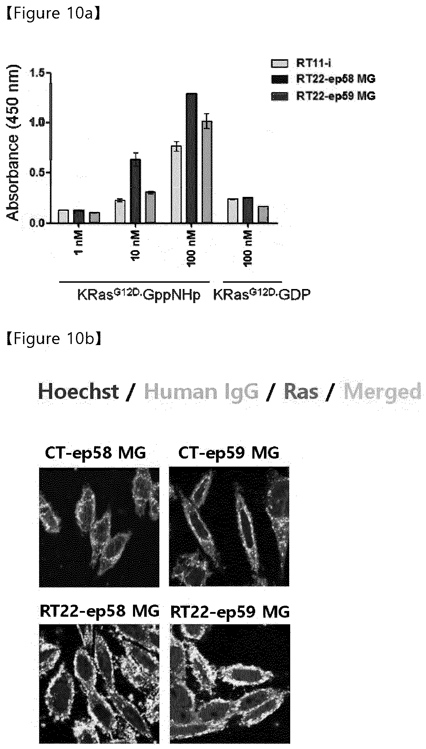

FIG. 10A shows the result of ELISA to analyze the binding ability to 1, 10 or 100 nM Avi-KRas^{G12D}·GppNHp or 100 nM Avi-KRas^{G12D}·GDP of anti-Ras·GTP iMabs including light-chain variable regions (hT4-ep58 MG to hT4-ep59 MG VL) fused with EpCAM-targeting protopeptide and a modified antibody framework to improve the production yield thereof.

Specifically, ELISA was conducted in the same manner as in Example 4, and tumor-tissue-specific anti-Ras·GTP iMabs having light chains introduced with mutants of CDR1 and CDR2 were fixed on a 96-well EIA/RIA plate, after which the GppNHp-bound KRas protein was bound at various concentrations of 100 nM, 10 nM and 1 nM, and the GDP-bound KRas protein was bound at a concentration of 100 nM and was then bound to an HRP-conjugated anti-his antibody (HRP-conjugated anti-his mAb) as a labeling antibody. The result was reacted with TMB ELISA solution, and the absorbance at 450 nm was quantified.

The result of analysis on the antigen-binding ability by ELISA showed that, compared to the conventional RT11-i, hT4-58 and hT4-59 light-chain variable regions improved binding capacity to the GppNHp-bound KRas^{G12D} antigen.

FIG. 10B shows the result of confocal microscopy analysis to determine the overlap with the activated intracellular Ras after treating the KRas mutant colorectal cancer cell line SW480 at 37°C for 12 hours with 1 µM of anti-Ras·GTP iMabs (RT22-ep58, RT22-ep59) including light chains (hT4-58, hT4-59) fused with EpCAM-targeting protopeptide and having a modified antibody skeleton to improve the production yield based on hT4-38 and hT4-39.

Specifically, an SW480 cell line was diluted in 0.5 ml at 2x10⁴ cells/well in a 24-well plate, cultured for 12 hours at 37°C under 5% CO₂, and then treated with 1 µM of CT-ep58 MG and anti-Ras·GTP iMabs, RT22-ep58 MG and RT22-ep59 MG with improved affinity and then cultured at 37°C for 12 hours. Then, the medium was removed, the residue was washed with PBS, and proteins attached to the cell surface were removed with a weakly acidic solution (200 mM glycine, 150 mM NaCl pH 2.5). After washing with PBS, 4% paraformaldehyde was added, and cells were immobilized at 25 degrees for 10 minutes. After washing with PBS, the cells were cultured in a buffer containing PBS supplemented with 0.1% saponin, 0.1% sodium azide and 1% BSA at 25°C for 10 minutes, and a hole was formed in the cell membrane. Then, the cells were washed with PBS again and reacted with a buffer containing 2% BSA in addition to PBS for 1 hour at 25°C in order to inhibit non-specific binding. Each antibody was stained with an antibody that specifically recognizes human Fc linked with Alexa-488 (green fluorescence) . The nuclei were stained (blue fluorescence) using Hoechst33342, and KRas-labeled antibodies were stained (red fluorescence) and then observed with a confocal microscope. All of the anti-Ras·GTP iMabs excluding CT-ep59 were found to have fluorescence overlapping that of the intracellular Ras.

### Example 18. Determination of HSPG-binding ability of anti-Ras·GTP iMab introduced with light-chain variable-region (VL) mutants having modified antibody framework to improve production yield

The decrease level in HSPG-binding ability of cytotransmabs and the anti-Ras·GTP iMabs introduced with light-chain variable-region (VL) mutants having a modified antibody framework to improve production yield constructed in Example 17 above, compared to cytotransmab (TMab4) using the conventional hT4 light-chain, was observed using a confocal microscope and cell-based ELISA.

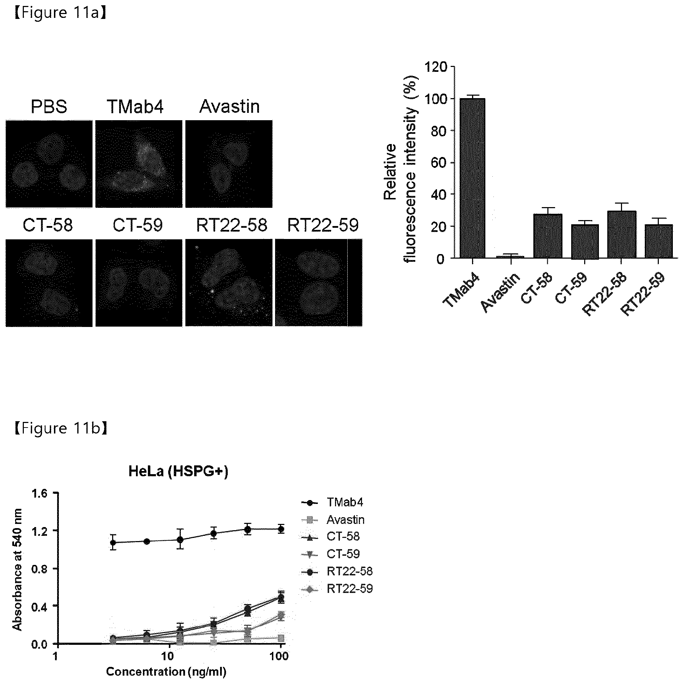

FIG. 11A shows the result of confocal microscopy measured after treatment of HeLa cells with 1 µM of the antibody at 37°C for 6 hours to determine a decrease in, or the removal of, HSPG-binding ability and cytoplasmic penetration of cytotransmabs and anti-Ras·GTP iMabs including light-chain variable regions introduced with mutants of CDR1 and CDR2 for improving production yield and inhibiting HSPG-binding ability, and is a bar graph showing quantified Alexa488 fluorescence (green fluorescence).

Specifically, the HeLa cell line expressing HSPG was seeded at 5 × 10⁴ cells/well in a 24-well plate in 0.5 ml of a medium containing 10% FBS and cultured at 5% CO₂ and 37°C for 12 hours. When the cells were stabilized, PBS, TMab4, Avastin, RT22-58, RT22-59, CT-58 and CT-59 were cultured at 1 µM at 37°C for 6 hours. After washing with PBS and a weakly acidic solution in the same manner as in Example 4, the cells were immobilized, perforated and then blocked. Each antibody was stained with an antibody that specifically recognizes human Fc conjugated with Alexa488 (green fluorescence). Then, the nuclei were stained (blue fluorescence) using Hoechst33342 and observed under a confocal microscope.

The result showed that anti-RasGTP iMabs located in the cytoplasm through HSPG exhibited fluorescence intensity in the order of TMab4 (100%), RT22-58 (30%), CT-58 (27%), CT-59 and RT22-59 (20%).

FIG. 11B shows the result of non-specific cell-surface-binding ELISA in the HeLa cell line expressing HSPG to determine the HSPG-binding ability of cytotransmabs and the anti-Ras·GTP iMabs including the light-chain variable regions introduced with mutants of CDR1 and CDR2 for improving production yield and inhibiting HSPG-binding ability.

Specifically, the HeLa (HSPG⁺) cell line was cultured in a 96-well plate such that the cells completely filled the bottom of the well and then washed three times with a washing buffer (HBSS buffer, 50 mM HEPES) . Then, PBS, TMab4, Avastin, RT22-58, RT22-59, CT-58 and CT-59 were diluted to concentrations of 100, 50, 25, 12.5, 6.25 and 3.125 ng/ml in a blocking buffer (HBSS buffer, 50 mM HEPES, 1% BSA) and the cells were cultured for 2 hours at 4°C. After washing three times with washing buffer, the cells were bound to an HRP-conjugated anti-human mAb as a labeling antibody. The cells were reacted with TMB ELISA solution, and the absorbance at 450 nm was quantified.

With the same behavior as in FIG. 11A, cell-surface binding capacity was measured in the order of TMab4, RT22-58, CT-58, CT-59 and RT22-59.

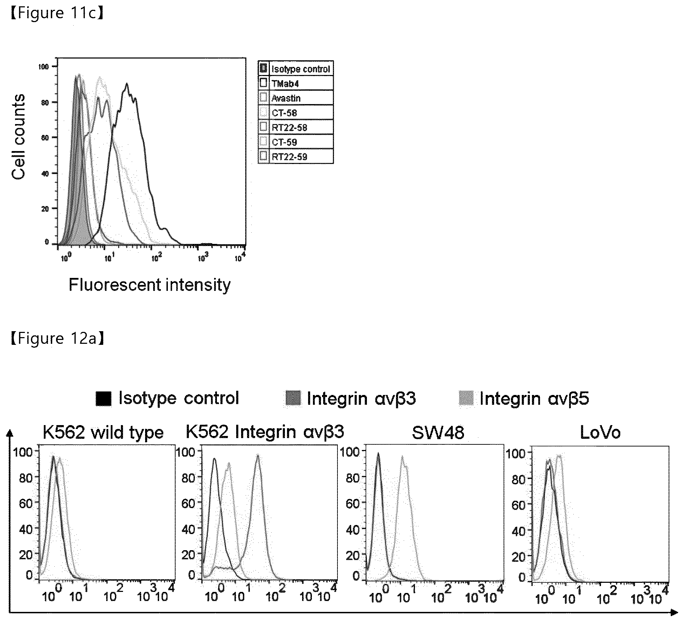

FIG. 11C shows the result of flow cytometry using FACS after treatment of the HeLa cell line with 500 nM of the antibody to determine the HSPG-binding ability of cytotransmabs and the anti-Ras·GTP iMabs including the light-chain variable regions introduced with mutants of CDR1 and CDR2 for improving production yield and inhibiting HSPG-binding ability.

Specifically, 1x10⁵ HeLa (HSPG⁺) cells were prepared for each sample. The cells were cultured in PBSF (PBS buffer, 2% BSA) supplemented with 500 nM of TMab4, Avastin, RT22-58, RT22-59, CT-58 and CT-59 for 1 hour at 4°C. Then, each antibody was reacted with an antibody specifically recognizing human Fc conjugated to Alexa488 (green fluorescence) for 30 minutes at 4°C. The result was washed with PBS and analyzed with flow cytometry. The result showed that fluorescence intensity binding to the cells was measured in the order of TMab4, TMab4, RT22-58, CT-58, CT-59 and RT22-59, in the same behavior as in the confocal microscopy results.

The result showed that the light-chain variable region (VL) mutants having a modified antibody framework to improve production yield also have no HSPG-binding ability.

### Example 19. Expression and purification of tumor-tissue-specific anti-Ras·GTP iMab introduced with Ras·GTP-specific heavy-chain variable region (VH) having improved affinity based on RT22, and light-chain variable region (VL) having cytoplasmic penetration specific for tumor tissue cells and improved production yield.

RT31, RT36 and RT37 heavy-chain animal expression vectors and light-chain expression vectors including a light-chain variable region fused with integrin or EpCAM- targeting protopeptides for providing tumor-tissue specificity and having inhibited HSPG-binding ability and improved production yield were subjected to transient co-transfection into HEK293F-protein-expressing cells in the same manner as in Example 4, and then the process of purifying the antibody was conducted in the same manner as in Example 4.

In addition, the quantitative affinity for GppNHp-bound KRas^{G12D} of the anti-Ras·GTP iMabs introduced with the RT31, RT36 and RT37 heavy-chain animal expression vectors and the light-chain variable regions fused with integrin or EpCAM-targeting protopeptide for providing tumor-tissue specificity and having inhibited HSPG-binding ability and improved production yield was analyzed using a BIACORE2000 instrument.

Specifically, RT31-i37 MG, RT31-ep37 MG, RT36-i37 MG, RT36-i38 MG, RT36-i39 MG, RT36-ep37 MG, RT36-ep38 MG and RT36-ep39 MG were diluted to a concentration of 20 µl/ml in 10 mM NaAc buffer (pH 4.0) and immobilized at approximately 1800 response units (RU) on a CM5 sensor chip (GE Healthcare). Subsequently, Tris buffer (20 mM Tris-HCl, pH 7.4, 137 mM NaCl, 5 mM MgCl₂, 0.01% Tween 20) was analyzed at a flow rate of 30 µl/min, and complete GppNHp-bound GST-KRas^{G12D} was analyzed at a concentration of 50 nM to 3.125 nM. After binding and dissociation analysis, regeneration of the CM5 chip was performed by flowing a buffer (20 mM NaOH, 1M NaCl, pH 10.0) at a flow rate of 30 µl/min for 1 minute. Each sensorgram, obtained by binding for 3 minutes and dissociation for 3 minutes, was normalized with reference to a blank cell and subtracted, and the affinity was thus calculated.

The following Table 16 shows the production yield of the tumor-tissue-specific anti-RasGTP iMabs introduced with the RT31, RT36 and RT37 heavy-chain animal expression vectors and the light-chain variable regions fused with integrin or EpCAM-targeting protopeptide for providing tumor-tissue specificity, and having inhibited HSPG-binding ability and improved production yield, and the result of analysis of the affinity of tumor-tissue-specific anti-Ras·GTP iMabs based on SPR using a BIACORE2000 instrument.

**[Table 16]**

| IgG1,κ format | Heavy chain | Light chain | Production yield (mg/l L of transfected cells)^{a} | KRas^{G12D}. GppNHp K_{D}(M)^{b} |
|---|---|---|---|---|
| RT31-I37 MG | RT31 | hT4-I37 MG | 1.4 ± 0.20 | 3.1 ± 0.12×10⁻⁹ |
| RT31-ep37 MG | | hT4-ep37 MG | 4.7 ± 2.12 | ND |
| RT36-i37 MG | RT36 | hT4-i37 MG | 3.3 ± 1.72 | 2.9 ± 0.05×10⁻⁹ |
| RT36-i38 MG | | hT4-i38 MG | 48.1 ± 2.12 | 5.3 ± 0.30×10⁻⁹ |
| RT36-i39 MG | | hT4-i39 MG | 19.1 ± 1.35 | 2.2 ± 0.11×10⁻⁸ |
| RT36-ep37 MG | | hT4-ep37 MG | 3.1 ± 1.32 | ND |
| RT36-ep58 MG | | hT4-ep58 MG | 24.4 ± 1.99 | 1.2 ± 0.11×10⁻⁸ |
| RT36-ep59 MG | | hT4-ep59 MG | 35.8 ± 19.86 | 1.13 ± 0-07×10⁻⁸ |
| RT37-i37 MG | RT37 | hT4-i37 MG | < 1 | ND |

| | | | | |
|---|---|---|---|---|
| ^{a}The 2 plasmids that encode the HC and LC of each IgG antibody were co-transfected with the equivalent molar ratio into HEK293F cells in 1 L of culture media. After 6 d of culture, antibodies were purified from the cell culture supernatant using a protein-A affinity column. ^{b} ND, not determined | | | | |

The antibodies (RT31-i37 MG, RT31-ep37 MG, RT37-i37 MG) introduced with RT31 and RT37 heavy chains with improved affinity based on RT22 and light-chain variable region (VL) mutants for improving production yield and inhibiting HSPG-binding capacity have a problem of low production yield, and the antibodies (RT36-i38 MG, RT36-i39 MG, RT36-ep58 MG and RT36-ep59 MG) introduced with RT36 and light-chain variable-region (VL) mutants having improved production yield and tumor-tissue-cell-specific cytoplasmic penetration have a problem of low affinity for Ras ·GTP, compared to RT22-i38 MG, RT22-i39 MG, RT22-ep58 MG and RT22-ep59 MG. Therefore, experiments were conducted on RT22-i38 MG, RT22-i39 MG, RT22-ep58 MG and RT22-ep59 MG, having high production yield and excellent affinity for Ras·GTP.

### Example 20. Determination of binding ability to integrin or EpCAM on cell surface of anti-Ras·GTP iMab including light-chain variable region (VL) fused with integrin or EpCAM-targeting protopeptide and having cytoplasmic penetration specific for tumor tissue cells.

FIG. 12A shows the result of a test using FACS to identify the expression of integrin αvβ3 or integrin αvβ5 in human colorectal cancer cell lines SW480 and LoVo, and human blood cancer cell lines wild-type K562 and integrin αvβ3-expressing K562.

Specifically, 1 × 10⁵ of each of SW480, LoVo, K562 and K562 αvβ3 cell lines were prepared for each sample. The cells were cultured in a dilution of PE-conjugated anti-integrin αvβ3 or PE-conjugated anti-integrin αvβ5 at 1:100 at 4°C for 1 hour. The cells were washed with PBS and analyzed by flow cytometry. The result showed that the αvβ3 antibody bound to the K562 αvβ3-expressing cell line and the αvβ5 antibody bound to SW480 and LoVo cells.

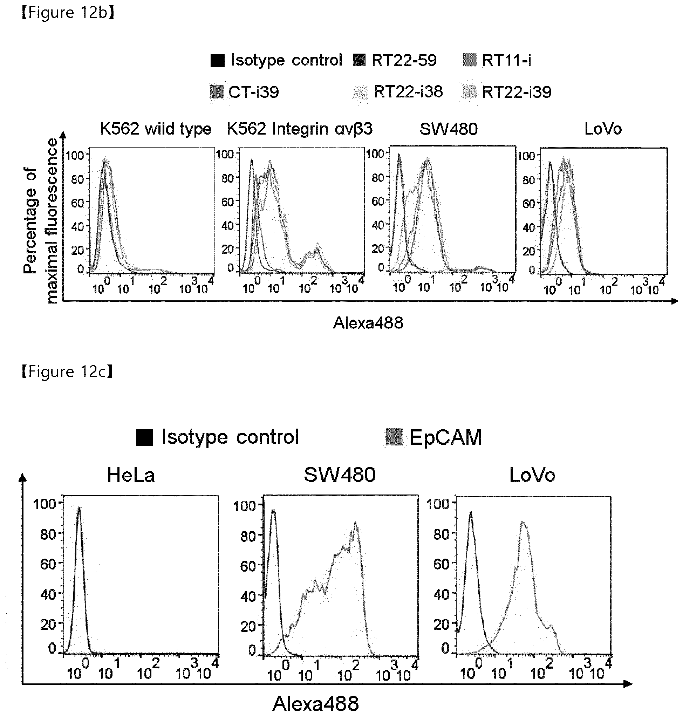

FIG. 12B shows the result of a test using FACS to determine the binding ability to cell-surface integrin αvβ3 or integrin αvβ5 of anti-Ras cell-penetrating antibodies specific for tumor tissue integrin (RT22-i38 MG and RT22-i39 MG), having no HSPG-binding ability including a heavy chain (RT22) having improved affinity for Ras·GTP and of conventional anti-Ras cell-penetrating antibodies specific for tumor tissue integrin (RT11-i).

Specifically, 1×10⁵ of each of SW480, LoVo, K562 and K562 αvβ3 cells were prepared for each sample. The cells were cultured for 1 hour at 4°C in PBSF (PBS buffer, 2% BSA) supplemented with 500 nM of RT11-i, RT22-i38 MG, RT22-i39 MG and CT-i39 MG. Then, each antibody was reacted with an antibody specifically recognizing human Fc conjugated to Alexa488 (green fluorescence) for 30 minutes at 4 degrees. The cells were washed with PBS and analyzed by flow cytometry. The result showed that the fluorescence intensities of RT11-i, RT22-i38 MG, RT22-i39 MG and CT-i39 MG antibodies bound to SW480, LoVo and K562 αvβ3 cells expressing αvβ3 or αvβ5 were measured.

FIG. 12C shows the result of a test using FACS to determine the expression of EpCAM in the human colorectal cancer cell lines SW480 and LoVo, and the human cervical cancer cell line HeLa.

Specifically, 1×10⁵ of each of SW480, LoVo, K562 and K562 αvβ3 cells were prepared for each sample. The anti-EpCAM antibody was diluted at 1:200 and cultured for 1 hour at 4°C. Then, the primary antibody was reacted with an antibody specifically recognizing murine Fc conjugated to which Alexa488 (green fluorescence) for 30 minutes at 4°C. The cells were washed with PBS and analyzed by flow cytometry. As a result, the fluorescence intensities of the αvβ3 antibody bound to K562 αvβ3 cells and the αvβ5 antibody bound to SW480 and LoVo cells were measured.

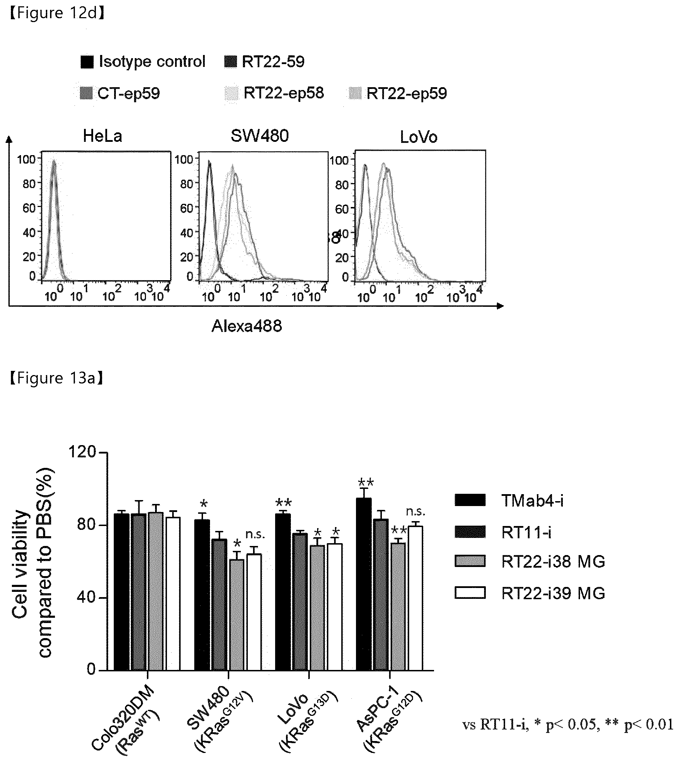

FIG. 12D shows the result of a test using FACS to determine the binding ability to the cell surface EpCAM of tumor-tissue EpCAM-specific anti-Ras cell-penetrating antibodies (RT22-ep58 MG and RT22-ep59 MG) having no HSPG-binding ability and including a heavy chain (RT22) having improved affinity for Ras·GTP.

Specifically, 1×10⁵ of each SW480, LoVo and HeLa cells were prepared for each sample. The cells were cultured for 1 hour at 4°C in PBSF (PBS buffer, 2% BSA) supplemented with 500 nM of RT22-ep58 MG, RT22-ep59 MG, and CT-ep59 MG. Then, each antibody was reacted with an antibody specifically recognizing human Fc conjugated to Alexa488 (green fluorescence) for 30 minutes at 4°C. Then, the cells were washed with PBS and analyzed by flow cytometry. As a result, the fluorescence intensities of the RT22-ep58 MG, RT22-ep59 MG and CT-ep59 MG antibodies bound to SW480 and LoVo cells expressing EpCAM were measured.

### Example 21. Determination of growth inhibition of adherent cells of tumor-tissue-specific anti-Ras·GTP iMab including combination of light-chain variable region (VL) having improved production yield and tumor-tissue-cell-specific cytoplasmic penetration ability with heavy-chain variable region having improved affinity for Ras·GTP

FIG. 13A is a graph showing cell growth inhibition ability, determined through WST assay, after treatment at 37°C for 48 hours with 0.5 µM of anti-Ras·GTP iMab combined with a light-chain variable region fused with integrin-targeting protopeptide and having improved production yield and tumor-tissue-cell-specific cytoplasmic penetration in several Ras mutant and wild-type cell lines.

Specifically, the cell line was diluted at a density of 1x10⁴ cells/well in 0.2 ml of a medium containing 10% FBS in a 96-well plate and cultured for 24 hours at 37°C and at 5% CO₂. Then, the resulting cells were treated with 0.5 µM of TMab4-i, RT11-i, RT22-i38 MG and RT22-i39 MG for 48 hours, after which 10 µl of a WST solution (Dojindo) was added thereto and the absorbance at 450 nm was quantified.

As can be shown from FIG. 13A, RT22-i38 MG and RT22-i39 MG in various Ras mutant cell lines exhibited significantly higher cell-growth inhibitory ability than the unmodified anti-Ras·GTP iMab RT11-i used as a control group. In addition, there was no difference in the wild-type cell line Colo320DM between all anti-Ras·GTP iMabs and the cytoplasmic penetration antibody (cytotransmab, TMab4-i) having no Ras inhibitory ability used as a control group.

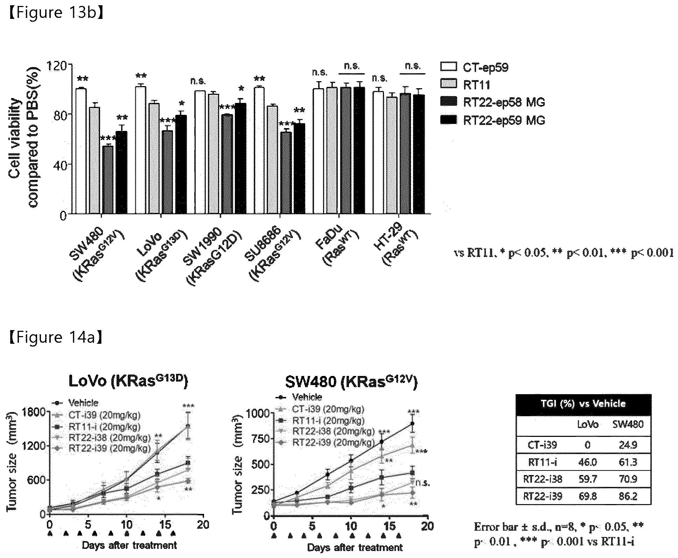

FIG. 13B is a graph showing cell growth inhibition ability, determined by WST assay, after treatment at 37°C for 48 hours with 1 µM of anti-Ras·GTP iMab combined with a light-chain variable region fused with EpCAM-targeting protopeptide and having improved production yield and inhibited HSPG-binding ability in several Ras mutant and wild-type cell lines.

Specifically, the cell lines were diluted at a density of 1x10⁴ cells/well in 0.2 ml of a medium containing 10% FBS in a 96-well plate and cultured for 24 hours at 37°C under 5% CO₂. Then, the resulting cells were treated with 1 µM of CT-ep59, RT11, RT22-ep58 MG and RT22-ep59 MG for 36 hours, after which 10 µl of a WST solution (Dojindo) was added thereto and absorbance at 450 nm was quantified.

As can be shown from FIG. 13B, RT22-ep58 MG and RT22-ep59 MG in various Ras mutant cell lines expressing EpCAM exhibited significantly higher cell-growth inhibitory ability than the unmodified anti-Ras·GTP iMab RT11 used as a control group. In addition, there was no difference in wild-type cell lines FaDu and HT-29 between all anti-Ras ·GTP iMabs and the cytoplasmic penetration antibody having no Ras inhibitory ability (cytotransmab, CT-ep59) used as a control group.

As a result, when compared with the conventional anti-Ras·GTPs (RT11, RT11-i) *in vitro,* RT22-i38 MG, RT22-i39 MG, RT22-ep58 MG and RT22-ep59 MG have an improved cell growth inhibitory effect specific for Ras mutant cell lines. Thus, the subsequent experiment was conducted to determine whether or not the effect of inhibiting the tumor growth by the antibodies was also improved in an *in-vivo* animal model.

### Example 22. Determination of improved tumor growth inhibitory ability of tumor-tissue integrin-specific anti-Ras·GTP iMab

FIG. 14A shows the result of a test to compare the tumor growth inhibition effect when intravenously injecting 20 mg/kg of anti-Ras cell-penetrating antibodies (RT22-i38 MG, RT22-i39 MG), fused with integrin-targeting protopeptide and having no HSPG-binding ability, and the conventional anti-Ras cell-penetrating antibody (RT11-i), fused with integrin-targeting protopeptide, into human colorectal cancer KRas mutant cell lines LoVo and SW480 xenograft mice a total of 9 times at 2-day intervals.

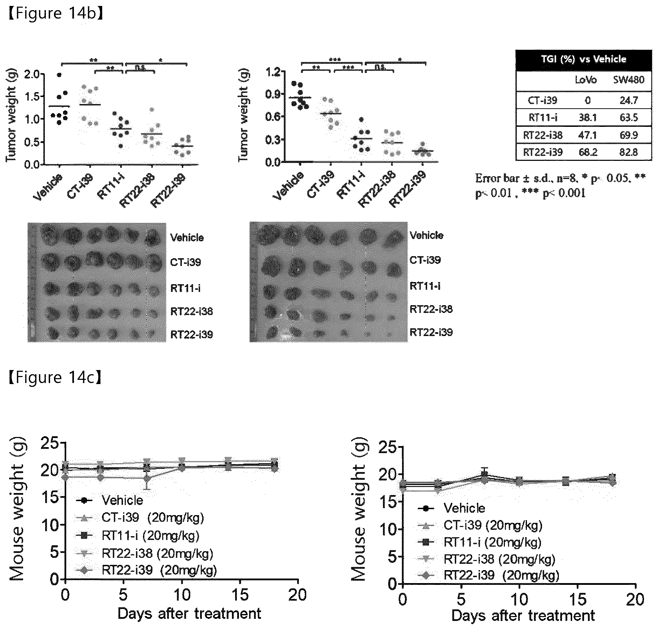

FIG. 14B is a graph showing the weight of an extracted tumor and an image showing the tumor after treatment with the anti-Ras cell-penetrating antibody fused with the integrin-targeting protopeptide and having no HSPG-binding ability.

FIG. 14C is a graph showing the weight of the mice measured to determine the non-specific side effects of the anti-Ras cell-penetrating antibody fused with the integrin-targeting protopeptide and having no HSPG-binding ability.

Specifically, in order to determine the improved tumor growth inhibition ability of RT22-i38 MG and RT22-i39 MG, tumor-tissue integrin-specific anti-Ras·GTP iMabs, having improved affinity and having no HSPG-binding ability, compared to the conventional tumor-tissue integrin-specific anti-Ras·GTP iMab, RT11-I *in vivo,* the KRas^{G12V} mutant human colorectal cancer cell line SW480 was subcutaneously injected into BALB/c nude mice at a density of 5×10⁶ cells/mouse, and the KRas^{G13D} mutant human colorectal cancer cell line LoVo was subcutaneously injected into BALB/c nude mice at a density of 2×10⁶ cells/mouse. After about 10 days, when the tumor volume reached about 50 to 80 mm³, the equivalent volume of a PBS vehicle control group, control group CT-i39 MG (TMab4 VH), and experimental groups RT11-i, RT22-i38 MG and RT22-i39 MG were injected intravenously at 20 mg/kg. A total of 8 intravenous injections were performed every 2 days, and the tumor volume was measured for 16 days using a caliper.

As can be shown from FIG. 14A, RT11-i, RT22-i38 MG and RT22-i39 MG inhibited cancer cell growth, compared to the control group, CT-i39 MG, and RT22-i38 MG and RT22-i39 MG inhibited tumor growth more effectively than RT11-i. Quantitative tumor growth inhibitions were determined as CT-i39 (0%), RT11-i (46%), RT22-i38 (59.7%) and RT22-i39 (69.8%) with respect to the vehicle control group.

FIG. 14B shows the result of the tumor growth inhibition rates obtained based on the body weight of the extracted tumor and the tumor growth inhibition rates were determined as CT-i39 (0%), RT11-i (38.1%), RT22-i38 (47.1%) and RT22-i39 (68.2%).

In addition, as can be seen from FIG. 14D, RT22-i38 MG and RT22-i39 MG experimental group mice had no change in body weight, which means that there was no other toxicity.

### Example 23. Determination of tumor growth inhibitory ability of tumor tissue EpCAM-specific anti-Ras·GTP iMab

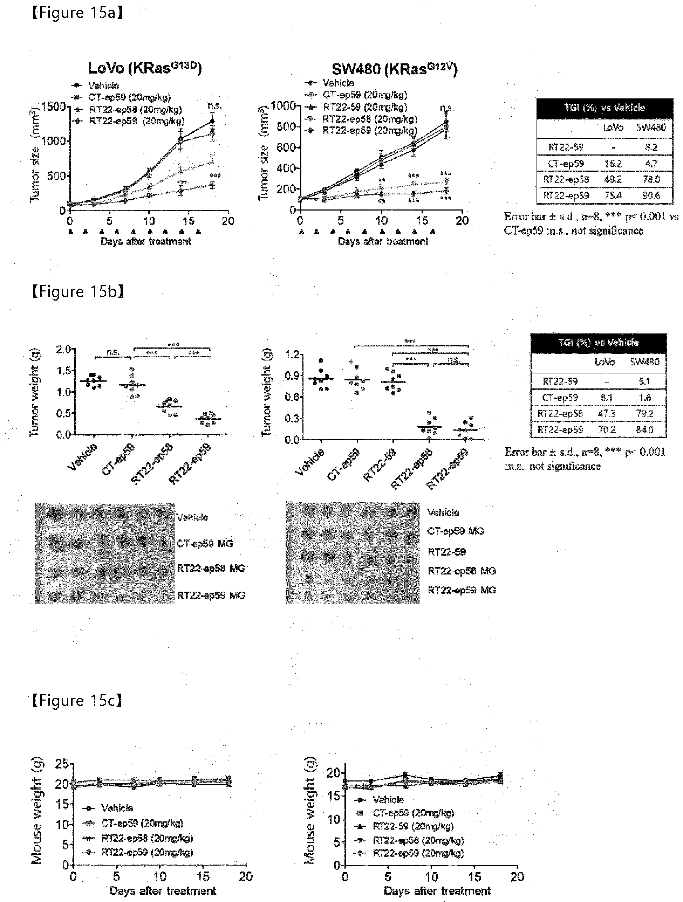

FIG. 15A shows the result of a test to compare the tumor growth inhibition effect when intravenously injecting 20 mg/kg of anti-Ras cell-penetrating antibodies (RT22-ep58 MG, RT22-ep59 MG), fused with EpCAM -targeting protopeptide and having no HSPG-binding ability, into human colorectal cancer cell lines LoVo and SW480 xenograft mice a total of 9 times at 2-day intervals.

FIG. 15B is a graph showing the weight of the extracted tumor and an image showing the tumor, after treatment with the anti-Ras cell-penetrating antibody, fused with the EpCAM-targeting protopeptide and having no HSPG-binding ability.

FIG. 15C is a graph showing the weight of the mice measured to determine the non-specific side effects of the anti-Ras cell-penetrating antibody fused with the EpCAM-targeting protopeptide and having no HSPG-binding ability.

Specifically, in order to determine the *in-vivo* tumor growth inhibition of RT22-ep58 MG and RT22-ep59 MG, tumor-tissue EpCAM-specific anti-Ras·GTP iMabs, the KRas^{G12V} mutant human colorectal cancer cell line SW480 was subcutaneously injected into BALB/c nude mice at a density of 5×10⁶ cells/mouse, and the KRas^{G13D} mutant human colorectal cancer cell line LoVo was subcutaneously injected into BALB/c nude mice at a density of 2×10⁶ cells/mouse. After about 10 days, when the tumor volume reached about 50 to 80 mm³, the equivalent volume of a PBS vehicle control group, a control group, CT-ep59 MG, and experimental groups RT22-ep58 MG and RT22-ep59 MG were injected intravenously at 20 mg/kg. A total of 9 intravenous injections were performed every 2 days, and the tumor volume was measured for 18 days using a caliper.

As can be shown from FIG. 15A, compared to the control group, CT-ep59 MG, RT22-ep58 MG and RT22-ep59 MG exhibited inhibited cancer cell growth. Quantitative tumor growth inhibitions were determined as CT-ep59 (16.2%), RT22-ep58 (49.2%) and RT22-ep59 (75.4%) with respect to the vehicle control group.

FIG. 15B shows the result of the tumor growth inhibition rates obtained based on the body weight of the extracted tumor and specifically, the tumor growth inhibition rates were determined as CT-ep59 (8.1%), RT22-ep58 (47.3%) and RT22-ep59 (70.2%).

In addition, as can be seen from FIG. 15C, RT22-ep58 MG and RT22-ep59 MG experimental group mice had no change in body weight, which means that there was no other toxicity.

### Example 24. Determination of tumor growth inhibition ability of tumor-tissue integrin-specific anti-Ras·GTP iMab depending on dose, administration interval and administration route

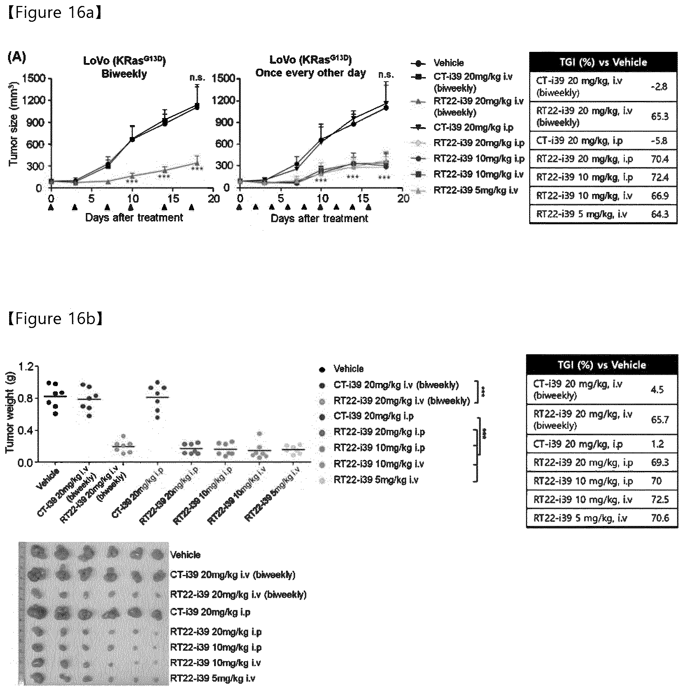

FIG. 16A shows the result of a test to compare tumor growth inhibition ability depending on dose, administration interval and administration route of the anti-Ras cell-penetrating antibody (RT22-i39 MG) fused with integrin-targeting protopeptide and having no HSPG-binding ability in human colorectal cancer KRas mutant cell line LoVo xenograft mice.

FIG. 16B is a graph showing the weight of the extracted tumor and an image showing the tumor, after treatment with the anti-Ras cell-penetrating antibody fused with the integrin-targeting protopeptide and having no HSPG-binding ability.

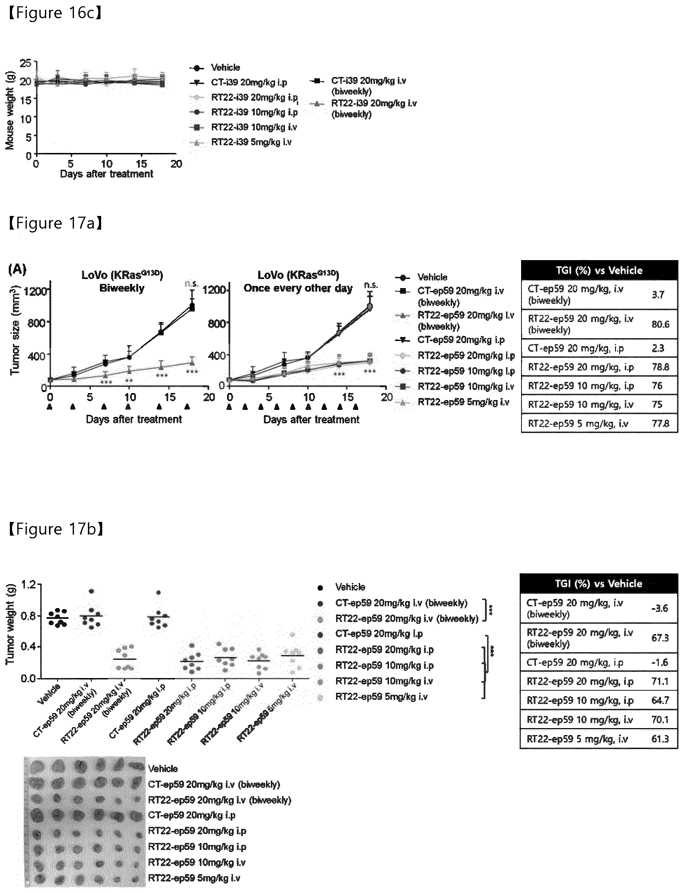

FIG. 16C is a graph showing the weight of the mice measured to determine the non-specific side effects of the anti-Ras cell-penetrating antibody fused with the integrin-targeting protopeptide and having no HSPG-binding ability.

Specifically, FIG. 16C shows the result of an *in-vivo* test to compare the tumor growth inhibition effect, depending on dose, administration interval and administration route, of the tumor-tissue integrin-specific anti-Ras·GTP iMab (RT22-i39 MG) having improved affinity and having no HSPG-binding ability.

In order to determine the tumor growth inhibition effect, the KRas^{G13D} mutant human colorectal cancer cell line LoVo was subcutaneously injected into BALB/c nude mice at a density of 2×10⁶ cells/mouse. After about 10 days, when the tumor volume reached about 50 to 80 mm³, the equivalent volume of a PBS vehicle control group, control group CT-i39 MG (TMab4 VH), and experimental group RT22-i39 MG were injected intravenously or intraperitoneally at 5, 10 and 20 mg/kg. A total of 9 intravenous injections were performed every 2 days, or a total of 6 intravenous injections were performed every week, and the tumor volume was measured for 18 days using a caliper.

As can be shown from FIG. 16A, compared to the control group CT-i39 MG, RT22-i39 MG inhibited cancer cell growth. There was no great difference in the tumor growth inhibition effect depending on the dose, administration interval or administration route. Quantitative tumor growth inhibitions were determined as CT-i39 20 mg/kg i.v. biweekly (-2.8%), RT22-i39 20 mg/kg i.v. biweekly (65.3%), CT-i39 20 mg/kg i.p. (-5.8%), RT22-i39 20 mg/kg i.p. (70.4%), RT22-i39 10 mg/kg i.p. (72.4%), RT22-i39 10 mg/kg i.v. (66.9%), and RT22-i39 5 mg/kg i.v. (64.3%), compared to the vehicle control group.

FIG. 16B shows the result of the tumor growth inhibition rates obtained based on the body weight of the extracted tumor, specifically the tumor growth inhibition rates were determined as CT-i39 20 mg/kg i.v. biweekly (4.5%), RT22-i39 20 mg/kg i.v. biweekly (65.7%), CT-i39 20 mg/kg i.p. (1.2%), RT22-i39 20 mg/kg i.p. (69.3%), RT22-i39 10 mg/kg i.p. (70%), RT22-i39 10 mg/kg i.v. (72.5%), and RT22-i39 5 mg/kg i.v. (70.6%).

In addition, as can be seen from FIG. 16C, RT22-i39 MG experimental group mice had no change in body weight, which means that there was no other toxicity.

### Example 25. Determination of tumor growth inhibition ability of tumor tissue EpCAM-specific anti-Ras ·GTP iMab depending on dose, administration interval and administration route

FIG. 17A shows the result of a test to compare the tumor growth inhibition ability depending on dose, administration interval and administration route of the anti-Ras cell-penetrating antibody (RT22-ep59 MG) fused with integrin-targeting protopeptide and having no HSPG-binding ability in human colorectal cancer KRas mutant cell line LoVo xenograft mice.

FIG. 17B is a graph showing the weight of the extracted tumor and an image showing the tumor, after treatment with the anti-Ras cell-penetrating antibody fused with the EpCAM-targeting protopeptide and having no HSPG-binding ability.

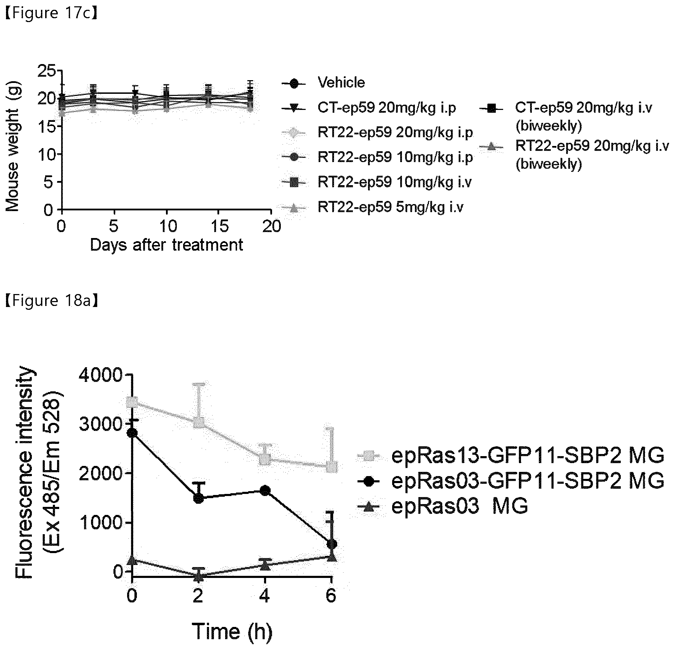

FIG. 17C is a graph showing the weight of the mice measured to determine the non-specific side effects of the anti-Ras cell-penetrating antibody fused with the integrin-targeting protopeptide and having no HSPG-binding ability.

Specifically, in order to determine the *in-vivo* tumor growth inhibition of RT22-ep59 MG, tumor-tissue EpCAM-specific anti-Ras·GTP iMab, depending on dose, administration interval and administration route, the KRas^{G13D} mutant human colorectal cancer cell line LoVo was subcutaneously injected into BALB/c nude mice at a density of 2×10⁶ cells/mouse. After about 10 days, when the tumor volume reached about 50 to 80 mm³, the equivalent volume of a PBS vehicle control group, control group CT-ep59 MG, and experimental group RT22-ep59 MG were injected intravenously or intraperitoneally at 5, 10, 20 mg/kg. A total of 9 intravenous injections were performed every 2 days, or a total of 6 intravenous injections were performed every week, and the tumor volume was measured for 18 days using a caliper.

As can be shown from FIG. 17A, RT22-ep59 MG inhibited cancer cell growth, compared to the control group CT-ep59 MG. There was no great difference in tumor growth inhibition effect depending on the dose, administration interval or administration route. Quantitative tumor growth inhibitions were determined as CT-ep59 20 mg/kg i.v. biweekly (3.7%), RT22-ep59 20 mg/kg i.v. biweekly (80.6%), CT-ep59 20 mg/kg i.p. (2.3%), RT22-ep59 20 mg/kg i.p. (78.8%), RT22-ep59 10 mg/kg i.p. (76%), RT22-ep59 10 mg/kg i.v. (75%), and RT22-ep59 5 mg/kg i.v. (77.8%) with respect to the vehicle control group.

FIG. 17B shows the result of the tumor growth inhibition rates obtained based on the weight of the extracted tumor and specifically, the tumor growth inhibition rates were determined as CT-ep59 20 mg/kg i.v. biweekly (-3.6%), RT22-ep59 20 mg/kg i.v. biweekly (67.3%), CT-ep59 20 mg/kg i.p. (-1.6%), RT22-ep59 20 mg/kg i.p. (71.1%), RT22-ep59 10mg/kg i.p. (64.7%), RT22-ep59 10mg/kg i.v. (70.1%), and RT22-ep59 5mg/kg i.v. (61.3%) with respect to the vehicle control group.

In addition, as can be seen from FIG. 17C, RT22-ep59 MG experimental group mice had no change in body weight, which means that there was no other toxicity.

### Example 26. Design and expression/purification of heavy-chain modified variant to improve intracytoplasmic stability, in-vivo persistence and tumor-tissue-targeting ability of anti-Ras·GTP iMab

Intracytoplasmic proteins are generally degraded by a ubiquitin-proteasome mechanism. Likewise, an intact immunoglobulin-type antibody in the cytoplasm is bound to TRIM21 E3 ligase and is degraded by the ubiquitin-proteasome mechanism, and the degradation of the antibody is inhibited when a mutation is introduced into the N434 amino acid in the region CH3 of the antibody that binds to TRIM21. Therefore, in order to improve the intracytoplasmic stability of the anti-Ras·GTP iMab, the intact immunoglobulin-type antibody, a variant in which a N434D mutation was introduced into the CH3 region was constructed. Specifically, in the same manner as in Example 4, the cytoplasmic penetration humanized light-chain expression vector (hT4-ep59 MG LC) and the heavy-chain expression vector (RT22 IgG1 N434D) of the anti-Ras·GTP iMab having improved intracytoplasmic stability were subjected to transient co-transfection into HEK293F-protein-expressing cells to express anti-Ras·GTP iMab mutations with improved intracytoplasmic stability.

In addition, the immunoglobulin-type antibody binds to the Fc receptor of immune cells in the body and is removed through an ADCC mechanism. Among the IgG subclass, IgG2 and IgG4 have weak binding force to the Fc gamma receptor and thus inhibition in this function. Therefore, in order to improve the *in-vivo* stability of the anti-Ras·GTP iMab, the intact immunoglobulin IgG1 antibody, a variant introduced with heavy-chain constant regions of IgG4 (CH1 to CH3) was constructed. At this time, the S228P mutation was further introduced into the Hinge site so that Fab-arm exchange, which is the characteristic of IgG4, does not occur in the body. Specifically, in the same manner as in Example 4, cytoplasm-penetrating humanized light-chain expression vector (hT4-ep59 MG LC) and the heavy-chain expression vector (RT22 IgG4 S228P) of the anti-Ras·GTP iMab having improved *in-vivo* persistence were subjected to transient co-transfection into HEK293F protein-expressing cells to express an anti-Ras·GTP iMab mutation with improved *in-vivo* persistence.

The following Table 17 shows the sequence information of the heavy-chain constant region (CH1-CH3) modified to improve the intracytoplasmic stability and *in-vivo* persistence of anti-Ras·GTP iMab.

**[Table 17]**

| Heavy Chain Constant Region | Sequence | SEQ ID NO. |
|---|---|---|
| IgG1 | 1 10 20 30 40 50 60  | 64 |
| IgG1 N434D | 1          10          20          30          40          50          60 ASTKGPSVFPLAPSSKSTSGGTAALGCLVKDYFPEPVTVSWNSGALTSGVHTFPAVLQSS 70          80          90          100          110          120 GLYSLSSVVTVPSSSLGTQTYICNVNHKPSNTKVDKKVEPKSCDKTHTCPPCPAPELLGG 130          140          150          160          170          180 PSVFLFPPKPKDTLMISRTPEVTCVVVDVSHEDPEVKFNWYVDGVEVHNAKTKPREEQYN 190          200          210          220          230          240 STYRVVSVLTVLHQDWLNGKEYKCKVSNKALPAPIEKTISKAKGQPREPQVYTLPPSRDE 250          260          270          280          290          300 LTKNQVSLTCLVKGFYPSDIAVEWESNGQPENNYKTTPPVLDSDGSFFLYSKLTVDKSRW 310          320          330 QQGNVFSCSVMHEALHDHYTQKSLSLSPGK | 65 |
| IgG4 S228P | 1 10 20 30 40 50 60 ASTKGPSVFPLAPCSRSTSESTAALGCLVKDYFPEPVTVSWNSGALTSGVHTFPAVLQSS 70 80 90 100 110 120 GLYSLSSVVTVPSSSLGTKTYTCNVDHKPSNTKVDKRVESKYGPPCPPCPAPEFLGGPSV 130 140 150 160 170 180 FLFPPKPKDTLMISRTPEVTCVVVDVSQEDPEVQFNWYVDGVEVHNAKTKPREEQFNSTY 190 200 210 220 230 240 RVVSVLTVLHQDWLNGKEYKCKVSNKGLPSSIEKTISKAKGQPREPQVYTLPPSQEEMTK 250 260 270 280 290 300 NQVSLTCLVKGFYPSDIAVEWESNGQPENNYKTTPPVLDSDGSFFLYSRLTVDKSRWQEG 310 320 327 NVFSCSVMHEALHNHYTQKSLSLSLGK | 66 |
| IgG4 S228P, N434D | 1 10 20 30 40 50 60 ASTKGPSVFPLAPCSRSTSESTAALGCLVKDYFPEPVTVSWNSGALTSGVHTFPAVLQSS 70 80 90 100 110 120 GLYSLSSVVTVPSSSLGTKTYTCNVDHKPSNTKVDKRVESKYGPPCPPCPAPEFLGGPSV 130 140 150 160 170 180 FLFPPKPKDTLMISRTPEVTCVVVDVSQEDPEVQFNWYVDGVEVHNAKTKPREEQFNSTY 190 200 210 220 230 240 RVVSVLTVLHQDWLNGKEYKCKVSNKGLPSSIEKTISKAKGQPREPQVYTLPPSQEEMTK 250 260 270 280 290 300 NQVSLTCLVKGFYPSDIAVEWESNGQPENNYKTTPPVLDSDGSFFLYSRLTVDKSRWQEG 310 320 327 NVFSCSVMHEALHDHYTQKSLSLSLGK | 67 |

In addition, in order to improve the tumor-tissue-targeting ability of anti-Ras·GTP iMab, a clone that binds to the ep133 peptide having EpCAM targeting ability to the N-terminus of the anti-Ras·GTP iMab heavy-chain variable region was constructed, expressed and purified. Specifically, in the same manner as in Example 4, the cytoplasm-penetrating humanized light-chain expression vector (hT4-ep59 MG LC) and the heavy-chain expression vectors (epRT22 GS, epRT22 MG, epRT22 (G4S)2) of the anti-Ras ·GTP iMab having improved tumor-tissue-targeting ability were subjected to transient co-transfection into HEK293F protein-expressing cells to express anti-Ras·GTP iMab mutations having improved tumor-tissue-targeting ability.

The following Table 18 shows the heavy-chain variable-region sequences of anti-Ras·GTP iMab having improved tumor-tissue-targeting ability.

**[Table 18]**

| VH name | Sequence | SEQ ID NO. |
|---|---|---|
| epRT22 GS | 10          20          30          40          50          60 EHLHCLGSLCWPGSEVQLVESGGGLVQPGGSLRLSCAASGFTFSDFSMSWVRQAPGKGLE 70          80          90          100         110         120 WVSYISRTSHTTYYADSVKGRFTISRDNSKNTLYLQMNSLRAEDTAVYYCARGFKMDYWGQ 128 GTLVTVSS | 61 |
| epRT22 MG |         10          20          30          40          50          60 EHLHCLGSLCWPMGSSSNEVQLVESGGGLVQPGGSLRLSCAASGFTFSDFSMSWVRQAPG 70          80          90          100         110         120 KGLEWVSVISRTSHTTYYADSVKGRFTISRDNSKNTLYLQMNSLRAEDTAVYYCARGFKMD 130 YWGQGTLVTVSS | 62 |
| epRT22 (G45)₂ | 10 20 30 40 50 60 EHLHCLGSLCWPGGGGSGGGGSEVQLVESGGGLVQPGGSLRLSCAASGFTFSDFSMSWVR 70 80 90 100 110 120 QAPGKGLEWVSYISRTSHTTYYADSVKGRFTISRDNSKNTLYLQMNSLRAEDTAVYYCARG 130 FKMDYWGQGTLVTVSS | 63 |

The following Table 19 shows the production yield of anti-Ras·GTP iMab having improved intracytoplasmic stability, *in-vivo* persistence and tumor-tissue-targeting ability

**[Table 19]**

| IgG1,κ format | Heavy chain | | Light chain | Production yield (mg /l L of transfected cells)^{a} |
|---|---|---|---|---|
| | VH | CH1-CH3 | | |
| epRas03 MG (=RT22-ep59 MG) | RT22 | IgG1 | hT4-ep59 MG | 69.8 ± 6.6 |
| epRas13 MG | | IgG1 N434D | | 67.1 ± 2.9 |
| epRas23 MG | epRT22 GS | IgG1 | | 30.2 ± 4.8 |
| epRas33 MG | | IgG1 N434D | | ND |
| epRas03 MG IgG4 | RT22 | IgG4 S228P | | 48.1 ± 3.7 |
| epRas13 MG IgG4 | | IgG4 S228P, N434D | | 48.2 ± 1.2 |
| epRas23 MG IgG4 | epRT22 GS | IgG4 | | ND |
| epRas33 MG IgG4 | | IgG4 S226P. N434D | | 50.2 ± 2.0 |
| inRas03 (-RT22-i39 MG) | RT22 | IgG1 | hT4-i39 MG | 75.1 ± 7.7 |
| inRas13 | | IgG1 N434D | | 76.0 ± 2.3 |
| Ras03 | RT22 | IgG1 | hT4-59 | 67.3 ± 3.8 |

| | | | | |
|---|---|---|---|---|
| The 2 plasmids that encode the HC and LC of each IgG antibody were co-transfected with the equivalent molar ratio into HEK293F cells in 1 L of culture media. After 6 d of culture, antibodies were purified from the cell culture supernatant using a protein-A affinity column. ^{b} ND, not determined | | | | |

In Table 19 above, the conventional RT22-ep59 MG was newly named "epRas03 MG", the RT22-i39 MG was named "inRas03", and the heavy chain constant region variants were named based thereon.

### Example 27. Determination of reduced intracytoplasmic degradation and non-adherent cell growth inhibition of anti-Ras·GTP iMab having improved intracytoplasmic stability

An improved split green fluorescent protein complementation system (Korean Patent Application No. 10-2015-0143278) was used to determine the decrease in degradation of the anti-Ras·GTP iMab having improved intracytoplasmic stability constructed in Example 25. For this purpose, epRas03-GFP11-SBP2 MG and epRas13-GFP11-SBP2 MG, having a GFP11-SBP2 peptide fused to the heavy-chain C-terminus of epRas03 MG and epRas13 MG, were constructed.

FIG. 18A shows the result of determination of the reduced intracytoplasmic degradation of tumor-tissue EpCAM-specific anti-Ras·GTP iMab using the improved split green fluorescent protein complementation system.

Specifically, an SW480 cell line expressing SA-GFP1-10 at a density of 1x10⁴ cells/well in a 96-well assay plate was diluted in 0.1 ml of medium containing 10% FBS and cultured for 24 hours at 37°C and 5% CO₂. Then, the cells were treated with 2 µM of epRas03 MG, epRas03-GFP11-SBP2 MG and epRas13-GFP11-SBP2 MG for 6 hours, and then the medium was replaced with fresh medium. Then, the cells were cultured for 0, 2, 4, and 6 hours, and green fluorescence at an excitation wavelength of 485 nm and an emission wavelength of 528 nm was quantified using a fluorescent plate reader.

As shown in FIG. 18A, epRas13-GFP11-SBP2 MG with reduced cytoplasmic degradation still retained 60% of original green fluorescence after 6 hours, but the control group epRas03-GFP11-SBP2 MG retained only 5% of original green fluorescence thereof, which indicates that almost all antibodies were degraded. The control group epRas03 MG exhibited little green fluorescence. This indicates that epRas13 MG has improved intracytoplasmic stability.

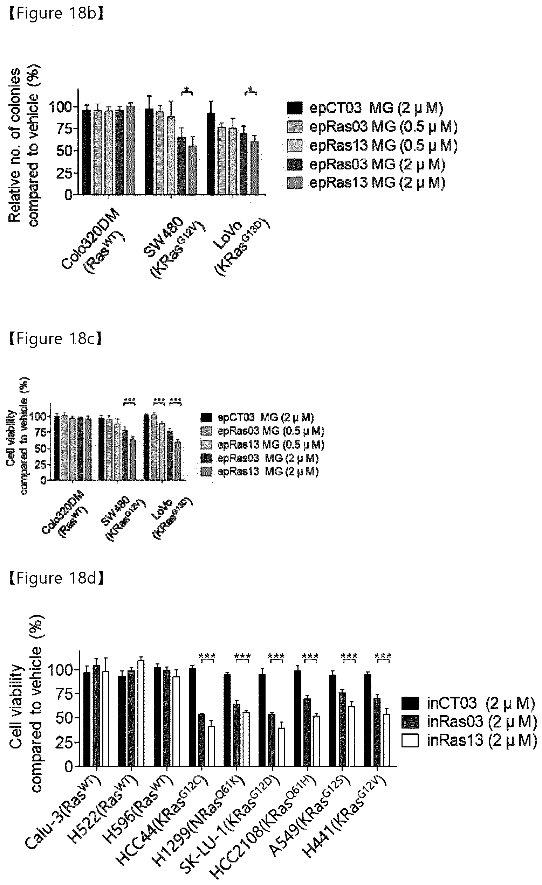

FIG. 18B shows the result of a soft agar colony formation method to determine the inhibitory activity of non-adherent cell growth in a human colorectal cancer cell line by tumor-tissue EpCAM-specific anti-Ras GTP iMab having improved intracytoplasmic stability.

Specifically, 0.2 ml of a mixture of a 1.2% agarose solution and a 2X RPMI + 20% FBS medium in a ratio of 1:1 was spread on a 24-well plate. After the bottom agar was hardened, a 0.7% agarose solution and a medium of 2X RPMI + 20% FBS containing 1x10³ cells + 1 µM or 4 µM antibody were mixed at a ratio of 1:1, and 0.2 ml of the mixture was spread thereon. After the top agar was hardened, 0.2 ml of medium was added thereto. Subsequently, the medium was replaced with a medium containing 0.5 µM or 2 µM of the antibody every 3 days. After 2 to 3 weeks, the colonies were stained with a BCIP/NBT solution, and then the number of colonies having a size of 200 µm or more was measured.

As shown in FIG. 18B, epRas13 MG, having improved intracytoplasmic stability in the Ras mutant cell lines SW480 and Lovo, was found to have cell growth inhibitory ability superior to epRas03 MG, used as a control group. In addition, the wild-type cell line Colo320DM had no difference from the cytoplasmic penetration antibody (epCT03 MG) having no Ras inhibitory ability used as a control group.

FIG. 18C shows the result of an anoikis method conducted to determine the inhibitory activity of non-adherent cell growth in a human colorectal cancer cell line by tumor-tissue EpCAM-specific anti-Ras·GTP iMab having improved intracytoplasmic stability.

Specifically, the cell lines were diluted at a density of 1x10³ cells/well in 0.1 ml of 10% FBS medium in a low-attachment 96-well plate, and cultured along with a 0.5 µM or 2 µM concentration of antibody for 48 hours at 37°C and 5% CO₂. Subsequently, the cells were further treated with 0.5 and 2 µM antibody concentrations and cultured for 48 hours. After culturing for a total of 96 hours, 50 µl of CellTiterGlo (Promega) was added to quantify luminescence.

As shown in FIG. 18C, epRas13 MG, having improved cytoplasmic stability in Ras mutant cell lines SW480 and LoVo, was found to have cell growth inhibitory ability superior to epRas03 MG, used as a control group. In addition, the wild-type cell line Colo320DM had no difference from the cytoplasmic penetration antibody (epCT03 MG) having no Ras inhibitory ability used as a control group.

FIG. 18D shows the result of an anoikis method conducted to determine the inhibitory activity of non-adherent cell growth in a human lung cancer cell line by tumor-tissue integrin-specific anti-Ras·GTP iMab having improved intracytoplasmic stability.

Specifically, the cell lines were diluted at a density of 1x10³ cells/well in 0.1 ml medium containing 10% FBS in a low-attachment 96-well plate, and cultured along with a 2 µM antibody for 48 hours at 37°C, and 5% CO₂. Subsequently, the cells were further treated with the 2 µM antibody and cultured for 48 hours. After culture for a total of 144 hours, 50 µl of CellTiterGlo (Promega) was added to quantify luminescence.

As shown in FIG. 18D, inRas13, having improved cytoplasmic stability in Ras mutant cell lines, was found to have superior cell growth inhibitory ability to inRas03, used as a control group. In addition, the wild-type cell line had no difference from the cytoplasmic penetration antibody (inCT03), having no Ras inhibitory ability used as a control group.

The result showed that both epRas13 MG and inRas13 had cytoplasmic stability superior to the conventional epRas03 MG and inRas03, and thus exhibited an improved cell growth inhibitory effect specific for the Ras mutant cell line.

### Example 28. Pharmacokinetic evaluation of anti-Ras·GTP iMab having improved in-vivo persistence

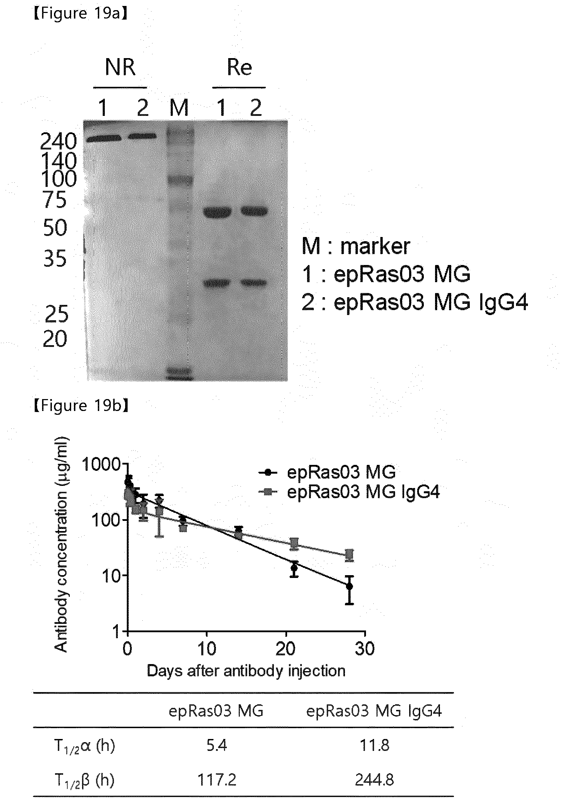

FIG. 19A shows the result of analysis through 12% SDS-PAGE in reducing or non-reducing conditions after purification of tumor-tissue EpCAM-specific anti-Ras GTP having improved *in-vivo* persistence.

Specifically, both epRas03 MG and epRas03 MG IgG4 were found to have a molecular weight of about 150 kDa under non-reducing conditions, and the molecular weight of the heavy chain and the molecular weight of the light chain were found to be 50 kDa and 25 kDa, respectively, under reducing conditions. This indicated that the expressed and purified individual clones were present as monomers (alone) in the solution, and did not form dimers or oligomers through non-natural disulfide bonds.

FIG. 19B shows the result of pharmacokinetic evaluation of tumor tissue EpCAM-specific anti-Ras GTP having improved *in-vivo* persistence in BALB/c node mice.

Specifically, 20 mg/kg of experimental group epRas03 MG IgG4 and control group epRas03 MG were intravenously injected into BALB/c nude mice. After 0.5, 1, 4, 8, 12 and 24 hours and 2, 4, 7, 14, 21 and 28 days, mouse blood was collected. The collected blood was centrifuged at 12,000 rpm for 10 minutes at 4°C and the supernatant plasma was stored at -80°C. Thereafter, ELISA was performed to analyze the concentrations of epRas03 MG IgG4 and epRas03 MG in the plasma. Specifically, the anti-human Fab antibody (Sigma) was bound at a concentration of 2.5 µg/ml to a 96-well half-area plate (Corning) for 1 hour at room temperature and washed three times with 0.1% PBST (PBS, pH 7.4, 0.1% Tween20) . The antibody was bound to 1% PBSB (PBS, pH 7.4, 1% BSA) for 1 hour, the collected blood and purified antibody (for the reference curve) was diluted in 1% PBSB, bound for 2 hours, and then washed three times with 0.1% PBST. Then, the HRP-conjugated anti-human IgG, Fc antibody (Sigma) was bound as a labeling antibody for 1 hour, followed by washing 3 times with 0.1% PBST. The result was reacted with TMB ELISA solution, the reaction was stopped with a sulfuric acid solution, and the absorbance at 450 nm was quantified.

As can be seen from FIG. 19B, the epRas03 MG IgG4 with improved *in-vivo* persistence had a half-life twice as high as that of the control group epRas03 MG.

### Example 29. Determination of improvement of EpCAM-targeting ability of tumor-tissue EpCAM-specific anti-Ras·GTP iMab having improved tumor-tissue-targeting ability

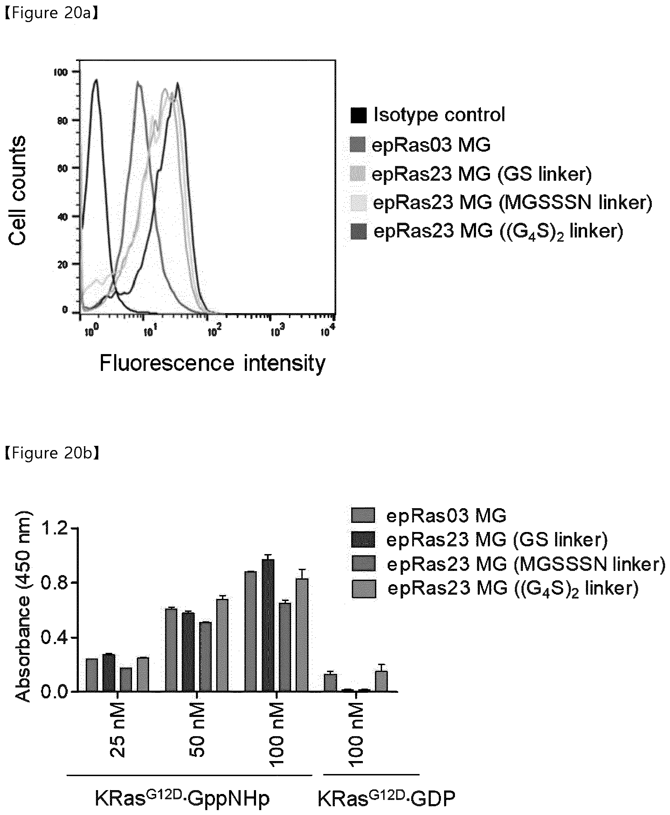

FIG. 20A shows the result of a test using FACS to determine the binding ability to cell-surface EpCAM of anti-Ras·GTP iMab, epRas23 MG, fused with the N-terminal of the heavy-chain variable region in order to improve tumor-tissue-targeting ability.

Specifically, 1×10⁵ LoVo cells were prepared for each sample. The cells were cultured for 1 hour at 4°C in PBSF (PBS buffer, 2% BSA) supplemented with 100 nM of Ras03, epRas03 MG and epRas23 MG. Then, each antibody was reacted with an antibody specifically recognizing human Fc conjugated to Alexa488 (green fluorescence) for 30 minutes at 4°C. Then, the cells were washed with PBS and analyzed by flow cytometry. As a result, the fluorescence intensities of epRas03 MG and epRas23 MG antibodies bound to LoVo cells expressing EpCAM were measured, and epRas23 MG fused with more EpCAM-targeting peptides had higher fluorescence intensity.

FIG. 20B shows the result of ELISA performed to analyze the binding ability to 25, 50 and 100 nM Avi-KRas^{G12D}·GppNHp or 100 nM Avi-KRas^{G12D}·GDP of anti-Ras·GTP iMabs including EpCAM-targeting peptide fused with the N-terminal of the heavy-chain variable region in order to improve tumor-tissue-targeting ability.

Specifically, ELISA was conducted in the same manner as in Example 4, and epRas03 MG and epRas23 MG, tumor-tissue-specific anti-Ras·GTP iMabs including EpCAM-targeting peptide fused with the N-terminal of the heavy-chain variable region in order to improve tumor-tissue-targeting ability were fixed on a 96-well EIA/RIA plate, after which the GppNHp-bound KRas protein was bound at various concentrations of 100 nM, 50 nM and 25 nM, and the GDP-bound KRas protein was bound at a concentration of 100 nM and was then bound to an HRP-conjugated anti-his antibody (HRP-conjugated anti-his mAb) as a labeling antibody. The result was reacted with TMB ELISA solution, and the absorbance at 450 nm was quantified.

The result showed that the fusion of EpCAM-targeting peptide with the N-terminal of the heavy-chain variable region caused no difference in the affinity for Ras.

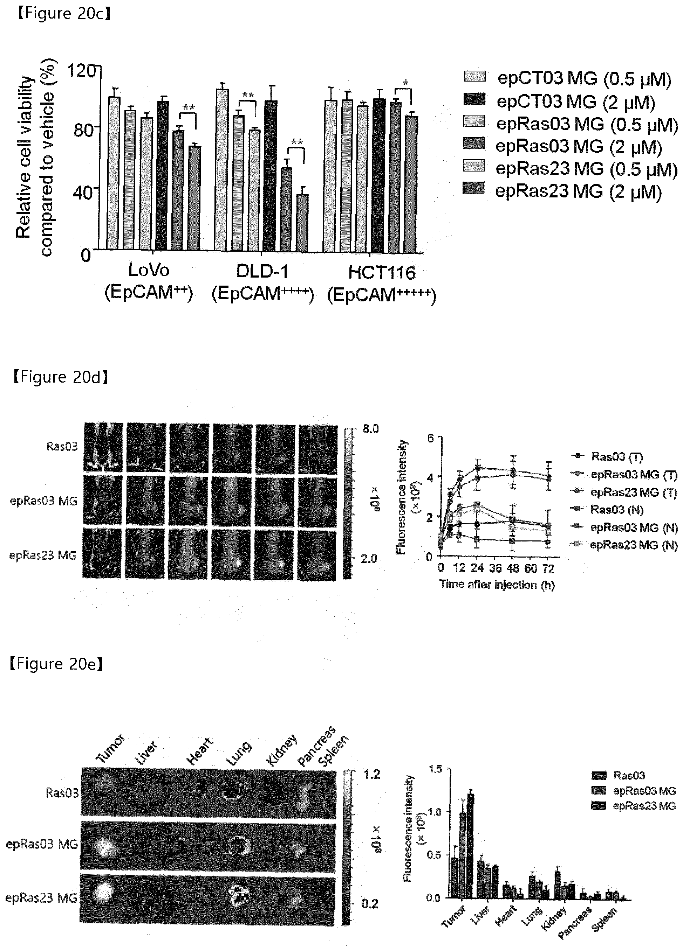

FIG. 20C shows the result of an anoikis method conducted to determine the inhibitory activity of non-adherent cell growth in a human lung cancer cell line by epRas23 MG, anti-Ras·GTP iMab having an EpCAM-targeting peptide fused with the N-terminal of the heavy-chain variable region to improve tumor-tissue-targeting ability.

Specifically, LoVo, DLD-1 and HCT116 cell lines were each diluted at a density of 1x10³ cells/well in 0.1 ml of a 10% FBS medium in a low-attachment 96-well plate, and cultured along with a 0.5 or 2 µM antibody for 48 hours at 37°C under 5% CO₂. Subsequently, the cells were further treated with the 0.5 or 2 µM antibody and cultured for 48 hours. After culture for a total of 96 hours, 50 µl of CellTiterGlo (Promega) was added thereto to quantify luminescence.

As can be seen from FIG. 20C, epRas23 MG, having improved cytoplasmic stability in Ras mutant cell lines, LoVo, DLD-1 and HCT116, was found to have cell growth inhibitory ability superior to epRas03 MG used as a control group.

FIG. 20D is an image showing the bio-distribution in a mouse of anti-Ras·GTP iMab, epRas23 MG, including the EpCAM-targeting peptide fused with the N-terminal of the heavy-chain variable region to improve tumor-tissue-targeting ability, and a graph showing the fluorescence of the tumor and the entire body.

Specifically, 20 µg of DyLight fluorescently labeled anti-Ras·GTP iMabs, Ras03, epRas03 MG, and epRas23 MG were injected into BALB/c nude mice, and fluorescence emitted from the entire bodies of the mice was observed at 0, 6, 12, 24, 48 and 72 hours with an IVIS Lumina XRMS Series III (Perkin Elmer). At this time, the mice were anesthetized using 1.5-2.5% isoflurane (Piramal Critical Care). Each image shows fluorescence values of the entire body quantified using Living Image software (Perkin Elmer).

As can be seen from FIG. 20D, epRas03 MG and epRas23 MG have larger amounts of antibodies present in tumor tissue than Ras03 not fused with the EpCAM-targeting peptide. In addition, it can be seen that epRas23 MG has a larger amount of antibodies in tumor tissue over time after antibody injection than epRas03 MG.

FIG. 20E is an image showing the bio-distribution in a mouse of anti-Ras·GTP iMab, epRas23 MG, including the EpCAM-targeting peptide fused with the N-terminal of the heavy-chain variable region to improve tumor-tissue-targeting ability, and a graph showing the fluorescence quantified using the extracted organ.

Specifically, following the experiment, 72 hours after antibody injection, the mice were euthanized, the tumors, heart, lungs, liver, kidneys, pancreas and spleen were extracted and the fluorescence in each of these organs was quantified using Living Image software (Perkin Elmer).

As can be seen from FIG. 20E, epRas03 MG and epRas23 MG had larger amounts of antibodies present in tumor tissues and smaller amounts of antibodies present in other organs than Ras03 not fused with the EpCAM-targeting peptide. In addition, the amounts of antibody in tumor tissues increased in the order of epRas23 MG, epRas03 MG and Ras03.

### Example 30. Design and expression/purification of heavy-chain modified variant to improve in-vivo persistence of anti-Ras·GTP iMab

An immunoglobulin-type antibody binds to the Fc receptor of immune cells in the body and is removed through an ADCC mechanism. IgG1 has weak binding force to the Fc gamma receptor due to the introduction of L234A, L235A and P239G mutations and thus inhibition of this function. Therefore, in order to improve the *in-vivo* stability of anti-Ras·GTP iMab, the intact immunoglobulin IgG1 antibody, a mutant (IgG1 LALA-PG) in which L234A, L235A and P239G mutations (LALA-PG) were introduced into the heavy-chain constant regions (CH1 to CH3) of IgG1 was constructed. Specifically, in the same manner as in Example 4, cytoplasm-penetrating humanized light-chain expression vector (hT4-ep59 GSSG LC) and the heavy-chain expression vector (RT22 IgG1 LALA-PG or RT22 IgG1 LALA-PG, N434D) of the anti-Ras·GTP iMab having improved *in-vivo* persistence were subjected to transient co-transfection into HEK293F protein-expressing cells to express an anti-Ras·GTP iMab mutant with improved *in-vivo* persistence.

The following Table 20 shows the sequence information of the heavy-chain constant region (CH1-CH3) introduced with the LALA-PG mutant to improve the *in-vivo* persistence of anti-Ras·GTP iMab.

**[Table 20]**

| VH name | Sequence | SEQ ID NO. |
|---|---|---|
| IgG1 LALA-PG | 1 10 20 30 40 50 60 ASTKGPSVFPLAPSSKSTSGGTAALGCLVKDYFPEPVTVSWNSGALTSGVHTFPAVLQSS 70 80 90 100 110 120 GLYSLSSVVTVPSSSLGTQTYICNVNHKPSNTKVDKKVEPKSCDKTHTCPPCPAPEAAGG 130 140 150 160 170 180 PSVFLFPPKPKDTLMISRTPEVTCVVVDVSHEDPEVKFNWYVDGVEVHNAKTKPREEQYN 190 200 210 220 230 240 STYRVVSVLTVLHQDWLNGKEYKCKVSNKALGAPIEKTISKAKGQPREPQVYTLPPSRDE 250 260 270 280 290 300 LTKNQVSLTCLVKGFYPSDIAVEWESNGQPENNYKTTPPVLDSDGSFFLYSKLTVDKSRW 310 320 330 QQGNVFSCSVMHEALHNHYTQKSLSLSPGK | **68** |
| IgG1 LALA-PG, N434D | 1          10          20          30          40          50          60 ASTKGPSVFPLAPSSKSTSGGTAALGCLVKDYFPEPVTVSWNSGALTSGVHTFPAVLQSS 70          80          90          100         110         120 GLYSLSSVVTVPSSSLGTQTYICNVNHKPSNTKVDKKVEPKSCDKTHTCPPCPAPEAAGG 130         140         150         160         170         180 PSVFLFPPKPKDTLMISRTPEVTCVVVDVSHEDPEVKFNWYVDGVEVHNAKTKPREEQYN 190         200         210         220         230         240 STYRVVSVLTVLHQDWLNGKEYKCKVSNKALGAPIEKTISKAKGQPREPQVYTLPPSRDE 250         260         270         280         290         300 LTKNQVSLTCLVKGFYPSDIAVEWESNGQPENNYKTTPPVLDSDGSFFLYSKLTVDKSRW 310         320         330 QQGNVFSCSVMHEALHDHYTQKSLSLSPGK | **69** |

### Example 31. Pharmacokinetic evaluation of LALA-PG mutant to improve in-vivo persistence of tumor-tissue-specific anti-Ras·GTP iMab

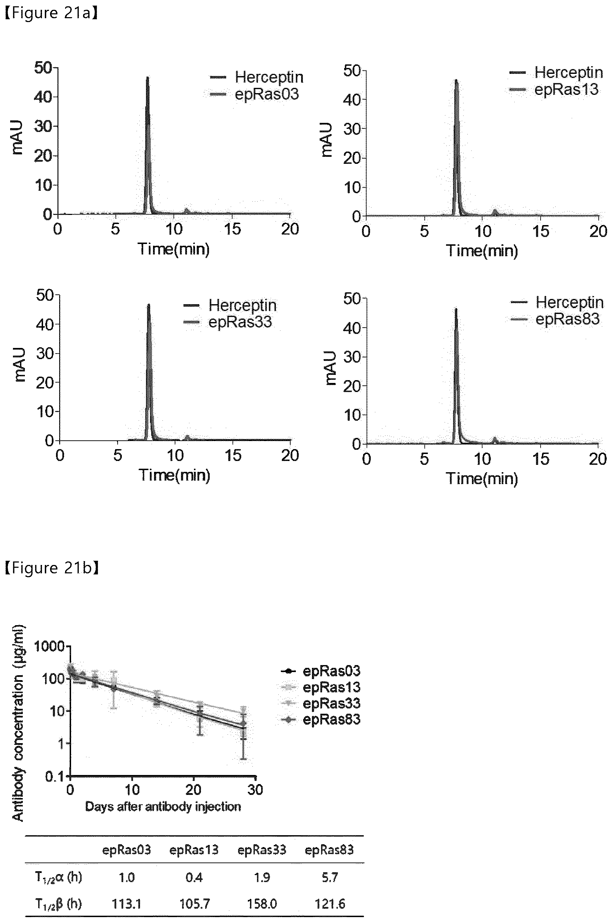

FIG. 21A shows the result of size-exclusion chromatography to determine whether or not the LALA-PG mutant to improve *in-vivo* persistence of tumor-tissue-specific anti-Ras·GTP iMab is a multimer.

Specifically, the size-exclusion chromatography was performed using an Agilent Technologies HPLC 1200 series. The column used herein was a Zenix® SEC-300 column. The experiment was conducted at a flow rate of 1 ml/min. The mobile phase included 150 mM sodium phosphate (pH 7.0). The diluent was the mobile phase, and analysis was performed with 280 nm UV. It was identified that the antibody was purified as a monomer, not a multimer, from all iMabs.

FIG. 21B shows the result of pharmacokinetic evaluation, in BALB/c nude mice, of the LALA-PG mutant to improve *in-vivo* persistence of tumor-tissue-EpCAM- specific anti-Ras·GTP iMab.

Specifically, epRas03, epRas13, epRas33 and epRas83 were intravenously injected at 20 mg/kg into BALB/c nude mice. After 0.5, 1, 4, 8, 12 and 24 hours and 2, 4, 7, 14, 21 and 28 days, mouse blood was collected. The collected blood was centrifuged at 12,000 rpm for 10 minutes at 4°C and only the supernatant plasma was stored at -80°C. Then, ELISA was performed to analyze the concentrations of epRas03, epRas13, epRas33 and epRas83 in the plasma. Specifically, in the same manner as in Example 28, an anti-human Fab antibody (Sigma) was bound on a 96-well half-area plate (Corning) at a concentration of 2.5 µg/ml for 1 hour at room temperature and then washed three times with 0.1% PBST (PBS, pH 7.4, 0.1% Tween20) . After binding with 1% PBSB (PBS, pH 7.4, 1% BSA) for 1 hour, the collected blood and purified antibody (for the reference curve) was diluted in 1% PBSB, bound for 2 hours, and then washed three times with 0.1% PBST 3. Then, the antibody was bound to an HRP-conjugated anti-human IgG, Fc antibody (Sigma) as a labeling antibody for 1 hour and then washed 3 times with 0.1% PBST. The result was reacted with TMB ELISA solution, the reaction was stopped with a sulfuric acid solution, and the absorbance at 450 nm was quantified.

As can be seen from FIG. 21, epRas33 and epRas83 introduced with the LALA-PG mutant had a longer half-life than the control group, epRas03, and the epRas13 had a slightly reduced half-life compared to epRas03.

### Example 32. Evaluation of binding ability to GppNHp-bound KRas^{G12D} and neonatal Fc receptor (FcRn) of LALA-PG mutant to improve in-vivo persistence of anti-Ras·GTP iMab

The binding ability was analyzed based on SPR (surface plasmon resonance) using a Biacore2000 instrument to identify that the LALA-PG mutant to improve *in-vivo* persistence of anti-Ras·GTP iMab maintains the binding ability to GppNHp-bound KRas^{G12D} and has no influence on the binding ability to FcRn.

Specifically, in order to determine the binding ability to GppNHp-bound KRas^{G12D}, epRas03, epRas13, epRas83, inRas03 MG, inRas13 MG and inRas83 MG were diluted to a concentration of 20 µl/ml in 10 mM NaAc buffer (pH 4.0) and immobilized at approximately 1800 response units (RU) on a CM5 sensor chip (GE Healthcare). Subsequently, Tris buffer (20 mM Tris-HCl, pH 7.4, 137 mM NaCl, 5 mM MgCl₂, 0.01% Tween 20) was analyzed at a flow rate of 30 µl/min, and complete GppNHp-bound GST-KRas^{G12D} was analyzed at a concentration of 100 nM to 6.25 nM. After binding and dissociation analysis, regeneration of the CM5 chip was performed by flowing a buffer (20 mM NaOH, 1M NaCl, pH 10.0) at a flow rate of 30 µl/min for 1 minute. Each sensorgram, obtained by binding for 3 minutes and dissociation for 3 minutes, was normalized with reference to a blank cell and subtracted, and the affinity was thus calculated.

Specifically, in order to determine the binding ability to FcRn, FcRn was diluted to a concentration of 20 µl/ml in 10 mM NaAc buffer (pH 4.0) and immobilized at approximately 650 response units (RU) on a CM5 sensor chip (GE Healthcare). Subsequently, a phosphate buffer (12 mM phosphate, pH 6.0, 137 mM NaCl, 0.01% Tween 20) was analyzed at a flow rate of 30 µl/min, epRas03 was analyzed at a concentration of 200 nM to 6.25 nM, and epRas13 and epRas83 were analyzed at a concentration of 2000 nM to 62.5 nM. After binding and dissociation analysis, regeneration of the CM5 chip was performed by flowing a phosphate buffer (12 mM phosphate, pH 7.4, 137 mM NaCl, 0.01% Tween 20) at a flow rate of 30 µl/min for 3 minutes. Each sensorgram, obtained by binding for 3 minutes and dissociation for 6 minutes, was normalized with reference to a blank cell and subtracted, and the affinity was thus calculated.

The following Table 21 shows the result of analysis, based on SPR using a BIACORE2000 instrument, of the affinity for complete GppNHp-bound KRas^{G12D} and FcRn of the LALA-PG mutant to improve the *in-vivo* persistence of anti-Ras·GTP iMabs.

**[Table 21]**

| IgG1,κ format | Heavy chain | | Light chain | KRas^{G12D.} GppNHp *K*_{D}(M)^{a} | hFcRn *K*_{D}(M)^{a} at pH 6.0 |
|---|---|---|---|---|---|
| | VH | CH1-CH3 | | | |
| epRes03 | RT22 | IgG1 | hT4-ep59 GSSG | 8.59 ± 0.32×19⁻⁹ | 1.46×10⁻⁷ |
| epRas13 | | IgG1 N434D | | 8.97 ± 0.66×10⁻⁹ | 2.7×10⁻⁶ |
| epRas33 | | IgG1 LALA-PG | | ND | ND |
| epRas83 | | IgG1 LALA-PG, N434D | | 8.94×10⁻⁹ | 3.57×10⁻⁶ |
| inRas03 MG | | IgG1 | hT4-í59 MG | 6.4×10⁻⁹ | ND |
| inRas13 MG | | IgG1 N434D | | 7.53×10⁻⁹ | ND |
| inRas33 MG | | IgG1 LALA-PG | | ND | ND |
| inRas83 MG | | IgG1 LALA-PG, N434D | | 7.07×10⁻⁹ | ND |
| Herceptin | | | | ND | 2.28×10⁻⁷ |

| | | | | | |
|---|---|---|---|---|---|
| ^{a} ND, not determined | | | | | |

### Example 33. Determination of tumor growth inhibition ability of LALA-PG mutant to improve in-vivo persistence of tumor-tissue integrin-specific anti-Ras·GTP iMab

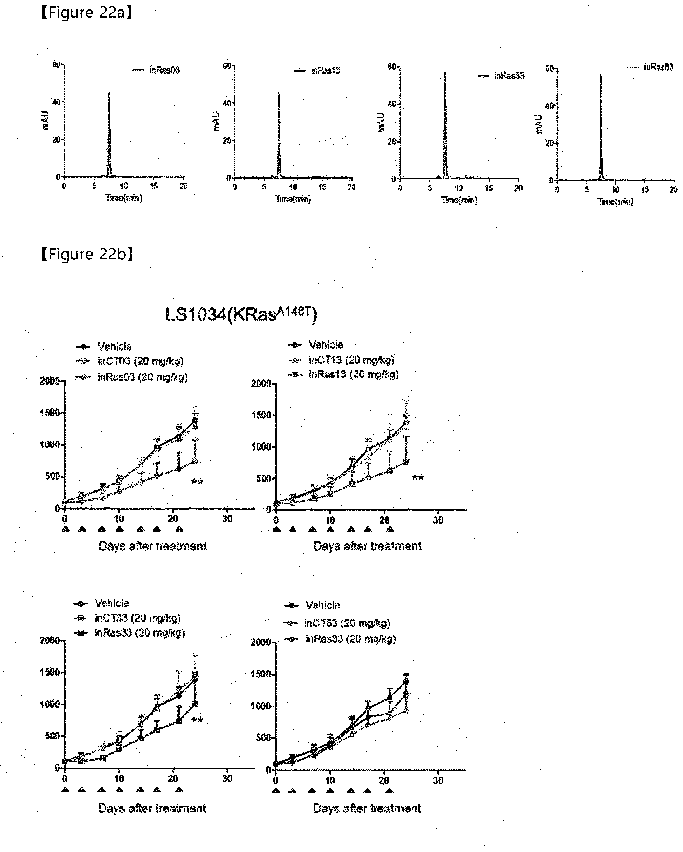

FIG. 22A shows the result of size-exclusion chromatography to determine the presence as a multimer in the LALA-PG mutant to improve the *in-vivo* persistence of tumor-tissue integrin-specific anti-Ras·GTP iMab.

Specifically, size-exclusion chromatography was performed using an Agilent Technologies HPLC 1200 series. The column used herein was a Zenix® SEC-300 column. The experiment was conducted at a flow rate of 1 ml/min. The mobile phase included 150 mM sodium phosphate (pH 7.0). The diluent was the mobile phase, and analysis was performed with 280 nm UV. It was identified that the antibody was purified as a monomer in 95% or more of all iMabs.

FIG. 22B shows the result of a test to compare the tumor growth inhibition effect of the LALA-PG mutant to improve the *in-vivo* persistence of tumor-tissue integrin-specific anti-Ras cell-penetrating antibody in human colorectal cancer KRas mutant cell line LS1034 xenograft mice.

Specifically, the KRas^{A146T} mutant human colorectal cancer cell line LS1034 was subcutaneously injected into BALB/c nude mice at a density of 1×10⁷ cells/mouse. After about 14 days, when the tumor volume reached about 120 mm³, the equivalent volume of a PBS vehicle control group, control groups (inCT03 MG, inCT13 MG, inCT33 MG, inCT83 MG), and experimental groups (inRas03 MG, inRas13 MG, inRas33 MG, inRas83 MG) were injected intravenously at 20 mg/kg. A total of 7 intravenous injections were performed every 3 to 4 days, that is, twice a week, and the tumor volume was measured for 24 days using a caliper.

As can be seen from FIG. 22B, cancer cell growth was inhibited in mice administered with inRas03 MG (control: inCT03 MG), inRas13 MG (control: inCT13 MG), and inRas33 MG (control: inCT33 MG). On the other hand, it can be seen that cancer cell growth was not inhibited in mice administered with inRas83 (control: inCT83 MG).

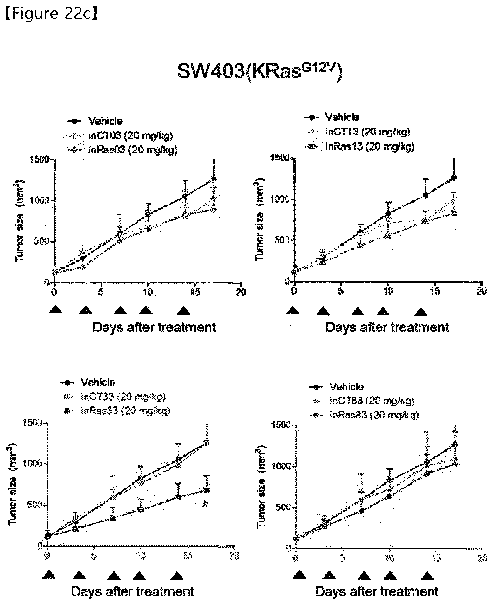

FIG. 22C shows the result of a test to compare the tumor growth inhibition effect of the LALA-PG mutant to improve the *in-vivo* persistence of tumor-tissue integrin-specific anti-Ras cell-penetrating antibody in human colorectal cancer KRas mutant cell line SW403 xenograft mice.

Specifically, the KRas^{G12V} mutant human colorectal cancer cell line SW403 was subcutaneously injected into BALB/c nude mice at a density of 1×10⁷ cells/mouse. After about 14 days, when the tumor volume reached about 120 mm³, the equivalent volume of a PBS vehicle control group, control groups (inCT03 MG, inCT13 MG, inCT33 MG, inCT83 MG), and experimental groups (inRas03 MG, inRas13 MG, inRas33 MG, inRas83 MG) were injected intravenously at 20 mg/kg. A total of 5 intravenous injections were performed every 3 to 4 days twice for a week, and the tumor volume was measured for 17 days using a caliper.

As can be seen from FIG. 22C, cancer cell growth was inhibited in mice administered with inRas33 MG (control: inCT33 MG). On the other hand, it can be seen that cancer cell growth was not inhibited in mice administered with inRas03 MG (control: inCT03 MG), inRas13 MG (control: inCT13 MG), or inRas83 (control: inCT83 MG).

As can be seen from FIG. 22, inRas33 MG improved tumor growth inhibitory ability, whereas inRas13 MG and inRas83 MG had similar or worsened tumor growth inhibitory ability.

### [Industrial availability]

The method for improving the tumor inhibition efficiency of an antibody that specifically penetrates into the cytoplasm of tumor tissue cells in the form of an intact immunoglobulin to directly inhibit intracellular Ras·GTP, provided by the present disclosure, is accomplished by selecting heavy-chain variable regions with improved affinity for Ras·GTP and developing light-chain variable regions with improved cytoplasmic penetration ability specifically for tumor tissues, and is capable of targeting Ras·GTP, which is located in certain tumor cells and is always activated through mutation, and of effectively inhibiting the activity thereof.

In addition, the light-chain variable region, imparting improved tumor-tissue-specific cytoplasmic penetration ability to the antibody provided by the present disclosure or the antibody including the same, lowers the binding ability to HSPG expressed in most normal cells and thereby makes it possible to provide endocytosis and endosomal escape through receptors expressed specifically for tumor tissues by the peptide fused for targeting tumor tissues, and the heavy-chain variable region (VH) having improved affinity for Ras·GTP has improved Ras inhibition ability when the antibody reaches the cytoplasm. The antibody, which specifically penetrates into the cytoplasm of tumor tissue cells in the form of an intact immunoglobulin including a combination of the modified light-chain variable region and the heavy-chain variable region, and directly inhibits intracellular Ras·GTP, exhibits improved tumor growth inhibitory ability based thereon.

The cytoplasmic penetration Ras-inhibitory antibody including a combination of the improved light-chain variable region and the heavy-chain variable region according to the present disclosure can be easily developed into a therapeutic drug owing to high production yield, is capable of effectively inhibiting mutant Ras through tumor-tissue-specific cytoplasmic penetration, and is thus expected to exert effective anti-cancer activity through treatment using a single drug or a combination thereof with a conventional therapeutic drug.

### [Sequence Listing Free Text]

An electronic file is attached.

## Claims

1. A heavy-chain variable region specifically binding to Ras(Ras·GTP) activated by GTP bound thereto, the heavy-chain variable region comprising:
CDR1 having an amino acid sequence represented by the following Formula 1;
CDR2 having an amino acid sequence represented by the following Formula 2; and
CDR3 having an amino acid sequence represented by the following Formula 3,
[Formula 1] D-X₁₁-SMS
wherein X₁₁ is F or Y,
[Formula 2] YISRTSHT-X₂₁-X₂₂-YADSVKG
wherein X₂₁ is T, I or L, and X₂₂ is Y, C, S, L or A,
[Formula 3] G-F-X₃₁-X₃₂-X₃₃-Y
wherein X₃₁ is K, F, R or N, X₃₂ is M or L, and X₃₃ is D or N.

2. The heavy-chain variable region according to claim 1, wherein
CDR1 having an amino acid sequence represented by the following Formula 1;
CDR2 having an amino acid sequence represented by the following Formula 2; and
CDR3 having an amino acid sequence represented by the following Formula 3,
[Formula 1] D-X₁₁-SMS
wherein X₁₁ is F or Y,
[Formula 2] YISRTSHT-X₂₁-X₂₂-YADSVKG
wherein X₂₁-X₂₂ is TY, IY, TC, TS, IS, LC, LL or IA,
[Formula 3] G-F-X₃₁-X₃₂-X₃₃-Y
wherein X₃₁-X₃₂-X₃₃ is KMD, RMD, FMN, RLD or NLD.

3. The heavy-chain variable region according to claim 1, wherein the CRD1 sequence of the heavy-chain variable region is selected from the group consisting of amino acid sequences represented by SEQ ID NOS: 2 to 4, the CDR2 sequence of the heavy-chain variable region is selected from the group consisting of amino acid sequences represented by SEQ ID NO: 5 and SEQ ID NOS: 10 to 16, and the CDR3 sequence of the heavy-chain variable region is selected from the group consisting of amino acid sequences represented by SEQ ID NOS: 6 to 9 and SEQ ID NOS: 17 to 18.

4. The heavy-chain variable region according to claim 1, wherein the heavy-chain variable region is selected from the group consisting of:
i) a heavy-chain variable region comprising CDR1 of SEQ ID NO: 2, CDR2 of SEQ ID NO: 5 and CDR3 of SEQ ID NO: 7;
ii) a heavy-chain variable region comprising CDR1 of SEQ ID NO: 3, CDR2 of SEQ ID NO: 5 and CDR3 of SEQ ID NO: 7;
iii) a heavy-chain variable region comprising CDR1 of SEQ ID NO: 4, CDR2 of SEQ ID NO: 5 and CDR3 of SEQ ID NO: 8;
iv) a heavy-chain variable region comprising CDR1 of SEQ ID NO: 3, CDR2 of SEQ ID NO: 5 and CDR3 of SEQ ID NO: 9;
v) a heavy-chain variable region comprising CDR1 of SEQ ID NO: 3, CDR2 of SEQ ID NO: 5 and CDR3 of SEQ ID NO: 8;
vi) a heavy-chain variable region comprising CDR1 of SEQ ID NO: 3, CDR2 of SEQ ID NO: 10 and CDR3 of SEQ ID NO: 7;
vii) a heavy-chain variable region comprising CDR1 of SEQ ID NO: 3, CDR2 of SEQ ID NO: 11 and CDR3 of SEQ ID NO: 7;
viii) a heavy-chain variable region comprising CDR1 of SEQ ID NO: 3, CDR2 of SEQ ID NO: 12 and CDR3 of SEQ ID NO: 7;
ix) a heavy-chain variable region comprising CDR1 of SEQ ID NO: 3, CDR2 of SEQ ID NO: 13 and CDR3 of SEQ ID NO: 7;
x) a heavy-chain variable region comprising CDR1 of SEQ ID NO: 3, CDR2 of SEQ ID NO: 14 and CDR3 of SEQ ID NO: 17;
xi) a heavy-chain variable region comprising CDR1 of SEQ ID NO: 3, CDR2 of SEQ ID NO: 15 and CDR3 of SEQ ID NO: 18; and
xii) a heavy-chain variable region comprising CDR1 of SEQ ID NO: 3, CDR2 of SEQ ID NO: 16 and CDR3 of SEQ ID NO: 7.

5. The heavy-chain variable region according to claim 1, wherein the heavy-chain variable region is selected from the group consisting of amino acid sequences represented by SEQ ID NOS: 20 to 32.

6. An intact immunoglobulin antibody comprising the heavy-chain variable region according to any one of claims 1 to 5.

7. The intact immunoglobulin antibody according to claim 6, wherein the antibody has cytoplasmic penetration ability.

8. The intact immunoglobulin antibody according to claim 6, wherein a light-chain variable region of the antibody is selected from the group consisting of amino acid sequences represented by SEQ ID NOS: 34 to 43.

9. The intact immunoglobulin antibody according to claim 6, wherein the light-chain variable region or the heavy-chain variable region of the antibody is fused with a peptide targeting EpCAM (epithelial cell adhesion molecule), integrin αvβ3 or integrin αvβ5.

10. The intact immunoglobulin antibody according to claim 9, wherein the light-chain variable region or the heavy-chain variable region is selected from the group consisting of amino acid sequences represented by SEQ ID NOS: 44 to 63.

11. The intact immunoglobulin antibody according to claim 6, wherein the antibody comprises a heavy-chain constant region or a light-chain constant region derived from human immunoglobulin selected from the group consisting of IgA, IgD, IgE, IgG1, IgG2, IgG3, IgG4, and IgM.

12. The intact immunoglobulin antibody according to claim 11, wherein the heavy-chain constant region comprises at least one mutation of N434D of the CH3, L234A, L235A and P329G of the CH2,
wherein the amino acid position is determined according to EU numbering.

13. The intact immunoglobulin antibody according to claim 12, wherein the mutation of the heavy-chain constant region is selected from the group consisting of amino acid sequences represented by SEQ ID NOS: 65 to 69.

14. The intact immunoglobulin antibody according to claim 6, wherein the antibody is selected from the group consisting of single-chain Fvs (scFV), single-chain antibodies, Fab fragments, F(ab') fragments, disulfide-binding Fvs (sdFV) and epitope-binding fragments of the antibodies.

15. The intact immunoglobulin antibody according to claim 6, wherein the antibody is a bispecific antibody (bispecific Ab).

16. The intact immunoglobulin antibody according to claim 6, wherein the antibody is fused with one or more selected from the group consisting of proteins, peptides, small-molecule drugs, toxins, enzymes, nucleic acids and nanoparticles.

17. A method for preparing an intact immunoglobulin-type antibody having improved affinity for intracellular Ras·GTP and tumor-tissue-specific cytoplasmic penetration ability, the method comprising:
(1) preparing an endosomal escape heavy-chain expression vector cloned with nucleic acids, substituted with a humanized heavy-chain variable region (VH) having improved affinity for intracellular Ras·GTP from a heavy-chain variable region (VH) included in a heavy chain comprising a human heavy-chain variable region (VH) and a human heavy-chain constant region (CH1-hinge-CH2-CH3);
(2) preparing a cytoplasmic penetration light-chain expression vector cloned with nucleic acids, substituted with a humanized light-chain variable region (VL) having cytoplasmic penetration ability and a humanized light-chain variable region (VL) having cytoplasmic penetration ability specific for tumor tissues from a light-chain variable region (VL) included in a light chain comprising a human light-chain variable region (VL) and a human light-chain constant region (CL) ;
(3) co-transforming the prepared heavy- and light-chain expression vectors into animal cells for protein expression to express an intact immunoglobulin-type antibody including a heavy-chain variable region (VH) having improved affinity for intracellular Ras·GTP and a light-chain variable region (VL) having cytoplasmic penetration ability specific for tumor tissues; and
(4) purifying and recovering the expressed intact immunoglobulin-type antibody.

18. A composition for preventing or treating cancer comprising the antibody according to any one of claims 1 to 16.

19. The composition according to claim 18, wherein the cancer has a mutation associated with an activated intracellular Ras.

20. The composition according to claim 19, wherein the mutation associated with the activated intracellular Ras is cancer having a mutation in 12nd, 13rd or 61st amino acid of the Ras.

21. The composition according to claim 18, wherein the preventing or treating cancer is **characterized in that** the antibody according to any one of claims 5 to 15 inhibits binding of activated Ras (Ras·GTP) to B-Raf, C-Raf or PI3K in the cytoplasm.

22. A composition for diagnosing tumors comprising the antibody according to any one of claims 1 to 16.

23. A polynucleotide encoding the antibody according to any one of claims 1 to 16.

24. A vector comprising the polynucleotide according to claim 23.

25. A method for constructing a heavy-chain variable region library specifically binding to Ras·GTP and having improved affinity therefor, the method comprising:
(1) determining an amino acid site of three complementarity determining regions (CDRs) having high potential to bind to intracellular Ras·GTP involved in antigen binding of a RT11 heavy-chain variable region (VH) library template;
(2) designing a degenerated codon primer capable of encoding an amino acid in need of inclusion in a library at the determined amino acid site; and
(3) expressing the heavy-chain variable-region of designed library in a form of scFab or Fab using a yeast surface expression system.

26. A library of a heavy-chain variable region specifically binding to Ras·GTP and having improved affinity therefor, constructed by the method according to claim 25.

27. A method for screening a heavy-chain variable region specifically binding to Ras·GTP and having improved affinity therefor, the method comprising:
(1) expressing the heavy-chain variable-region library capable of binding to Ras·GTP, prepared according to (3) in claim 25, using a yeast surface expression system;
(2) constructing Avi-KRas^{G12D} bound to GppNHp, a GTP analogue, in a stable form without deformation during biotinylation;
(3) binding the heavy-chain variable-region library with the GppNHp-bound Avi-KRas^{G12D}; and
(4) measuring affinity of binding between the heavy-chain variable-region library and the GppNHp-bound Avi-KRas^{G12D}.


<110>    ORUM THERAPEUTICS INC.

<120>    Antibody which internalize into the cytosol of cells and binds to
         inhibit activated Ras and use thereof

<130>    PP-B2124

<150>    KR 17/152,998
<151>    2017-11-16

<160>    69

<170>    PatentIn version 3.5

<210>    1
<211>    5
<212>    PRT
<213>    Artificial Sequence

<220>
<223>    RT11 VH-CDR1


<400>    1
Ser Tyr Ser Met Ser
1               5


<210>    2
<211>    5
<212>    PRT
<213>    Artificial Sequence

<220>
<223>    RT21 VH-CDR1


<400>    2
Asp Tyr Ser Met Ser
1               5


<210>    3
<211>    5
<212>    PRT
<213>    Artificial Sequence

<220>
<223>    RT22 VH-CDR1


<400>    3
Asp Phe Ser Met Ser
1               5


<210>    4
<211>    5
<212>    PRT
<213>    Artificial Sequence

<220>
<223>    RT23 VH-CDR1

<400>    4
Asp Tyr Ser Met Ser
 1               5


<210>    5
<211>    17
<212>    PRT
<213>    Artificial Sequence

<220>
<223>    RT11 VH-CDR2


<400>    5
Tyr Ile Ser Arg Thr Ser His Thr Thr Tyr Tyr Ala Asp Ser Val Lys
 1               5                   10                  15

Gly




<210>    6
<211>    6
<212>    PRT
<213>    Artificial Sequence

<220>
<223>    RT11 VH-CDR3


<400>    6
Gly Phe Phe Met Asp Tyr
 1               5


<210>    7
<211>    6
<212>    PRT
<213>    Artificial Sequence

<220>
<223>    RT21 VH-CDR3


<400>    7
Gly Phe Lys Met Asp Tyr
 1               5


<210>    8
<211>    6
<212>    PRT
<213>    Artificial Sequence

<220>
<223>    RT23 VH-CDR3


<400>    8
Gly Phe Arg Met Asp Tyr

1               5


<210>    9
<211>    6
<212>    PRT
<213>    Artificial Sequence

<220>
<223>    RT24 VH-CDR3


<400>    9
Gly Phe Phe Met Asn Tyr
1               5


<210>    10
<211>    17
<212>    PRT
<213>    Artificial Sequence

<220>
<223>    RT31 VH-CDR2


<400>    10
Tyr Ile Ser Arg Thr Ser His Thr Ile Tyr Tyr Ala Asp Ser Val Lys
1               5                   10                  15

Gly


<210>    11
<211>    17
<212>    PRT
<213>    Artificial Sequence

<220>
<223>    RT32 VH-CDR2


<400>    11
Tyr Ile Ser Arg Thr Ser His Thr Thr Cys Tyr Ala Asp Ser Val Lys
1               5                   10                  15

Gly


<210>    12
<211>    17
<212>    PRT
<213>    Artificial Sequence

<220>
<223>    RT33 VH-CDR2


<400>    12
Tyr Ile Ser Arg Thr Ser His Thr Thr Ser Tyr Ala Asp Ser Val Lys
1               5                   10                  15

Gly

<210> 13
<211> 17
<212> PRT
<213> Artificial Sequence

<220>
<223> RT34 VH-CDR2

<400> 13
Tyr Ile Ser Arg Thr Ser His Thr Ile Ser Tyr Ala Asp Ser Val Lys
1               5                   10                  15

Gly

<210> 14
<211> 17
<212> PRT
<213> Artificial Sequence

<220>
<223> RT35 VH-CDR2

<400> 14
Tyr Ile Ser Arg Thr Ser His Thr Leu Cys Tyr Ala Asp Ser Val Lys
1               5                   10                  15

Gly

<210> 15
<211> 17
<212> PRT
<213> Artificial Sequence

<220>
<223> RT36 VH-CDR2

<400> 15
Tyr Ile Ser Arg Thr Ser His Thr Leu Leu Tyr Ala Asp Ser Val Lys
1               5                   10                  15

Gly

<210> 16
<211> 17
<212> PRT
<213> Artificial Sequence

<220>
<223> RT37 VH-CDR2

<400>    16
Tyr Ile Ser Arg Thr Ser His Thr Ile Ala Tyr Ala Asp Ser Val Lys
1               5                   10                  15

Gly

<210>    17
<211>    6
<212>    PRT
<213>    Artificial Sequence

<220>
<223>    RT35 VH-CDR3

<400>    17
Gly Phe Arg Leu Asp Tyr
1               5

<210>    18
<211>    6
<212>    PRT
<213>    Artificial Sequence

<220>
<223>    RT36 VH-CDR3

<400>    18
Gly Phe Asn Leu Asp Tyr
1               5

<210>    19
<211>    115
<212>    PRT
<213>    Artificial Sequence

<220>
<223>    RT11 VH

<400>    19
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Phe Met Asp Tyr Trp Gly Gln Gly Thr Leu Val Thr
            100                 105                 110

Val Ser Ser
        115

<210>   20
<211>   115
<212>   PRT
<213>   Artificial Sequence

<220>
<223>   RT21 VH

<400>   20
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Lys Met Asp Tyr Trp Gly Gln Gly Thr Leu Val Thr
            100                 105                 110

Val Ser Ser
        115

<210>   21
<211>   115
<212>   PRT
<213>   Artificial Sequence

<220>
<223>   RT22 VH

<400>   21
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Tyr Tyr Ala Asp Ser Val
50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Lys Met Asp Tyr Trp Gly Gln Gly Thr Leu Val Thr
            100                 105                 110

Val Ser Ser
        115

<210>   22
<211>   115
<212>   PRT
<213>   Artificial Sequence

<220>
<223>   RT23 VH

<400>   22
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Arg Met Asp Tyr Trp Gly Gln Gly Thr Leu Val Thr
            100                 105                 110

Val Ser Ser
        115

<210>   23
<211>   115
<212>   PRT
<213>   Artificial Sequence

<220>
<223>   RT24 VH

<400>   23
Glu Val Gln Leu Val Glu Ser Gly Gly Gly Leu Val Gln Pro Gly Gly

1                 5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Phe Phe Met Asn Tyr Trp Gly Gln Gly Thr Leu Val Thr
            100                 105                 110

Val Ser Ser
        115


<210>    24
<211>    115
<212>    PRT
<213>    Artificial Sequence

<220>
<223>    RT25 VH


<400>    24
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Arg Met Asp Tyr Trp Gly Gln Gly Thr Leu Val Thr
            100                 105                 110

Val Ser Ser
        115


<210>    25
<211>    115
<212>    PRT
<213>    Artificial Sequence

<220>
<223>    RT26 VH

<400>    25
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Phe Met Asn Tyr Trp Gly Gln Gly Thr Leu Val Thr
            100                 105                 110

Val Ser Ser
        115

<210>    26
<211>    115
<212>    PRT
<213>    Artificial Sequence

<220>
<223>    RT31 VH

<400>    26
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Lys Met Asp Tyr Trp Gly Gln Gly Thr Leu Val Thr
            100                 105                 110

Val Ser Ser

        115

<210> 27
<211> 115
<212> PRT
<213> Artificial Sequence

<220>
<223> RT32 VH

<400> 27
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Cys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Lys Met Asp Tyr Trp Gly Gln Gly Thr Leu Val Thr
            100                 105                 110

Val Ser Ser
        115

<210> 28
<211> 115
<212> PRT
<213> Artificial Sequence

<220>
<223> RT33 VH

<400> 28
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Thr Ser Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Lys Met Asp Tyr Trp Gly Gln Gly Thr Leu Val Thr
            100                 105                 110

Val Ser Ser
        115

<210>   29
<211>   115
<212>   PRT
<213>   Artificial Sequence

<220>
<223>   RT34 VH

<400>   29
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Ile Ser Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Lys Met Asp Tyr Trp Gly Gln Gly Thr Leu Val Thr
            100                 105                 110

Val Ser Ser
        115

<210>   30
<211>   115
<212>   PRT
<213>   Artificial Sequence

<220>
<223>   RT35 VH

<400>   30
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Leu Cys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Arg Leu Asp Tyr Trp Gly Gln Gly Thr Leu Val Thr
            100                 105                 110

Val Ser Ser
        115

<210> 31
<211> 115
<212> PRT
<213> Artificial Sequence

<220>
<223> RT36 VH

<400> 31
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Leu Leu Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Asn Leu Asp Tyr Trp Gly Gln Gly Thr Leu Val Thr
            100                 105                 110

Val Ser Ser
        115

<210> 32
<211> 115
<212> PRT
<213> Artificial Sequence

<220>
<223> RT37 VH

<400> 32
Glu Val Gln Leu Val Glu Ser Gly Gly Gly Leu Val Gln Pro Gly Gly

1               5                   10                  15

Ser Leu Arg Leu Ser Cys Ala Ala Ser Gly Phe Thr Phe Ser Asp Phe
            20                  25                  30

Ser Met Ser Trp Val Arg Gln Ala Pro Gly Lys Gly Leu Glu Trp Val
        35                  40                  45

Ser Tyr Ile Ser Arg Thr Ser His Thr Ile Ala Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Lys Met Asp Tyr Trp Gly Gln Gly Thr Leu Val Thr
            100                 105                 110

Val Ser Ser
        115


<210>   33
<211>   114
<212>   PRT
<213>   Artificial Sequence

<220>
<223>   hT4-3 VL


<400>   33
Asp Leu Val Met Thr Gln Ser Pro Ser Ser Leu Ser Ala Ser Val Gly
1               5                   10                  15

Asp Arg Val Thr Ile Thr Cys Lys Ser Ser Gln Ser Leu Phe Asn Ser
            20                  25                  30

Arg Thr Arg Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg



<210>   34
<211>   114
<212>   PRT
<213>   Artificial Sequence

<220>
<223>    hT4-03 VL

<400>    34
Asp Leu Val Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asp Ser
            20                  25                  30

Asp Asp Gly Asn Thr Tyr Leu Ala Trp Tyr Gln Gln Lys Pro Gly Lys
        35                  40                  45

Ala Pro Lys Leu Leu Ile Tyr Trp Leu Ser Tyr Arg Ala Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Gln Gln
                85                  90                  95

Tyr Tyr Tyr His Met Tyr Thr Phe Gly Gln Gly Thr Lys Val Glu Ile
            100                 105                 110

Lys Arg

<210>    35
<211>    114
<212>    PRT
<213>    Artificial Sequence

<220>
<223>    hT4-33 VL

<400>    35
Asp Leu Val Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asp Ser
            20                  25                  30

Asp Asp Gly Asn Thr Tyr Leu Ala Trp Tyr Gln Gln Lys Pro Gly Lys
        35                  40                  45

Ala Pro Lys Leu Leu Ile Tyr Trp Leu Ser Tyr Arg Ala Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg

<210>    36
<211>    114
<212>    PRT
<213>    Artificial Sequence

<220>
<223>    hT4-34 VL

<400>    36
Asp Leu Val Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Thr Arg Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg

<210>    37
<211>    114
<212>    PRT
<213>    Artificial Sequence

<220>
<223>    hT4-35 VL

<400>    37
Asp Leu Val Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Asp Gly Asn Thr Tyr Leu Ala Trp Tyr Gln Gln Lys Pro Gly Lys
        35                  40                  45

Ala Pro Lys Leu Leu Ile Tyr Trp Leu Ser Tyr Arg Ala Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg

<210>   38
<211>   114
<212>   PRT
<213>   Artificial Sequence

<220>
<223>   hT4-36 VL

<400>   38
Asp Leu Val Met Thr Gln Ser Pro Ser Ser Leu Ser Ala Ser Val Gly
 1               5                  10                  15

Asp Arg Val Thr Ile Thr Cys Lys Ser Ser Gln Ser Leu Leu Asn Ser
            20                  25                  30

Asp Asp Arg Asn Thr Tyr Leu Ala Trp Tyr Gln Gln Lys Pro Gly Lys
        35                  40                  45

Ala Pro Lys Leu Leu Ile Tyr Trp Leu Ser Tyr Arg Ala Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg

<210>   39
<211>   114
<212>   PRT
<213>   Artificial Sequence

<220>
<223>   hT4-37 VL

<400>   39
Asp Leu Val Met Thr Gln Ser Pro Ser Ser Leu Ser Ala Ser Val Gly
 1               5                  10                  15

Asp Arg Val Thr Ile Thr Cys Lys Ser Ser Gln Ser Leu Leu Asn Ser
            20                  25                  30

Asp Asp Gly Lys Thr Tyr Leu Ala Trp Tyr Gln Gln Lys Pro Gly Lys
        35                  40                  45

Ala Pro Lys Leu Leu Ile Tyr Trp Leu Ser Tyr Arg Ala Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg

<210>    40
<211>    114
<212>    PRT
<213>    Artificial Sequence

<220>
<223>    hT4-38 VL

<400>    40
Asp Leu Val Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Thr Gly Lys Thr Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg

<210>    41
<211>    114
<212>    PRT
<213>    Artificial Sequence

<220>
<223>    hT4-39 VL

<400>    41
Asp Leu Val Met Thr Gln Ser Pro Ser Ser Leu Ser Ala Ser Val Gly

1               5                   10                  15

Asp Arg Val Thr Ile Thr Cys Lys Ser Ser Gln Ser Leu Leu Asn Ser
            20                  25                  30

Arg Asp Gly Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg



<210>   42
<211>   114
<212>   PRT
<213>   Artificial Sequence

<220>
<223>   hT4-58 VL


<400>   42
Asp Ile Gln Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Thr Gly Lys Thr Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg



<210>   43
<211>   114
<212>   PRT
<213>   Artificial Sequence

<220>
<223>    hT4-59 VL

<400>    43
Asp Ile Gln Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Asp Gly Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Phe Cys Gln Gln
                85                  90                  95

Tyr Trp Tyr Trp Met Tyr Thr Phe Gly Gln Gly Thr Lys Val Glu Ile
            100                 105                 110

Lys Arg

<210>    44
<211>    133
<212>    PRT
<213>    Artificial Sequence

<220>
<223>    hT4-i34 MG VL

<400>    44
Asp Gly Ala Arg Tyr Cys Arg Gly Asp Cys Phe Asp Gly Met Gly Ser
1               5                   10                  15

Ser Ser Asn Asp Leu Val Met Thr Gln Ser Pro Ser Ser Leu Ser Ala
            20                  25                  30

Ser Val Gly Asp Arg Val Thr Ile Thr Cys Lys Ser Ser Gln Ser Leu
        35                  40                  45

Leu Asn Ser Arg Thr Arg Lys Asn Tyr Leu Ala Trp Tyr Gln Gln Lys
    50                  55                  60

Pro Gly Lys Ala Pro Lys Leu Leu Ile Tyr Trp Ala Ser Thr Arg Glu
65                  70                  75                  80

Ser Gly Val Pro Ser Arg Phe Ser Gly Ser Gly Ser Gly Thr Asp Phe
                85                  90                  95

Thr Leu Thr Ile Ser Ser Leu Gln Pro Glu Asp Phe Ala Thr Tyr Phe
            100                 105                 110

Cys Gln Gln Tyr Trp Tyr Trp Met Tyr Thr Phe Gly Gln Gly Thr Lys

115                 120                 125

Val Glu Ile Lys Arg
    130



<210>   45
<211>   133
<212>   PRT
<213>   Artificial Sequence

<220>
<223>   hT4-i35 MG VL


<400>   45
Asp Gly Ala Arg Tyr Cys Arg Gly Asp Cys Phe Asp Gly Met Gly Ser
  1               5                  10                  15

Ser Ser Asn Asp Leu Val Met Thr Gln Ser Pro Ser Ser Leu Ser Ala
             20                  25                  30

Ser Val Gly Asp Arg Val Thr Ile Thr Cys Lys Ser Ser Gln Ser Leu
         35                  40                  45

Leu Asn Ser Arg Asp Gly Asn Thr Tyr Leu Ala Trp Tyr Gln Gln Lys
    50                  55                  60

Pro Gly Lys Ala Pro Lys Leu Leu Ile Tyr Trp Leu Ser Tyr Arg Ala
 65                  70                  75                  80

Ser Gly Val Pro Ser Arg Phe Ser Gly Ser Gly Ser Gly Thr Asp Phe
                 85                  90                  95

Thr Leu Thr Ile Ser Ser Leu Gln Pro Glu Asp Phe Ala Thr Tyr Phe
            100                 105                 110

Cys Gln Gln Tyr Trp Tyr Trp Met Tyr Thr Phe Gly Gln Gly Thr Lys
        115                 120                 125

Val Glu Ile Lys Arg
    130



<210>   46
<211>   133
<212>   PRT
<213>   Artificial Sequence

<220>
<223>   hT4-i36 MG VL


<400>   46
Asp Gly Ala Arg Tyr Cys Arg Gly Asp Cys Phe Asp Gly Met Gly Ser
  1               5                  10                  15

Ser Ser Asn Asp Leu Val Met Thr Gln Ser Pro Ser Ser Leu Ser Ala
             20                  25                  30

Ser Val Gly Asp Arg Val Thr Ile Thr Cys Lys Ser Ser Gln Ser Leu
         35                  40                  45

Leu Asn Ser Asp Asp Arg Asn Thr Tyr Leu Ala Trp Tyr Gln Gln Lys
    50                  55                  60

Pro Gly Lys Ala Pro Lys Leu Leu Ile Tyr Trp Leu Ser Tyr Arg Ala
65                  70                  75                  80

Ser Gly Val Pro Ser Arg Phe Ser Gly Ser Gly Ser Gly Thr Asp Phe
                85                  90                  95

Thr Leu Thr Ile Ser Ser Leu Gln Pro Glu Asp Phe Ala Thr Tyr Phe
            100                 105                 110

Cys Gln Gln Tyr Trp Tyr Trp Met Tyr Thr Phe Gly Gln Gly Thr Lys
        115                 120                 125

Val Glu Ile Lys Arg
    130

<210> 47
<211> 133
<212> PRT
<213> Artificial Sequence

<220>
<223> hT4-i37 MG VL

<400> 47
Asp Gly Ala Arg Tyr Cys Arg Gly Asp Cys Phe Asp Gly Met Gly Ser
1               5                   10                  15

Ser Ser Asn Asp Leu Val Met Thr Gln Ser Pro Ser Ser Leu Ser Ala
            20                  25                  30

Ser Val Gly Asp Arg Val Thr Ile Thr Cys Lys Ser Ser Gln Ser Leu
        35                  40                  45

Leu Asn Ser Asp Asp Gly Lys Thr Tyr Leu Ala Trp Tyr Gln Gln Lys
    50                  55                  60

Pro Gly Lys Ala Pro Lys Leu Leu Ile Tyr Trp Leu Ser Tyr Arg Ala
65                  70                  75                  80

Ser Gly Val Pro Ser Arg Phe Ser Gly Ser Gly Ser Gly Thr Asp Phe
                85                  90                  95

Thr Leu Thr Ile Ser Ser Leu Gln Pro Glu Asp Phe Ala Thr Tyr Phe
            100                 105                 110

Cys Gln Gln Tyr Trp Tyr Trp Met Tyr Thr Phe Gly Gln Gly Thr Lys
        115                 120                 125

Val Glu Ile Lys Arg
    130

<210> 48
<211> 133
<212> PRT
<213> Artificial Sequence

<220>

<223>    hT4-i38 MG VL

<400>    48
Asp Gly Ala Arg Tyr Cys Arg Gly Asp Cys Phe Asp Gly Met Gly Ser
1               5                   10                  15

Ser Ser Asn Asp Leu Val Met Thr Gln Ser Pro Ser Ser Leu Ser Ala
            20                  25                  30

Ser Val Gly Asp Arg Val Thr Ile Thr Cys Lys Ser Ser Gln Ser Leu
        35                  40                  45

Leu Asn Ser Arg Thr Gly Lys Thr Tyr Leu Ala Trp Tyr Gln Gln Lys
    50                  55                  60

Pro Gly Lys Ala Pro Lys Leu Leu Ile Tyr Trp Ala Ser Thr Arg Glu
65                  70                  75                  80

Ser Gly Val Pro Ser Arg Phe Ser Gly Ser Gly Ser Gly Thr Asp Phe
                85                  90                  95

Thr Leu Thr Ile Ser Ser Leu Gln Pro Glu Asp Phe Ala Thr Tyr Phe
            100                 105                 110

Cys Gln Gln Tyr Trp Tyr Trp Met Tyr Thr Phe Gly Gln Gly Thr Lys
        115                 120                 125

Val Glu Ile Lys Arg
    130

<210>    49
<211>    133
<212>    PRT
<213>    Artificial Sequence

<220>
<223>    hT4-i39 MG VL

<400>    49
Asp Gly Ala Arg Tyr Cys Arg Gly Asp Cys Phe Asp Gly Met Gly Ser
1               5                   10                  15

Ser Ser Asn Asp Leu Val Met Thr Gln Ser Pro Ser Ser Leu Ser Ala
            20                  25                  30

Ser Val Gly Asp Arg Val Thr Ile Thr Cys Lys Ser Ser Gln Ser Leu
        35                  40                  45

Leu Asn Ser Arg Asp Gly Lys Asn Tyr Leu Ala Trp Tyr Gln Gln Lys
    50                  55                  60

Pro Gly Lys Ala Pro Lys Leu Leu Ile Tyr Trp Ala Ser Thr Arg Glu
65                  70                  75                  80

Ser Gly Val Pro Ser Arg Phe Ser Gly Ser Gly Ser Gly Thr Asp Phe
                85                  90                  95

Thr Leu Thr Ile Ser Ser Leu Gln Pro Glu Asp Phe Ala Thr Tyr Phe
            100                 105                 110

Cys Gln Gln Tyr Trp Tyr Trp Met Tyr Thr Phe Gly Gln Gly Thr Lys
        115                 120                 125

Val Glu Ile Lys Arg
    130

<210>    50
<211>    132
<212>    PRT
<213>    Artificial Sequence

<220>
<223>    hT4-ep33 MG VL

<400>    50
Glu His Leu His Cys Leu Gly Ser Leu Cys Trp Pro Met Gly Ser Ser
 1               5                  10                  15

Ser Asn Asp Leu Val Met Thr Gln Ser Pro Ser Ser Leu Ser Ala Ser
             20                  25                  30

Val Gly Asp Arg Val Thr Ile Thr Cys Lys Ser Ser Gln Ser Leu Leu
         35                  40                  45

Asp Ser Asp Asp Gly Asn Thr Tyr Leu Ala Trp Tyr Gln Gln Lys Pro
    50                  55                  60

Gly Lys Ala Pro Lys Leu Leu Ile Tyr Trp Ala Ser Thr Arg Glu Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130

<210>    51
<211>    132
<212>    PRT
<213>    Artificial Sequence

<220>
<223>    hT4-ep34 MG VL

<400>    51
Glu His Leu His Cys Leu Gly Ser Leu Cys Trp Pro Met Gly Ser Ser
 1               5                  10                  15

Ser Asn Asp Leu Val Met Thr Gln Ser Pro Ser Ser Leu Ser Ala Ser
             20                  25                  30

Val Gly Asp Arg Val Thr Ile Thr Cys Lys Ser Ser Gln Ser Leu Leu
         35                  40                  45

Asn Ser Arg Thr Arg Lys Asn Tyr Leu Ala Trp Tyr Gln Gln Lys Pro
50                  55                  60

Gly Lys Ala Pro Lys Leu Leu Ile Tyr Trp Ala Ser Thr Arg Glu Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130


<210>    52
<211>    132
<212>    PRT
<213>    Artificial Sequence

<220>
<223>    hT4-ep35 MG VL


<400>    52
Glu His Leu His Cys Leu Gly Ser Leu Cys Trp Pro Met Gly Ser Ser
1               5                   10                  15

Ser Asn Asp Leu Val Met Thr Gln Ser Pro Ser Ser Leu Ser Ala Ser
            20                  25                  30

Val Gly Asp Arg Val Thr Ile Thr Cys Lys Ser Ser Gln Ser Leu Leu
        35                  40                  45

Asn Ser Arg Asp Gly Asn Thr Tyr Leu Ala Trp Tyr Gln Gln Lys Pro
    50                  55                  60

Gly Lys Ala Pro Lys Leu Leu Ile Tyr Trp Leu Ser Tyr Arg Ala Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130


<210>    53
<211>    132
<212>    PRT
<213>    Artificial Sequence

<220>
<223>    hT4-ep36 MG VL


<400>    53
Glu His Leu His Cys Leu Gly Ser Leu Cys Trp Pro Met Gly Ser Ser
1               5                   10                  15

Ser Asn Asp Leu Val Met Thr Gln Ser Pro Ser Ser Leu Ser Ala Ser
            20                  25                  30

Val Gly Asp Arg Val Thr Ile Thr Cys Lys Ser Ser Gln Ser Leu Leu
        35                  40                  45

Asn Ser Asp Asp Arg Asn Thr Tyr Leu Ala Trp Tyr Gln Gln Lys Pro
    50                  55                  60

Gly Lys Ala Pro Lys Leu Leu Ile Tyr Trp Leu Ser Tyr Arg Ala Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130


<210>    54
<211>    132
<212>    PRT
<213>    Artificial Sequence

<220>
<223>    hT4-ep37 MG VL


<400>    54
Glu His Leu His Cys Leu Gly Ser Leu Cys Trp Pro Met Gly Ser Ser
1               5                   10                  15

Ser Asn Asp Leu Val Met Thr Gln Ser Pro Ser Ser Leu Ser Ala Ser
            20                  25                  30

Val Gly Asp Arg Val Thr Ile Thr Cys Lys Ser Ser Gln Ser Leu Leu
        35                  40                  45

Asn Ser Asp Asp Gly Lys Thr Tyr Leu Ala Trp Tyr Gln Gln Lys Pro
    50                  55                  60

Gly Lys Ala Pro Lys Leu Leu Ile Tyr Trp Leu Ser Tyr Arg Ala Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130

<210>   55
<211>   132
<212>   PRT
<213>   Artificial Sequence

<220>
<223>   hT4-ep38 MG VL

<400>   55
Glu His Leu His Cys Leu Gly Ser Leu Cys Trp Pro Met Gly Ser Ser
1               5                   10                  15

Ser Asn Asp Leu Val Met Thr Gln Ser Pro Ser Ser Leu Ser Ala Ser
            20                  25                  30

Val Gly Asp Arg Val Thr Ile Thr Cys Lys Ser Ser Gln Ser Leu Leu
        35                  40                  45

Asn Ser Arg Thr Gly Lys Thr Tyr Leu Ala Trp Tyr Gln Gln Lys Pro
    50                  55                  60

Gly Lys Ala Pro Lys Leu Leu Ile Tyr Trp Ala Ser Thr Arg Glu Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130

<210>   56
<211>   132
<212>   PRT
<213>   Artificial Sequence

<220>
<223>   hT4-ep39 MG VL

<400>   56
Glu His Leu His Cys Leu Gly Ser Leu Cys Trp Pro Met Gly Ser Ser
1               5                   10                  15

Ser Asn Asp Leu Val Met Thr Gln Ser Pro Ser Ser Leu Ser Ala Ser
            20                  25                  30

Val Gly Asp Arg Val Thr Ile Thr Cys Lys Ser Ser Gln Ser Leu Leu

35                  40                  45

Asn Ser Arg Asp Gly Lys Asn Tyr Leu Ala Trp Tyr Gln Gln Lys Pro
    50                  55                  60

Gly Lys Ala Pro Lys Leu Leu Ile Tyr Trp Ala Ser Thr Arg Glu Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130


<210>   57
<211>   132
<212>   PRT
<213>   Artificial Sequence

<220>
<223>   hT4-ep58 MG VL


<400>   57
Glu His Leu His Cys Leu Gly Ser Leu Cys Trp Pro Met Gly Ser Ser
1               5                   10                  15

Ser Asn Asp Ile Gln Met Thr Gln Ser Pro Ser Ser Leu Ser Ala Ser
            20                  25                  30

Val Gly Asp Arg Val Thr Ile Thr Cys Lys Ser Ser Gln Ser Leu Leu
        35                  40                  45

Asn Ser Arg Thr Gly Lys Thr Tyr Leu Ala Trp Tyr Gln Gln Lys Pro
    50                  55                  60

Gly Lys Ala Pro Lys Leu Leu Ile Tyr Trp Ala Ser Thr Arg Glu Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130


<210>   58
<211>   132
<212>   PRT
<213>   Artificial Sequence

<220>
<223>    hT4-ep59 MG VL


<400>    58
Glu His Leu His Cys Leu Gly Ser Leu Cys Trp Pro Met Gly Ser Ser
1               5                   10                  15

Ser Asn Asp Ile Gln Met Thr Gln Ser Pro Ser Ser Leu Ser Ala Ser
            20                  25                  30

Val Gly Asp Arg Val Thr Ile Thr Cys Lys Ser Ser Gln Ser Leu Leu
        35                  40                  45

Asn Ser Arg Asp Gly Lys Asn Tyr Leu Ala Trp Tyr Gln Gln Lys Pro
    50                  55                  60

Gly Lys Ala Pro Lys Leu Leu Ile Tyr Trp Ala Ser Thr Arg Glu Ser
65                  70                  75                  80

Gly Val Pro Ser Arg Phe Ser Gly Ser Gly Ser Gly Thr Asp Phe Thr
                85                  90                  95

Leu Thr Ile Ser Ser Leu Gln Pro Glu Asp Phe Ala Thr Tyr Phe Cys
            100                 105                 110

Gln Gln Tyr Trp Tyr Trp Met Tyr Thr Phe Gly Gln Gly Thr Lys Val
        115                 120                 125

Glu Ile Lys Arg
    130


<210>    59
<211>    130
<212>    PRT
<213>    Artificial Sequence

<220>
<223>    hT4-ep59 GSSG VL


<400>    59
Glu His Leu His Cys Leu Gly Ser Leu Cys Trp Pro Gly Ser Ser Gly
1               5                   10                  15

Asp Ile Gln Met Thr Gln Ser Pro Ser Ser Leu Ser Ala Ser Val Gly
            20                  25                  30

Asp Arg Val Thr Ile Thr Cys Lys Ser Ser Gln Ser Leu Leu Asn Ser
        35                  40                  45

Arg Asp Gly Lys Asn Tyr Leu Ala Trp Tyr Gln Gln Lys Pro Gly Lys
    50                  55                  60

Ala Pro Lys Leu Leu Ile Tyr Trp Ala Ser Thr Arg Glu Ser Gly Val
65                  70                  75                  80

Pro Ser Arg Phe Ser Gly Ser Gly Ser Gly Thr Asp Phe Thr Leu Thr
                85                  90                  95

Ile Ser Ser Leu Gln Pro Glu Asp Phe Ala Thr Tyr Phe Cys Gln Gln

100                 105                 110

Tyr Trp Tyr Trp Met Tyr Thr Phe Gly Gln Gly Thr Lys Val Glu Ile
        115                 120                 125

Lys Arg
    130

<210>  60
<211>  136
<212>  PRT
<213>  Artificial Sequence

<220>
<223>  hT4-ep33 (G4S)2 VL

<400>  60
Glu His Leu His Cys Leu Gly Ser Leu Cys Trp Pro Gly Gly Gly Gly
1               5                   10                  15

Ser Gly Gly Gly Gly Ser Asp Leu Val Met Thr Gln Ser Pro Ser Ser
            20                  25                  30

Leu Ser Ala Ser Val Gly Asp Arg Val Thr Ile Thr Cys Lys Ser Ser
        35                  40                  45

Gln Ser Leu Leu Asp Ser Asp Asp Gly Asn Thr Tyr Leu Ala Trp Tyr
    50                  55                  60

Gln Gln Lys Pro Gly Lys Ala Pro Lys Leu Leu Ile Tyr Trp Ala Ser
65                  70                  75                  80

Thr Arg Glu Ser Gly Val Pro Ser Arg Phe Ser Gly Ser Gly Ser Gly
                85                  90                  95

Thr Asp Phe Thr Leu Thr Ile Ser Ser Leu Gln Pro Glu Asp Phe Ala
            100                 105                 110

Thr Tyr Phe Cys Gln Gln Tyr Trp Tyr Trp Met Tyr Thr Phe Gly Gln
        115                 120                 125

Gly Thr Lys Val Glu Ile Lys Arg
    130                 135

<210>  61
<211>  129
<212>  PRT
<213>  Artificial Sequence

<220>
<223>  epRT22 GS

<400>  61
Glu His Leu His Cys Leu Gly Ser Leu Cys Trp Pro Gly Ser Glu Val
1               5                   10                  15

Gln Leu Val Glu Ser Gly Gly Gly Leu Val Gln Pro Gly Gly Ser Leu
            20                  25                  30

Arg Leu Ser Cys Ala Ala Ser Gly Phe Thr Phe Ser Asp Phe Ser Met
            35                  40                  45

Ser Trp Val Arg Gln Ala Pro Gly Lys Gly Leu Glu Trp Val Ser Tyr
    50                  55                  60

Ile Ser Arg Thr Ser His Thr Thr Tyr Tyr Ala Asp Ser Val Lys Gly
65                  70                  75                  80

Arg Phe Thr Ile Ser Arg Asp Asn Ser Lys Asn Thr Leu Tyr Leu Gln
                85                  90                  95

Met Asn Ser Leu Arg Ala Glu Asp Thr Ala Val Tyr Tyr Cys Ala Arg
            100                 105                 110

Gly Phe Lys Met Asp Tyr Trp Gly Gln Gly Thr Leu Val Thr Val Ser
        115                 120                 125

Ser

<210>   62
<211>   133
<212>   PRT
<213>   Artificial Sequence

<220>
<223>   epRT22 MG

<400>   62
Glu His Leu His Cys Leu Gly Ser Leu Cys Trp Pro Met Gly Ser Ser
1               5                   10                  15

Ser Asn Glu Val Gln Leu Val Glu Ser Gly Gly Gly Leu Val Gln Pro
            20                  25                  30

Gly Gly Ser Leu Arg Leu Ser Cys Ala Ala Ser Gly Phe Thr Phe Ser
        35                  40                  45

Asp Phe Ser Met Ser Trp Val Arg Gln Ala Pro Gly Lys Gly Leu Glu
    50                  55                  60

Trp Val Ser Tyr Ile Ser Arg Thr Ser His Thr Thr Tyr Tyr Ala Asp
65                  70                  75                  80

Ser Val Lys Gly Arg Phe Thr Ile Ser Arg Asp Asn Ser Lys Asn Thr
                85                  90                  95

Leu Tyr Leu Gln Met Asn Ser Leu Arg Ala Glu Asp Thr Ala Val Tyr
            100                 105                 110

Tyr Cys Ala Arg Gly Phe Lys Met Asp Tyr Trp Gly Gln Gly Thr Leu
        115                 120                 125

Val Thr Val Ser Ser
    130

<210>   63
<211>   137
<212>   PRT

<213>    Artificial Sequence

<220>
<223>    epRT22 (G4S)2


<400>    63
Glu His Leu His Cys Leu Gly Ser Leu Cys Trp Pro Gly Gly Gly Gly
1               5                   10                  15

Ser Gly Gly Gly Gly Ser Glu Val Gln Leu Val Glu Ser Gly Gly Gly
            20                  25                  30

Leu Val Gln Pro Gly Gly Ser Leu Arg Leu Ser Cys Ala Ala Ser Gly
        35                  40                  45

Phe Thr Phe Ser Asp Phe Ser Met Ser Trp Val Arg Gln Ala Pro Gly
    50                  55                  60

Lys Gly Leu Glu Trp Val Ser Tyr Ile Ser Arg Thr Ser His Thr Thr
65                  70                  75                  80

Tyr Tyr Ala Asp Ser Val Lys Gly Arg Phe Thr Ile Ser Arg Asp Asn
                85                  90                  95

Ser Lys Asn Thr Leu Tyr Leu Gln Met Asn Ser Leu Arg Ala Glu Asp
            100                 105                 110

Thr Ala Val Tyr Tyr Cys Ala Arg Gly Phe Lys Met Asp Tyr Trp Gly
        115                 120                 125

Gln Gly Thr Leu Val Thr Val Ser Ser
    130                 135


<210>    64
<211>    330
<212>    PRT
<213>    Artificial Sequence

<220>
<223>    IgG1


<400>    64

<210> 65
<211> 330
<212> PRT
<213> Artificial Sequence

<220>
<223> IgG1 N434D

<400> 65
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser

35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asp His Tyr Thr
305                 310                 315                 320

Gln Lys Ser Leu Ser Leu Ser Pro Gly Lys
                325                 330


<210>   66
<211>   327
<212>   PRT
<213>   Artificial Sequence

<220>

<223>    IgG4 S228P

<400>    66
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>   67
<211>   327
<212>   PRT
<213>   Artificial Sequence

<220>
<223>   IgG4 S228P, N434D

<400>   67
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asp His Tyr Thr Gln Lys Ser
305                 310                 315                 320

Leu Ser Leu Ser Leu Gly Lys
                325

<210> 68
<211> 330
<212> PRT
<213> Artificial Sequence

<220>
<223> IgG1 LALA-PG

<400> 68
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Ala Ala Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn

195                 200                 205

Lys Ala Leu Gly Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>    69
<211>    330
<212>    PRT
<213>    Artificial Sequence

<220>
<223>    IgG1 LALA-PG, N434D


<400>    69
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Ala Ala Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Gly Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asp His Tyr Thr
305                 310                 315                 320

Gln Lys Ser Leu Ser Leu Ser Pro Gly Lys
                325                 330